# EUROPÄISCHE PATENTSCHRIFT

(11) **EP 3 292 181 B1**
(45) Veröffentlichungstag und Bekanntmachung des Hinweises auf die Patenterteilung: **11.09.2019**
(21) Anmeldenummer: 16715455.8
(22) Anmeldetag: 08.04.2016
(51) Int. Cl.: C09K 19/06, C09K 19/30, C09K 19/04, C09K 19/12, C09K 19/34, C09K 19/02, C09K 19/54, C07D 311/16, C07D 311/30, C07C 69/587, C07C 69/602, C07C 69/80, C07H 15/10, G02F 1/137

(54) **FLÜSSIGKRISTALLINES MEDIUM**
LIQUID-CRYSTAL MEDIUM
MILIEU CRISTALLIN LIQUIDE

(30) Priorität: 04.05.2015 EP 15001316
(43) Veröffentlichungstag der Anmeldung: 14.03.2018
(73) Patentinhaber: Merck Patent GmbH, 64293 Darmstadt (DE)
(72) Erfinder: HAENSEL, Helmut, 64367 Muehltal (DE); POHLE, Andreas, 64319 Pfungstadt (DE); SCHULER, Brigitte, 63762 Grossostheim (DE); JASPER, Christian, 63500 Seligenstadt (DE)
(86) Internationale Anmeldenummer: PCT/EP2016/000583
(87) Internationale Veröffentlichungsnummer: WO 2016/177445

(56) Entgegenhaltungen:
- EP-A2- 2 628 779
- US-A1- 2013 299 741

## Beschreibung

Die Erfindung betrifft ein flüssigkristallines Medium, welches mindestens eine Verbindung der Formel I, 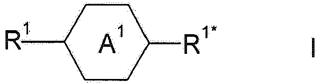 worin
- R¹ und R^{1*}: jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 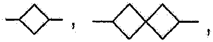 -O-, so ersetzt sein können, dass O-Atome nicht direkt miteinander verknüpft sind, und worin auch ein oder mehrere H-Atome durch Halogen ersetzt sein können,
- A¹: 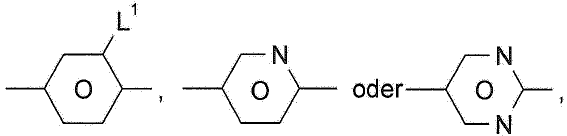
- L¹: F, Cl, CF₃, OCF₃ oder CHF₂,
bedeuten,
enthält.

Flüssigkristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen.

Derartige Vorrichtungen sind beispielsweise Zellen mit dynamischer Streuung, DAP-Zellen (Deformation aufgerichteter Phasen), Gast/Wirt-Zellen, TN Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210 288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschen-rechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Zur Realisierung von 3D-Effekten mittels Shutterbrillen werden insbesondere schnell schaltende Mischungen mit niedrigen Rotationsviskositäten und einer entsprechend hohen optischen Anisotropie (Δn) eingesetzt. Elektrooptische Linsensyteme, mit denen eine 2 dimensionale Darstellung eines Displays in eine 3 dimensionale autostereoskopische Darstellung geschaltet werden kann, können unter Verwendung von Mischungen mit hoher optischer Anisotropie (Δn) realisiert werden.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebens-dauer)
- kleine Schwellenspannung.

Mit den aus dem Stand der Technik zur Verfügung stehenden Medien, wie beispielsweise offenbart in US 2013/299741 A1 oder EP 2 628 779 A2,ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht. Insbesondere bei FK-Anzeigen für TV- und Video-Anwendungen (z. B. LCD-TV, Monitore, PDAs, Notebooks, Spielkonsolen) ist eine deutliche Verringerung der Schaltzeiten gewünscht. Dies erfordert FK-Mischungen mit niedrigen Rotationsviskositäten und hohen Werten für die Doppelbrechung Δn.

Der Erfindung liegt die Aufgabe zugrunde, Medien insbesondere für derartige MFK-, FFS-, IPS-, TN-, positive VA- oder STN-Anzeigen bereitzustellen, die die oben angegebenen Nachteile nicht oder nur in geringem Maße zeigen, und vorzugsweise schnelle Schaltzeiten und niedrige Rotationsviskositäten bei gleichzeitig hohem Klärpunkt, sowie eine hohe dielektrische Anisotropie und eine niedrige Schwellenspannung aufweisen.

Es wurde nun gefunden, dass diese Aufgabe gelöst werden kann, wenn man FK-Medien enthaltend eine oder mehrere Verbindungen der Formel I verwendet. Die Verbindungen der Formel I führen zu LC-Mischungen mit den oben angegebenen gewünschten Eigenschaften.

Sogenannte Einkerner (Verbindungen mit einem Ring) können in der Regel aufgrund der schlechten Phaseneigenschaften und niedrigen Klärpunkte nicht in nematischen Flüssigkristallmischungen verwendet werden. Die Verbindungen der Formel I weisen jedoch überraschenderweise gleichzeitig sehr niedrige Rotationsviskositäten, hohe Absolutwerte der dielektrischen Anisotropie, vergleichsweise hohe Absolutwerte der optischen Anisotropie, gute Löslichkeiten in Flüssigkristallmischungen, sowie eine vergleichsweise geringe Flüchtigkeit auf. Daher können Flüssigkristallmischungen, vorzugsweise IPS- und oder FFS Mischungen, hergestellt werden, die niedrige Schaltzeiten, gleichzeitig gute Phaseneigenschaften und ein gutes Tieftemperaturverhalten aufweisen.

Gegenstand der Erfindung ist somit ein flüssigkristallines Medium, welches mindestens eine Verbindung der Formel I enthält.

Die erfindungsgemäßen Mischungen zeigen vorzugsweise sehr breite nematische Phasenbereiche mit Klärpunkten ≥ 70 °C, vorzugsweise ≥ 75 °C, insbesondere ≥ 80 °C, sehr günstige Werte für die kapazitive Schwelle, relativ hohe Werte für die Holding Ratio und gleichzeitig sehr gute Tieftemperaturstabilitäten (LTS) bei -20 °C und -30 °C sowie sehr geringe Rotationsviskositäten und niedrige Schaltzeiten. Die erfindungsgemäßen Mischungen zeichnen sich weiterhin dadurch aus, dass zusätzlich zur Verbesserung der Rotationsviskosität γ¹, relativ hohe Werte der elastischen Konstanten K33 zur Verbesserung der Schaltzeiten zu beobachten sind.

Einige bevorzugte Ausführungsformen der erfindungsgemäßen Mischungen werden im Folgenden genannt.

In den Verbindungen der Formel I bedeuten R1 und R1* jeweils unabhängig voneinander vorzugsweise geradkettiges Alkoxy, insbesondere OC₂H₅, OC₃H₇, OC₄H₉, OC₅H₁₁, OC₆H₁₃, weiterhin Alkenyloxy, insbesondere OCH₂CH=CH₂, OCH₂CH=CHCH₃, OCH₂CH=CHC₂H₅, ferner Alkyl, insbesondere n-C₃H₇, n-C₄H₉, n-C₅H₁₁, n-C₆H₁₃.

Besonders bevorzugt bedeuten R1 und R1* jeweils unabhängig voneinander geradkettiges Alkoxy mit 1-6 C-Atomen, insbesondere Ethoxy, Butoxy, Pentoxy, Hexoxy oder geradkettiges Alkenyloxy mit 2-6 C-Atomen, insbesondere OCH₂CH=CH₂.

Bevorzugte Verbindungen der Formel I sind die Verbindungen der Formeln I-1 bis I-10, 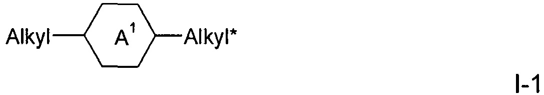 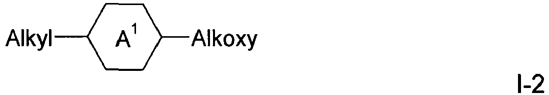 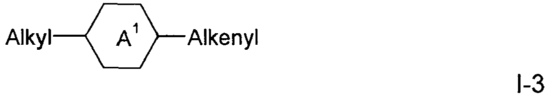 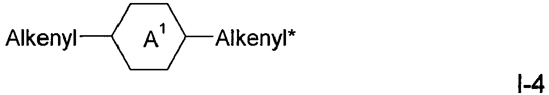 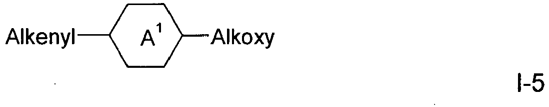 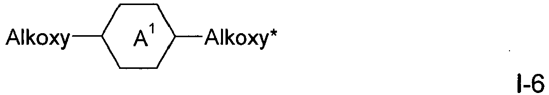 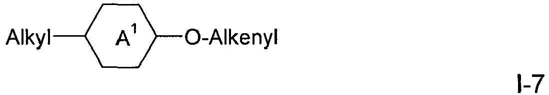 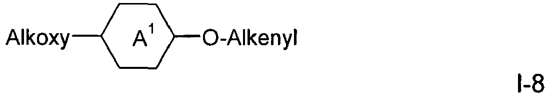 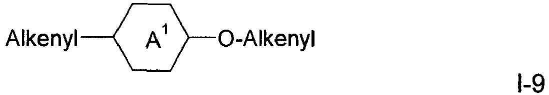 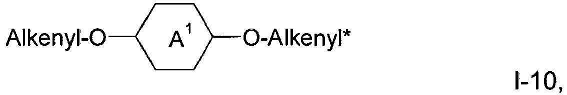 worin
Alkyl und Alkyl* jeweils unabhängig voneinander einen geradkettigen Alkylrest mit 1-6 C-Atomen, Alkenyl und Alkenyl* jeweils unabhängig voneinander einen geradkettigen Alkenylrest mit 2-6 C-Atomen, Alkoxy und Alkoxy* jeweils unabhängig voneinander einen geradkettigen Alkoxyrest mit 1-6 C-Atomen, bedeuten und A¹ die unter Formel I angegebene Bedeutung hat.

In den Verbindungen der Formeln I-1 bis I-10 bedeutet A¹ vorzugsweise 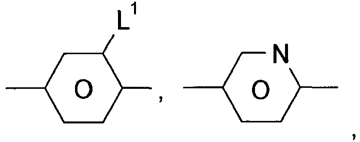 insbesondere

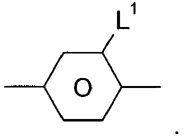

Besonders bevorzugt sind die Verbindungen der Formel I-6.

Ganz besonders bevorzugt enthält die erfindungsgemäße Mischung mindestens eine Verbindung der Formel I-6A oder I-6B:

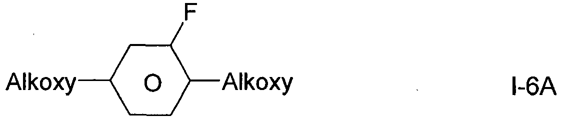

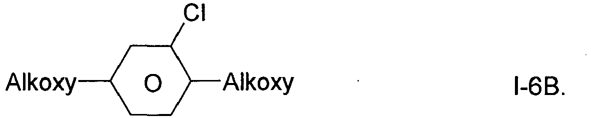

Ganz besonders bevorzugt enthalten die erfindungsgemäßen Mischungen mindestens eine Verbindung aus der folgenden Gruppe:

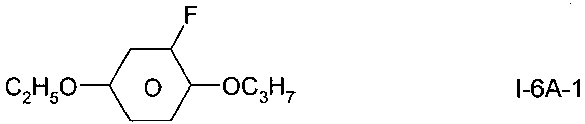

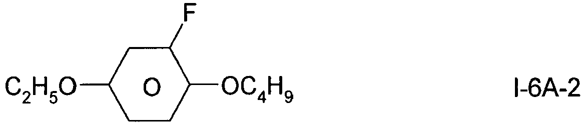

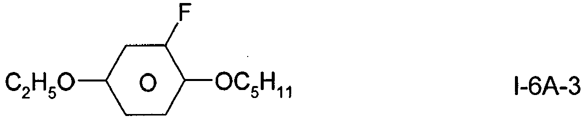

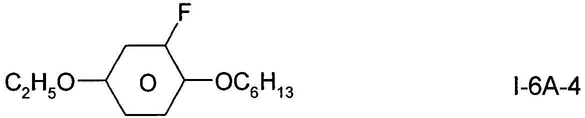

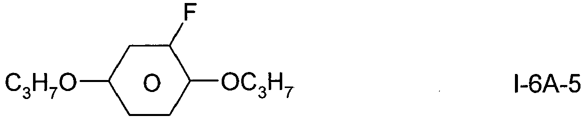

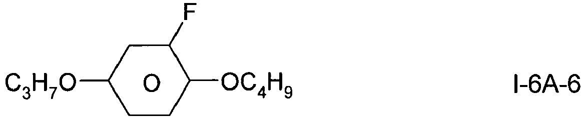

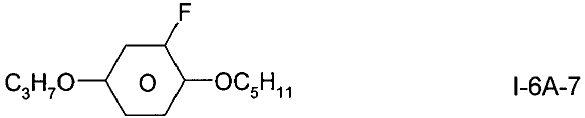

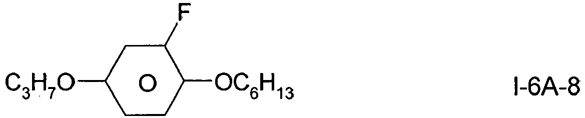

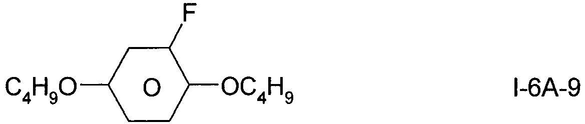

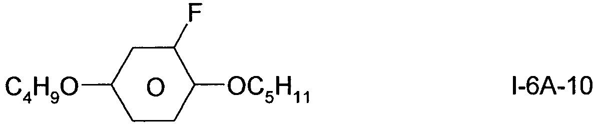

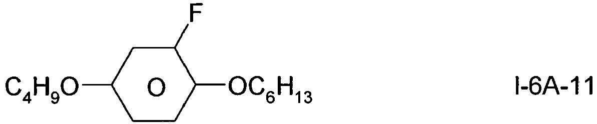

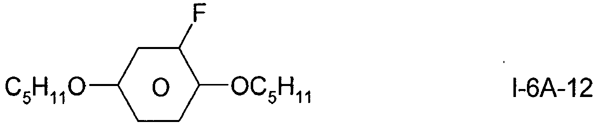

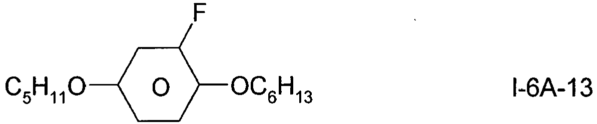

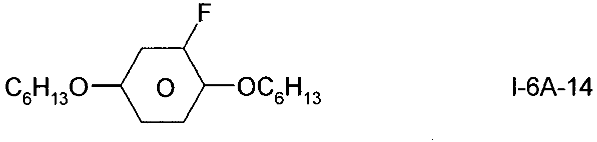

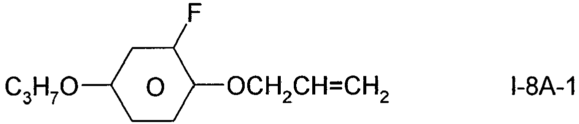

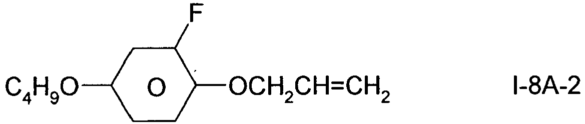

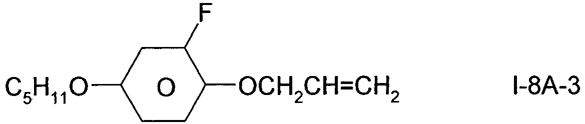

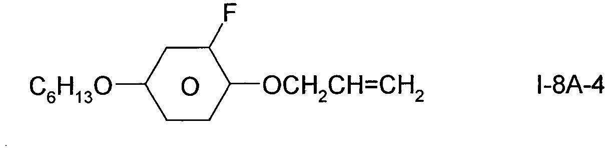

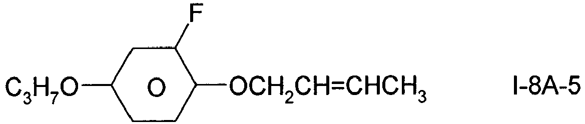

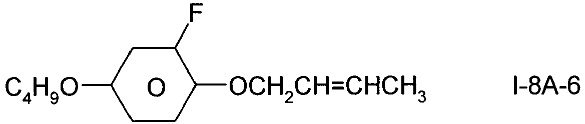

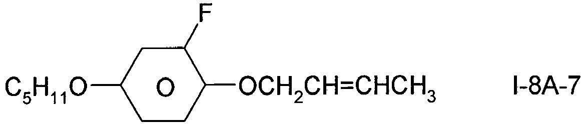

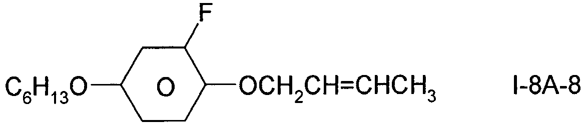

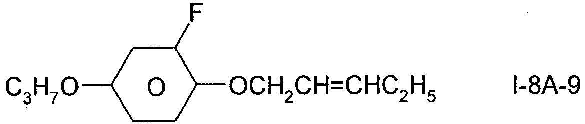

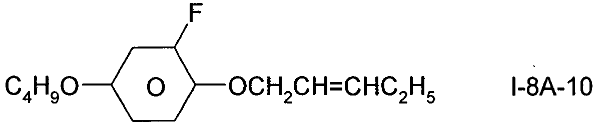

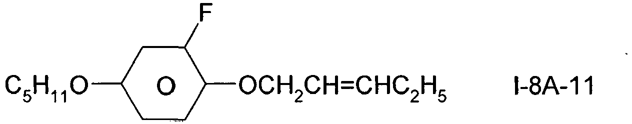

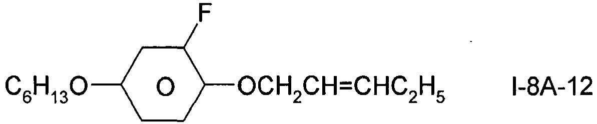

In den Verbindungen der Formel I und den Unterformeln bedeuten R¹ und R^{1*} vorzugsweise beide Alkoxy.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen. Die Verbindungen der Formel I können aber auch in analogerweise wie in WO 2011/64789 oder WO 2010/127208 angegeben dargestellt werden.

Die erfindungsgemäßen Medien enthalten vorzugsweise ein, zwei, drei, vier oder mehr, vorzugsweise ein, zwei oder drei, Verbindungen der Formel I.

Die Verbindungen der Formel I werden vorzugsweise im flüssigkristallinen Medium in Mengen von ≥ 0,5 Gew.%, vorzugsweise ≥ 1 Gew.%, bezogen auf das Gesamtgemisch, eingesetzt. Insbesondere bevorzugt sind flüssigkristalline Medien, die 0,5 - 15 Gew.% einer oder mehrerer Verbindungen der Formel I enthalten.

Weitere bevorzugte Ausführungsformen sind im Folgenden angegeben:
- Das Medium enthält zusätzlich eine oder mehrere Verbindungen ausgewählt aus den folgenden Formeln, 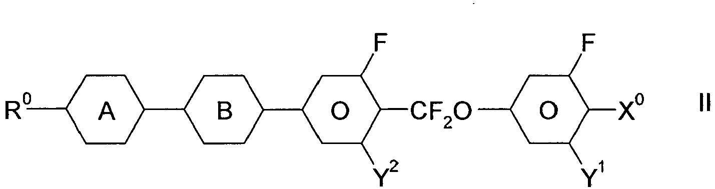 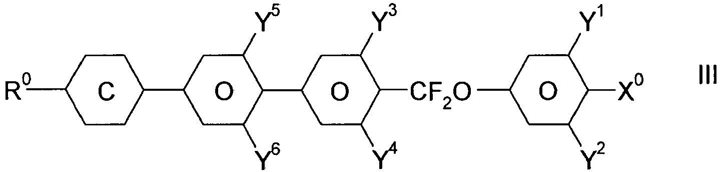
   - R⁰: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH, 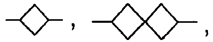 -O-, -CO-O- oder -O-CO- so ersetzt sein können, dass O-Atome nicht direkt miteinander verknüpft sind,
   - X⁰: F, Cl, CN, SF₅, SCN, NCS, halogenierter Alkylrest, halogenierter Alkenylrest, halogenierter Alkoxyrest oder halogenierter Alkenyloxyrest mit bis zu 6 C-Atomen, und
   - Y¹⁻⁶: jeweils unabhängig voneinander H, F oder Cl, und 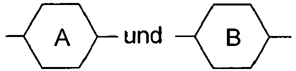 jeweils unabhängig voneinander 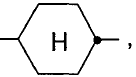 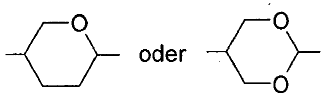 bedeuten, und 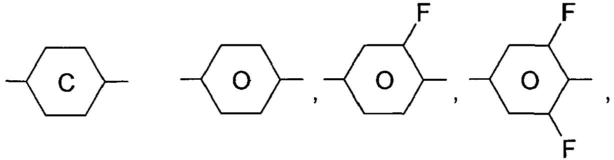 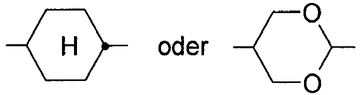 bedeutet;
- Die Verbindungen der Formeln II und III sind vorzugsweise ausgewählt aus den folgenden Formeln, 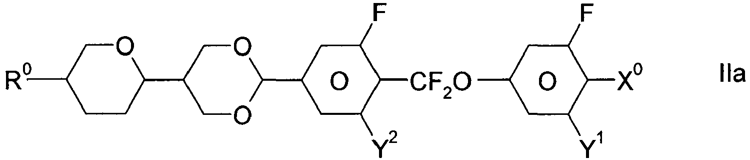 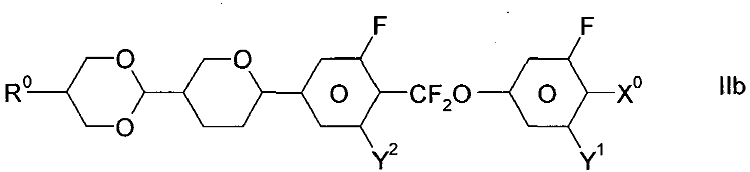 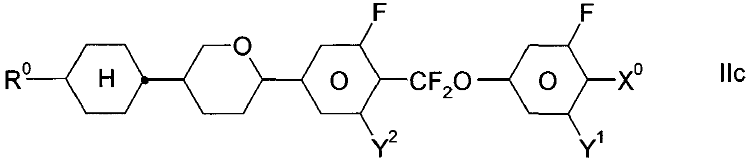 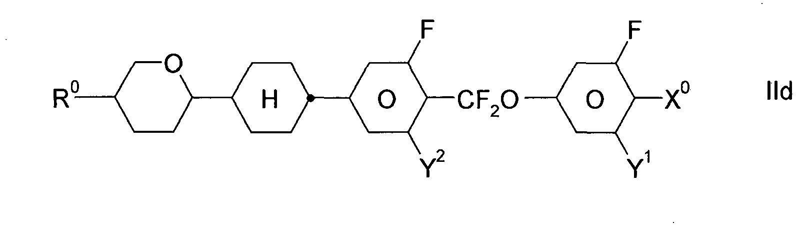 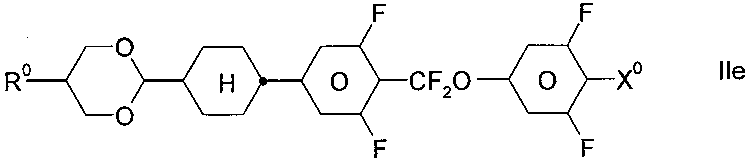 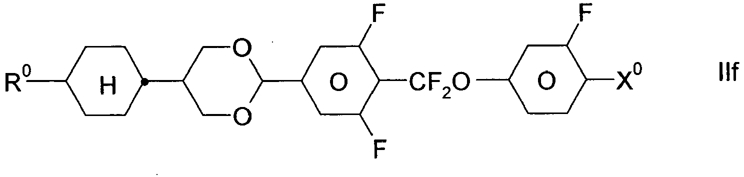 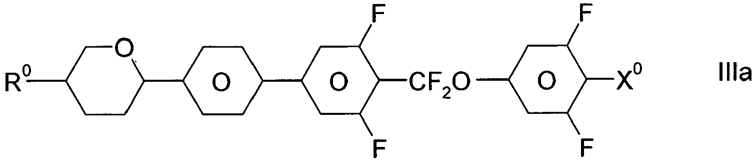 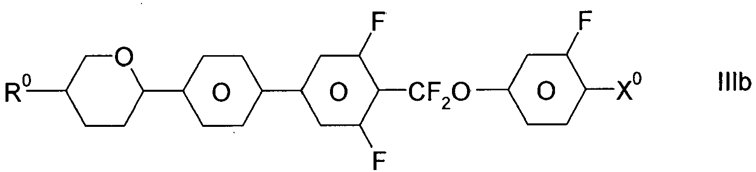 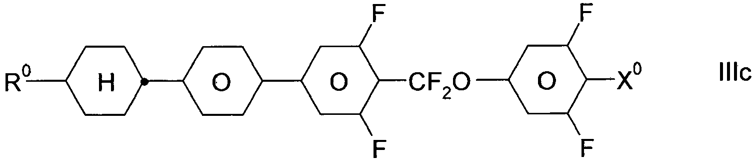 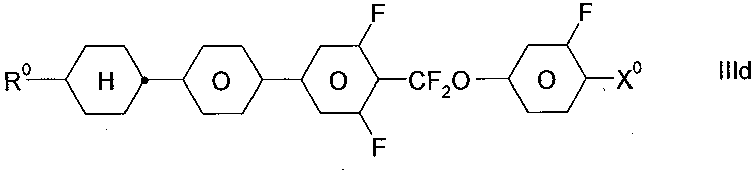 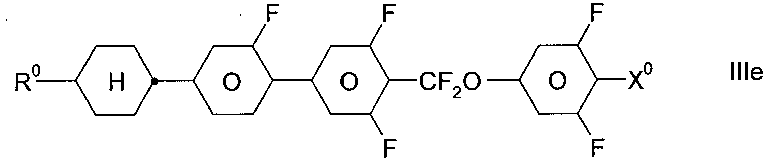 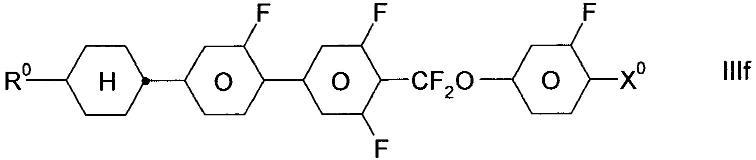 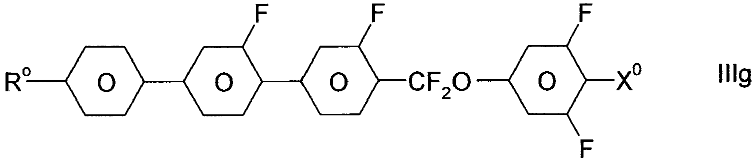 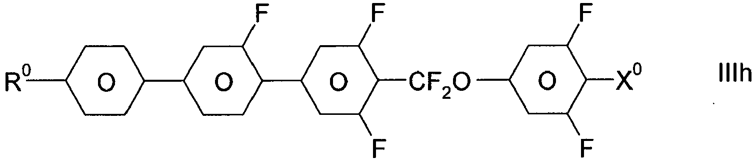 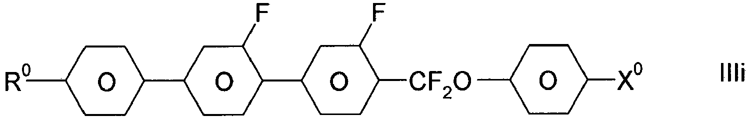 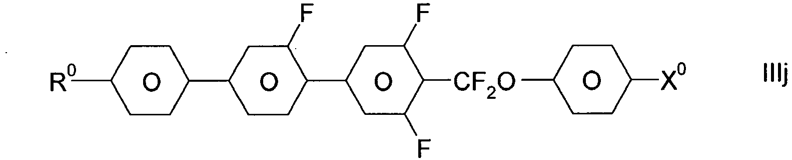 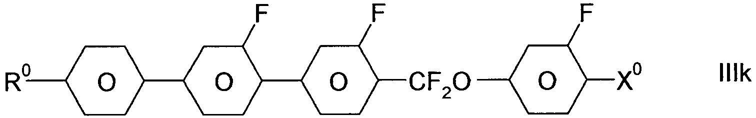 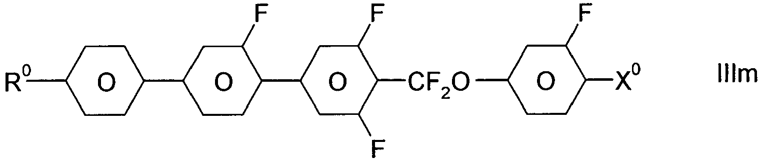 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F, ferner OCF₃. Besonders bevorzugte Verbindungen der Formeln II und IIa-IIf sind solche, worin Y¹ F und Y² H oder F, vorzugsweise F, bedeutet. Besonders bevorzugt enthält die erfindungsgemäße Mischung mindestens eine Verbindung der Formel IIIh.
- Das Medium enthält optional eine oder mehrere Verbindungen ausgewählt aus den folgenden Formeln, 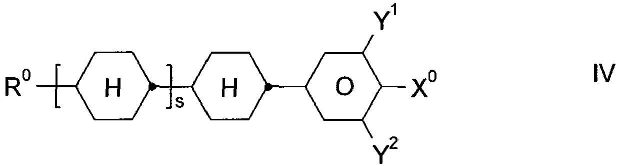 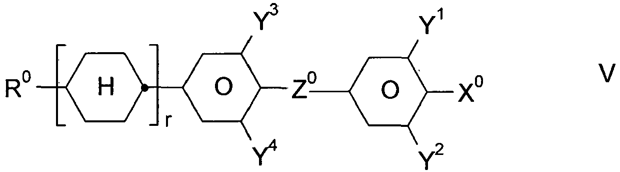 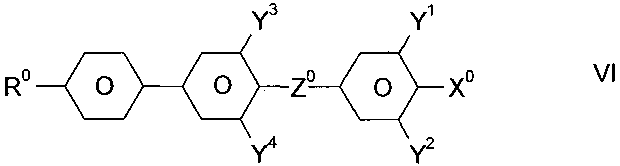 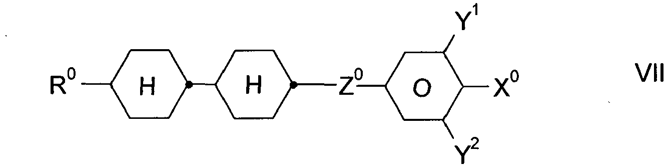 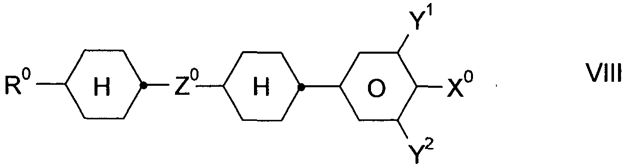 worin
   R⁰, X⁰ und Y¹⁻⁴ die oben angegebenen Bedeutungen besitzen, und
   Z⁰ -C₂H₄-, -(CH2)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in Formel V und VI auch eine Einfachbindung,
   r 0 oder 1, und
   s 0 oder 1
   bedeuten;
- Die Verbindungen der Formel IV sind vorzugsweise ausgewählt aus folgenden Formeln, 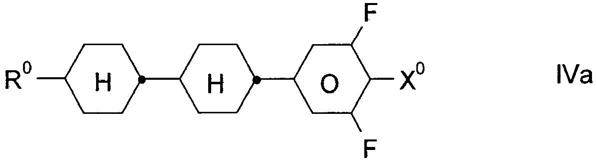 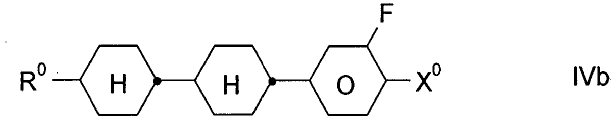 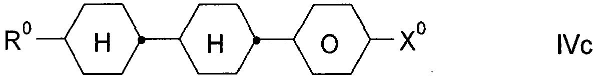 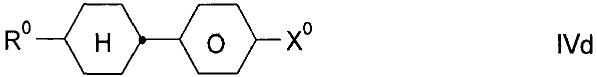 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F oder OCF₃, ferner CF₃, OCF=CF₂ und Cl;
- Die Verbindungen der Formel V sind vorzugsweise ausgewählt aus den folgenden Formeln, 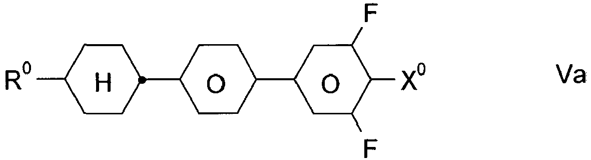 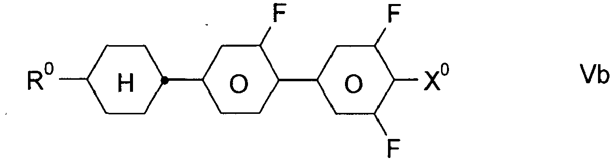 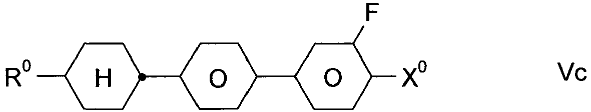 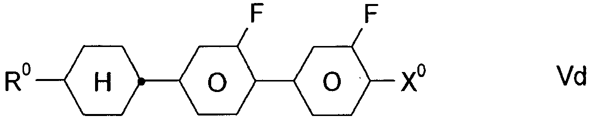 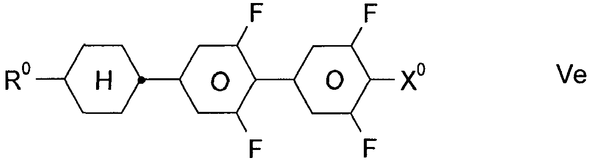 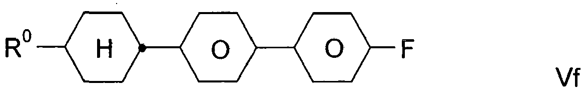 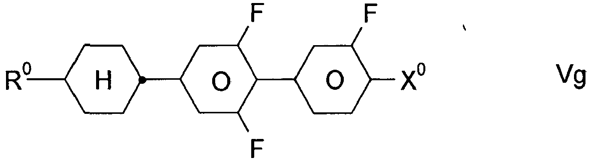 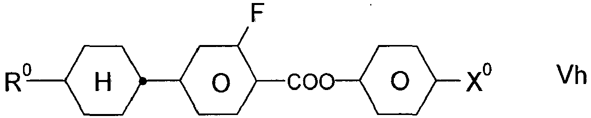 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F und OCF₃, ferner OCHF₂, CF₃, OCF=CF₂ und OCH=CF₂;
- Die Verbindungen der Formel VI sind vorzugsweise ausgewählt aus den folgenden Formeln, 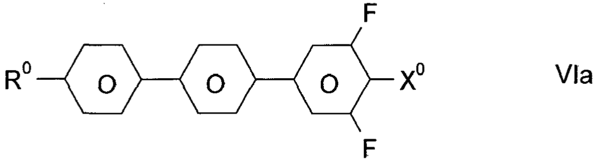 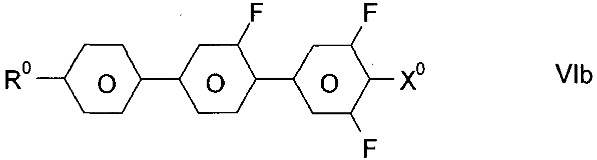 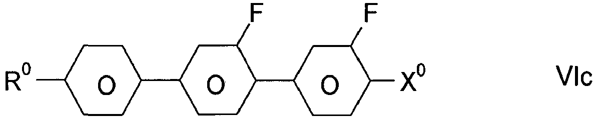 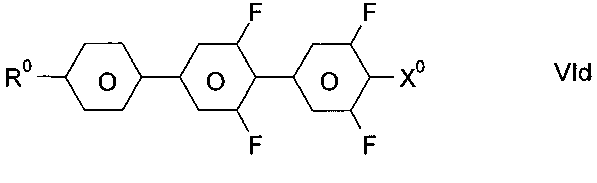 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F, ferner OCF₃, CF₃, CF=CF₂, OCHF₂ und OCH=CF₂;
- Die Verbindungen der Formel VII sind vorzugsweise ausgewählt aus den folgenden Formeln, 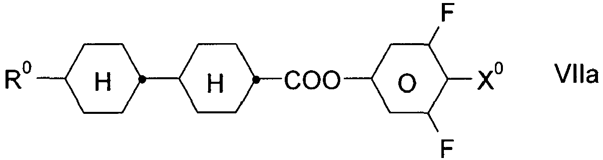 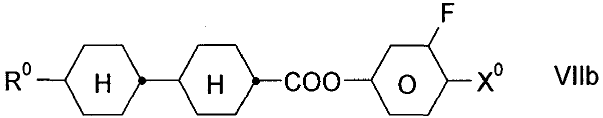 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃, OCHF₂ und OCH=CF₂.
- Das Medium enthält vorzugsweise eine oder mehrere Verbindungen ausgewählt aus den folgenden Formeln, 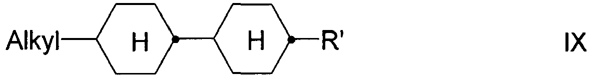 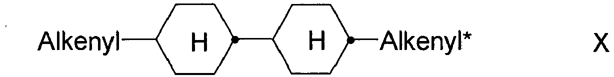 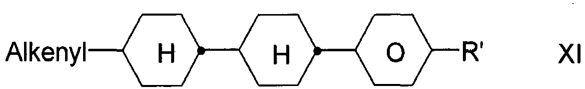 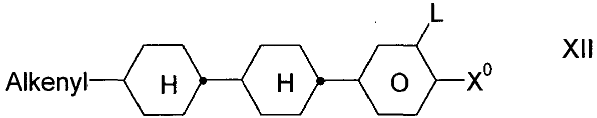 worin X⁰ die oben angegebenen Bedeutungen besitzen, und
   - L: H oder F,
   - "Alkyl": C₁₋₆-Alkyl,
   - R': C₁₋₆-Alkyl, C₁₋₆-Alkoxy oder C₂₋₆-Alkenyl, und
   "Alkenyl" und "Alkenyl*"jeweils unabhängig voneinander C₂₋₆-Alkenyl bedeuten.
- Die Verbindungen der Formeln IX-XII sind vorzugsweise ausgewählt aus folgenden Formeln, 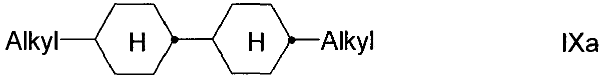 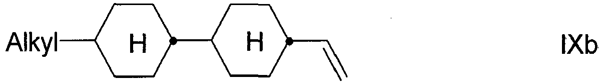 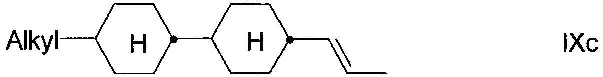 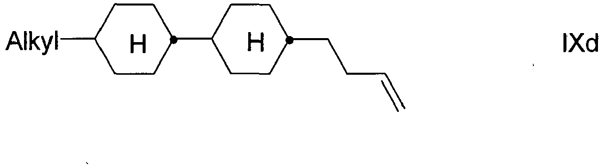 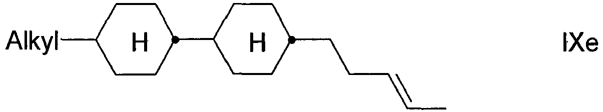 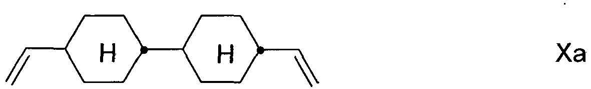 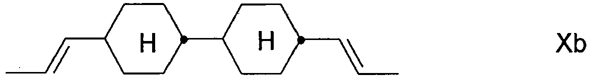 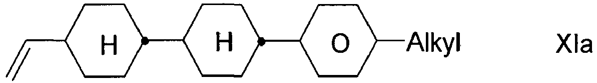 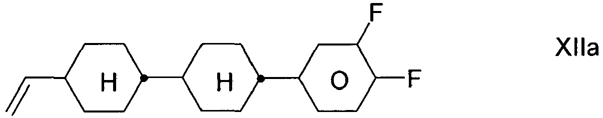 worin "Alkyl" die oben angegebene Bedeutung hat;
   Insbesondere bevorzugt sind die Verbindungen der Formeln XIa, IXb, IXc, Xa, Xb, XIa und XIIa. In den Formeln IXb und IX bedeutet "Alkyl" unabhängig voneinander vorzugsweise n-C₃H₇, n-C₄H₉ oder n-C₅H₁₁, insbesondere n-C₃H₇.
- Das Medium enthält optional eine oder mehrere Verbindungen ausgewählt aus den folgenden Formeln, 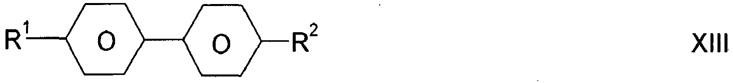 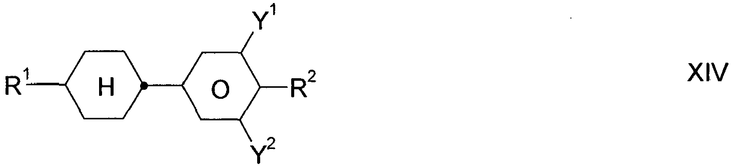 worin Y¹ und Y² die oben angegebenen Bedeutungen haben, und R¹ und R² jeweils unabhängig voneinander n-Alkyl, Alkoxy, Oxaalkyl, Fluoralkyl oder Alkenyl mit jeweils bis zu 6 C-Atomen bedeuten, und vorzugsweise jeweils unabhängig voneinander Alkyl mit 1 bis 6 C-Atomen bedeuten; In der Verbindung der Formel XIII bedeutet vorzugsweise mindestens einer der Reste R¹ und R² Alkenyl mit 2 bis 6 C-Atomen.
- Das Medium enthält vorzugsweise eine oder mehrere Verbindungen der Formel XIII, worin mindestens einer der Reste R¹ und R² Alkenyl mit 2 bis 6 C-Atomen bedeutet, vorzugsweise solche ausgewählt aus folgenden Formeln, 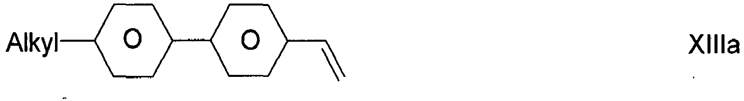 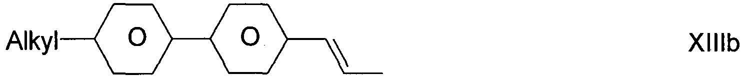 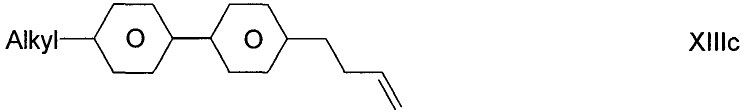 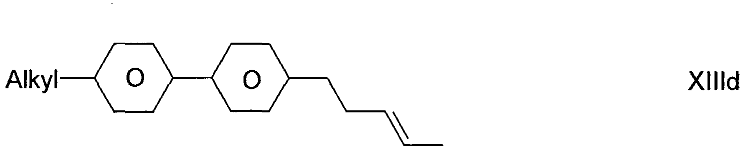
- Das Medium enthält eine oder mehrere Verbindungen der Formel XIIIe, 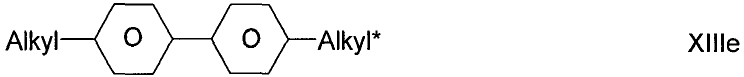 worin "Alkyl" und Alkyl*" die oben angegebenen Bedeutungen haben;
- Das Medium enthält optional eine oder mehrere Verbindungen der Formel XV und/oder XVI 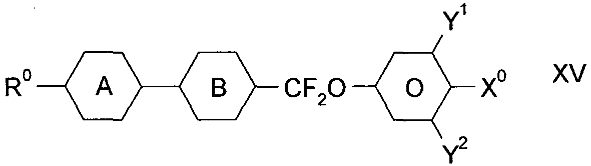 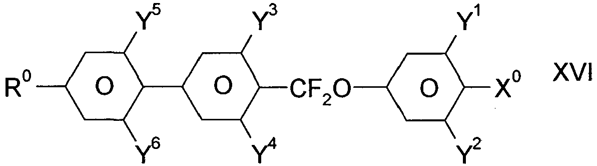 worin R⁰, X⁰ und Y¹⁻⁶ die in Formel II angegebenen Bedeutungen besitzen, und 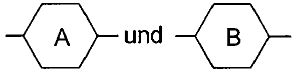 jeweils unabhängig voneinander 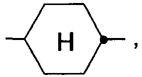 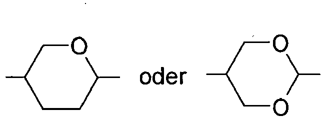 bedeuten.
- Die Verbindungen der Formel XV sind vorzugsweise ausgewählt aus den folgenden Formeln, 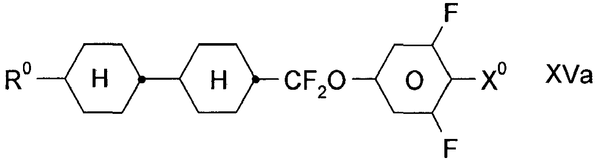 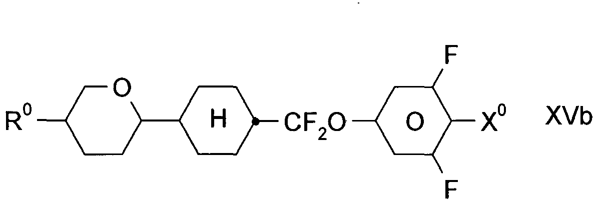 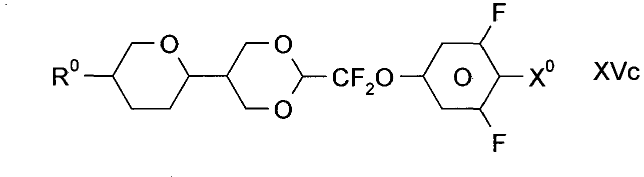 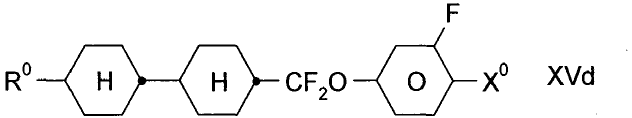 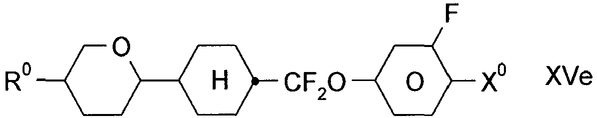 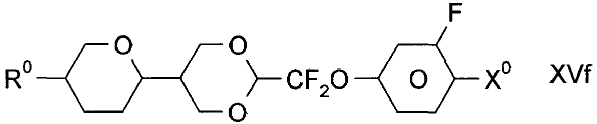 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃ und CF₃. Besonders bevorzugt sind Verbindungen der Formel XVa und XVb, insbesondere Verbindungen der Formeln XVa und XVb, worin X⁰ F bedeutet.
- Die Verbindungen der Formel XVI sind vorzugsweise ausgewählt aus den folgenden Formeln, 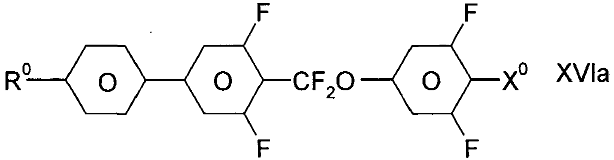 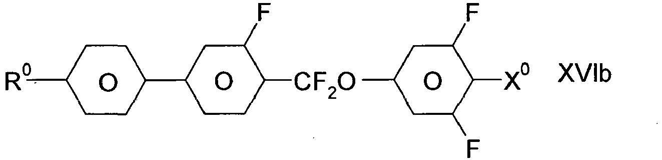 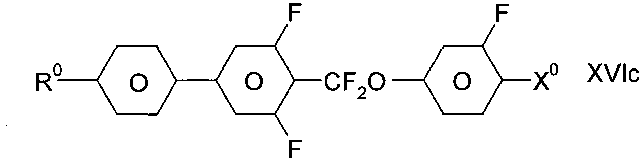 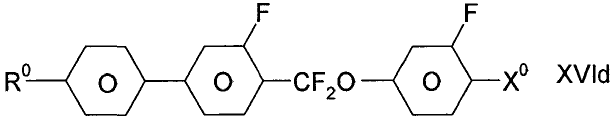 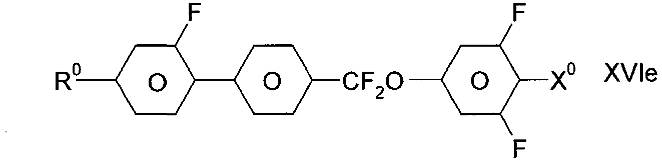 worin R⁰ und X⁰ die oben angegebenen Bedeutungen haben.
   Vorzugsweise bedeutet R⁰ Alkyl mit 1 bis 6 C-Atomen. X⁰ bedeutet vorzugsweise F, ferner OCF₃ und CF₃. Besonders bevorzugt sind Verbindungen der Formeln XVIa und XVIe, insbesondere Verbindungen der Formel XVIa;
- Das Medium enthält vorzugsweise eine oder mehrere Verbindungen der Formel XVII, 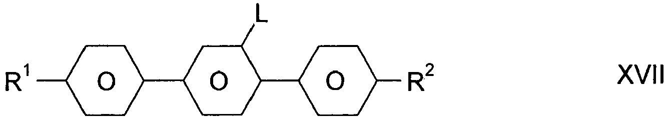 worin R¹ und R² die oben angegebenen Bedeutungen besitzen, und vorzugsweise jeweils unabhängig voneinander Alkyl mit 1 bis 6 C-Atomen bedeuten. L bedeutet H oder F.
   Besonders bevorzugte Verbindungen der Formel XVII sind solche der Unterformeln, 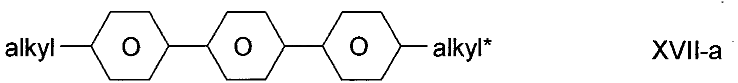 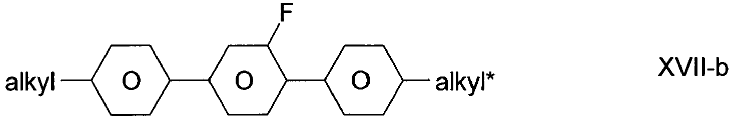 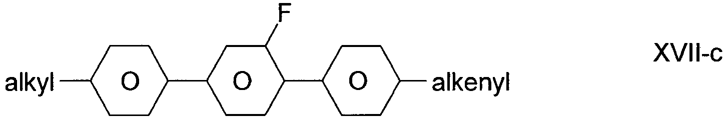 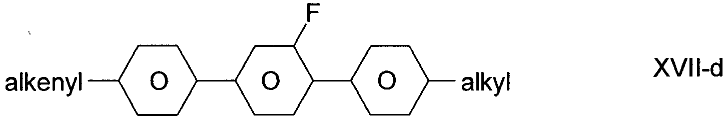 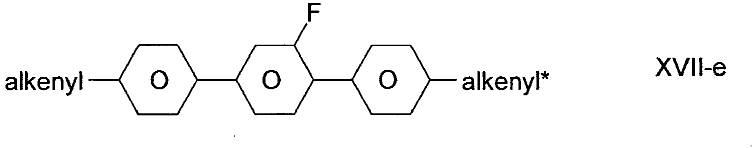 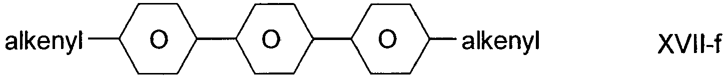 worin
   - alkyl und alkyl*: jeweils unabhängig voneinander geradkettiger Alkylrest mit 1-6 C-Atomen, insbesondere Ethyl, Propyl und Pentyl,
   - alkenyl und alkenyl*: jeweils unabhängig voneinander geradkettiger Alkenylrest mit 2-6 C-Atomen, insbesondere CH₂=CHC₂H₄, CH₃CH=CHC₂H₄, CH₂=CH und CH₃CH=CH,
   bedeuten.
   Besonders bevorzugt sind die Verbindungen der Formeln XVII-b und XVII-c. Ganz besonders bevorzugt sind die Verbindungen der Formeln 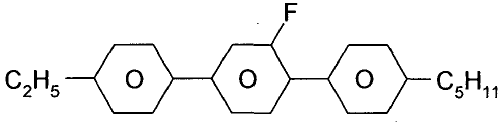 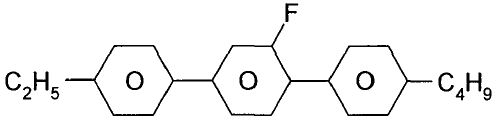 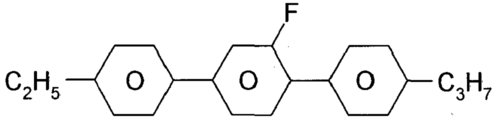 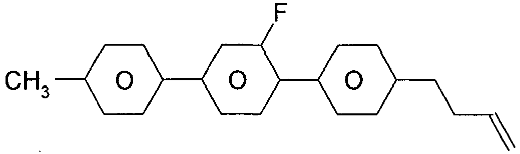 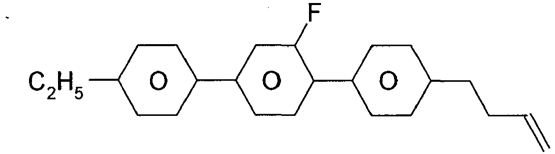 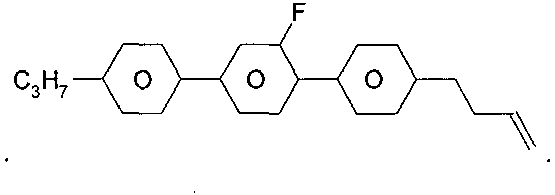
- Das Medium enthält vorzugsweise eine oder mehrere Verbindungen der folgenden Formeln, 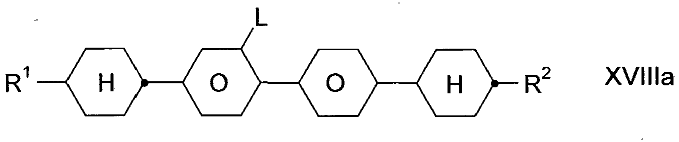 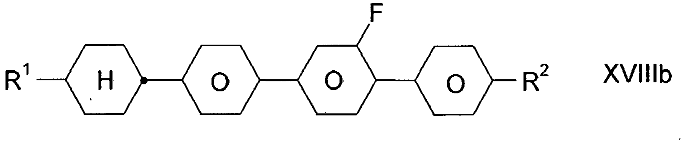 worin R¹ und R² die oben angegebenen Bedeutungen besitzen, und vorzugsweise jeweils unabhängig voneinander Alkyl mit 1 bis 6 C-Atomen bedeuten. L bedeutet H oder F;
- Das Medium enthält optional eine oder mehrere Verbindungen ausgewählt aus den folgenden Formeln, 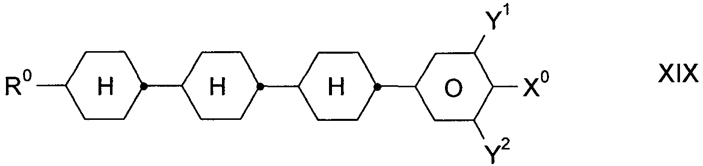 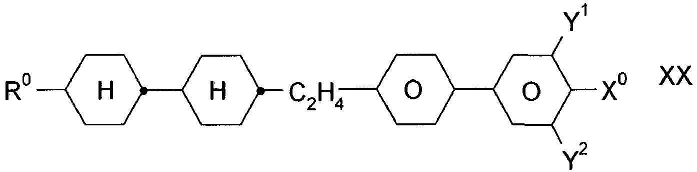 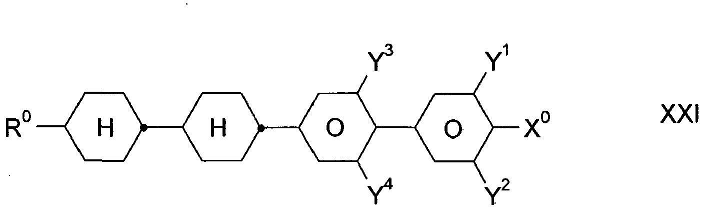 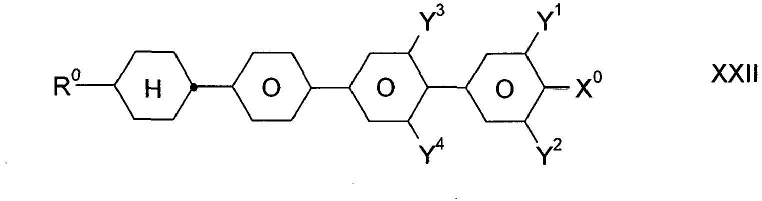 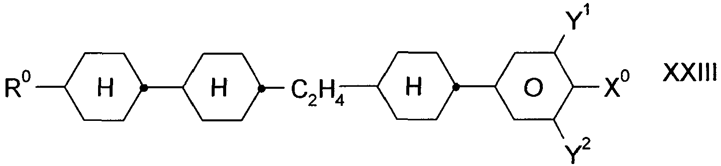 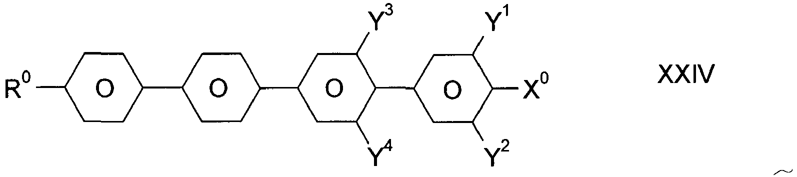 worin R⁰, Y¹⁻⁴ und X⁰ jeweils unabhängig voneinander eine der oben angegebenen Bedeutungen haben. Vorzugsweise bedeutet Y¹⁻⁴ jeweils unabhängig voneinander H oder F. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 6 C-Atomen.
   Besonders bevorzugt erhält die erfindungsgemäße Mischung eine oder mehrere Verbindungen der Formel XXIV-a, 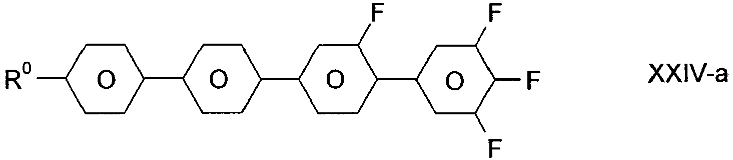 worin R⁰ die oben angegebene Bedeutungen hat. Vorzugsweise bedeutet R⁰ geradkettiges Alkyl, insbesondere Ethyl, n-Propyl, n-Butyl und n-Pentyl, und ganz besonders bevorzugt n-Propyl. Die Verbindung(en) der Formel XXIV-a werden vorzugsweise in Mengen von 0,5-20 Gew.%, besonders bevorzugt 1-15 Gew.% in den erfindungsgemäßen Mischungen eingesetzt.
- Das Medium enthält optional eine oder mehrere Verbindungen aus der Formel XXIV, 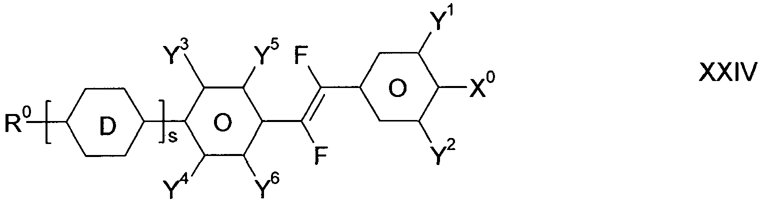 worin R⁰, X⁰ und Y¹⁻⁶ die oben angegebene Bedeutung besitzen, s 0 oder 1, und 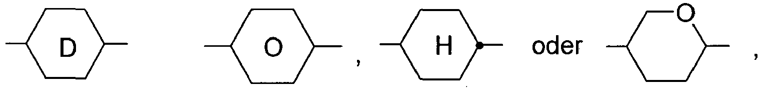 bedeuten.
   In der Formel XXIV kann X⁰ auch ein Alkylrest mit 1-6 C-Atomen oder ein Alkoxyrest mit 1-6 C-Atomen bedeuten. Vorzugsweise ist der Alkyl- oder Alkoxyrest geradkettig.
   Vorzugsweise bedeutet R⁰ Alkyl mit 1 bis 6 C-Atomen. X⁰ bedeutet vorzugsweise F;
- Die Verbindungen der Formel XXIV sind vorzugsweise ausgewählt aus den folgenden Formeln, 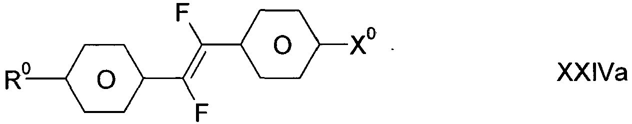 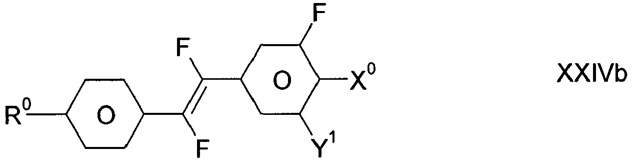 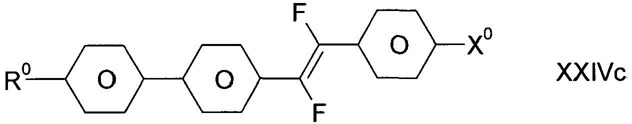 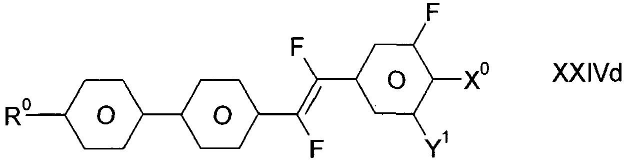 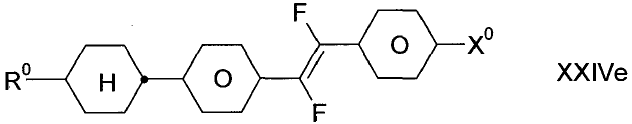 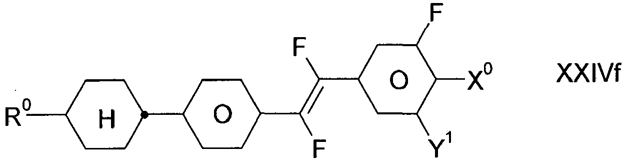 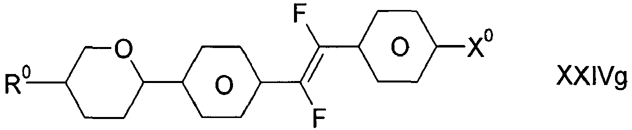 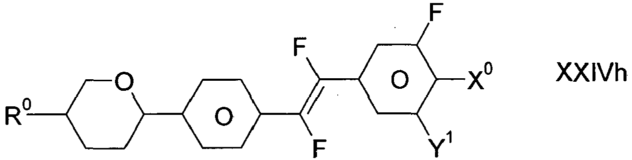 worin R⁰, X⁰ und Y¹ die oben angegebenen Bedeutungen haben. Vorzugsweise bedeutet R⁰ Alkyl mit 1 bis 6 C-Atomen. X⁰ bedeutet vorzugsweise F und Y¹ ist vorzugsweise F; 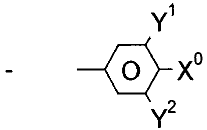 ist vorzugsweise 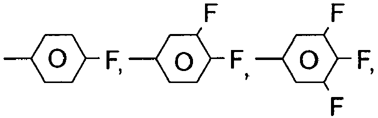 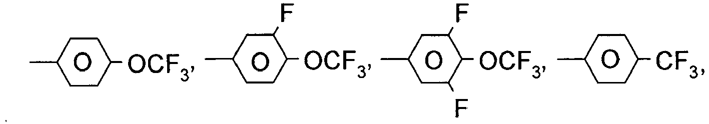 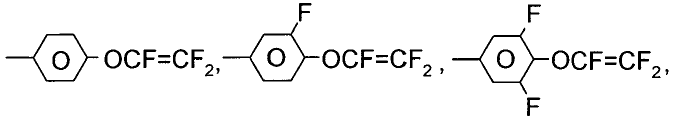 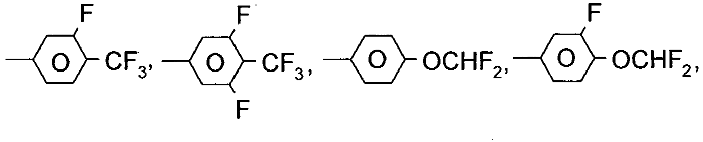 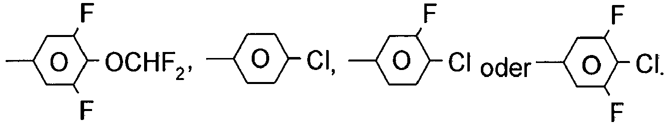
- R⁰ ist geradkettiges Alkyl oder Alkenyl mit 2 bis 6 C-Atomen;
- Das Medium enthält vorzugsweise eine oder mehrere Verbindungen der folgenden Formeln 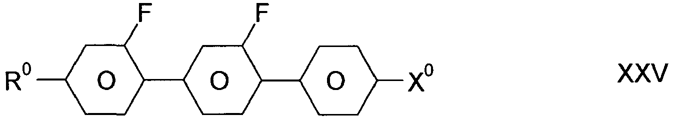 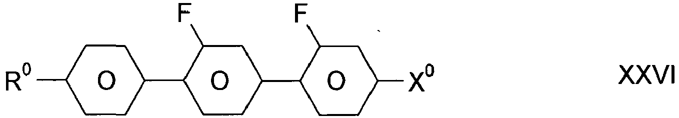 worin R⁰ und X⁰ die oben angegebenen Bedeutungen besitzen. R⁰ bedeutet vorzugsweise Alkyl mit 1 bis 6 C-Atomen. X⁰ bedeutet vorzugsweise F oder Cl. In der Formel XXV bedeutet X⁰ ganz besonders bevorzugt Cl.
- Das Medium enthält vorzugsweise eine oder mehrere Verbindungen der folgenden Formeln, 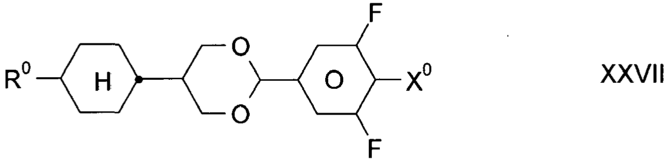 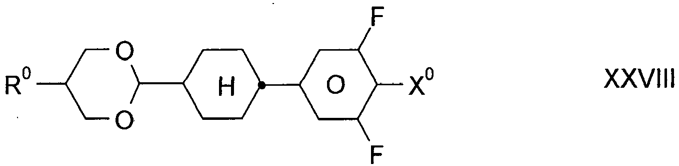 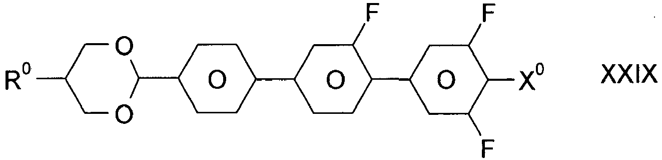 worin R⁰ und X⁰ die oben angegebene Bedeutung besitzen. R⁰ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel XXIX, worin X⁰ vorzugsweise F bedeutet. Die Verbindung(en) der Formeln XXVII - XXIX werden vorzugsweise in Mengen von 1-20 Gew.%, besonders bevorzugt 1-15 Gew.% in den erfindungsgemäßen Mischungen eingesetzt. Besonders bevorzugte Mischungen enthalten mindestens eine Verbindung der Formel XXIX.
- Insbesondere enthält das Medium optional eine oder mehrere Verbindungen der folgenden Formeln, 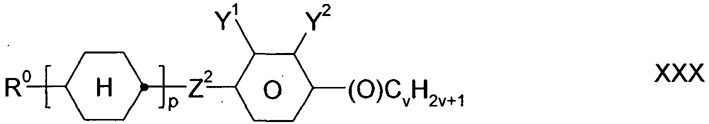 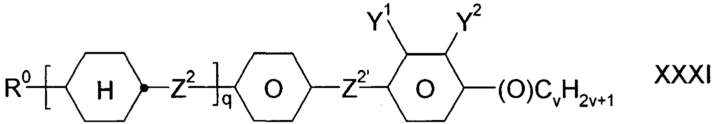 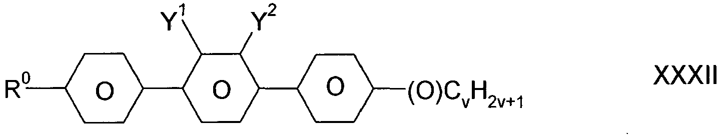 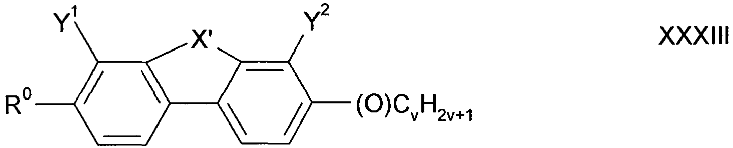 worin
   - R⁰, X⁰, Y¹ und Y²: die oben angegebenen Bedeutungen haben,
   - X': -O- oder -S-
   - Z² und Z^{2'}: jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
   - p: 0, 1 oder 2,
   - q: 0 oder 1, und
   - v: 1 bis 6
   bedeuten.
   In den Verbindungen der Formeln XXX und XXXI können Z² gleiche oder unterschiedliche Bedeutungen haben. In den Verbindungen der Formel XXXI können Z² und Z^{2'} gleiche oder verschiedene Bedeutungen aufweisen.
   In den Verbindungen der Formeln XXX, XXXI, XXXII und XXXIII bedeuten R⁰ jeweils vorzugsweise Alkyl mit 1-6 C-Atomen, insbesondere CH₃, C₂H₅, n-C₃H₇, n-C₄H₉, n-C₅H₁₁.
   In den Verbindungen der Formeln XXXIII bedeutet R⁰ zudem vorzugsweise Alkoxy mit 1-6 C-Atomen, insbesondere -OCH₃, -OC₂H₅, -OC₃H₇, -OC₄H₉, -OC₅H₁₁.
   In den Verbindungen der Formeln XXXI, XXXII und XXXIII bedeuten Y¹ und vorzugsweise Y¹ = Y² = F, Y¹ = F und Y² = Cl, Y¹ = Cl und Y² = F.
   Z² und Z^{2'} bedeuten in den Formeln XXX und XXXI vorzugsweise jeweils unabhängig voneinander eine Einfachbindung, ferner eine - C2H4-Brücke.
   Sofern in der Formel XXXI Z² = -C₂H₄- ist, ist Z^{2'} vorzugsweise eine Einfachbindung bzw. falls Z^{2'} = -C₂H₄- bedeutet, ist Z² vorzugsweise eine Einfachbindung. In den Verbindungen der Formeln XXX und XXXI bedeutet (O)CᵥH₂ᵥ₊₁ vorzugsweise OCᵥH₂ᵥ₊₁, ferner CᵥH₂ᵥ₊₁. In den Verbindungen der Formel XXXII und XXXIII bedeutet (O)CᵥH₂ᵥ₊₁ vorzugsweise CᵥH₂ᵥ₊₁ und in den Verbindungen der Formel XXXIII vorzugsweise OCᵥH₂ᵥ₊₁. In den Verbindungen der Formel XXXII und XXXIII bedeuten Y¹ und Y² vorzugsweise jeweils F.
- Das Medium enthält optional eine oder mehrere Verbindungen der folgenden Pyrimidin- oder Pyridin-Verbindungen der Formeln, 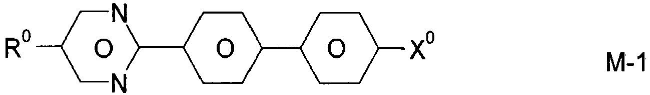 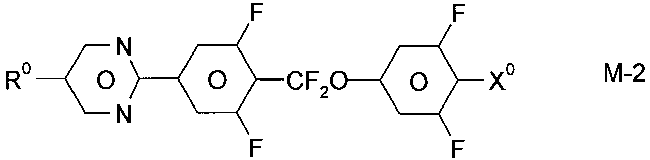 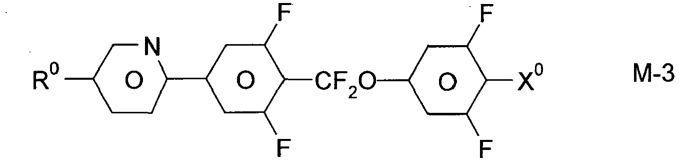 worin R⁰ und X⁰ die oben angegebene Bedeutung besitzen. R⁰ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel M-1, worin X⁰ vorzugsweise F bedeutet. Die Verbindung(en) der Formeln M-1 bis M-3 werden vorzugsweise in Mengen von 1-20 Gew.%, besonders bevorzugt 1-15 Gew.% in den erfindungsgemäßen Mischungen eingesetzt.

Nachfolgend werden weitere bevorzugte Ausführungsformen angegeben:
Die erfindungsgemäßen Mischungen enthalten vorzugsweise
- die Verbindung der Formel I, worin A¹ vorzugsweise 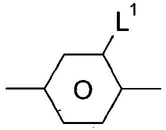 mit L¹ = F und R¹ = R^{1*} = Alkoxy bedeuten,
- Das Medium enthält neben einer oder mehreren Verbindungen der Formeln I ein oder mehrere Verbindungen ausgewählt aus der Gruppe der Verbindungen der Formeln II, III, IX-XIII, XVII und XVIII;
- Der Anteil an Verbindungen der Formeln II, III, IX-XIII, XVII und XVIII im Gesamtgemisch beträgt 40 bis 99 Gew.%;
- Das Medium enthält vorzugsweise 3-50 Gew.%, besonders bevorzugt 5-40 Gew.%, insbesondere bevorzugt 8-30 Gew.% an einer, zwei, drei oder mehreren Verbindungen der Formel II und/oder III;
- Das Medium enthält vorzugsweise 3-60 Gew.%, besonders bevorzugt 5-50 Gew.%, insbesondere bevorzugt 10-40 Gew.% an Verbindungen der Formeln IX-XIII;
- Das Medium enthält vorzugsweise 1-40 Gew.%, besonders bevorzugt 5-30 Gew.% an Verbindungen der Formel XVII;
- Das Medium enthält vorzugsweise 3-50 Gew.%, besonders bevorzugt 5-40 Gew.% an Verbindungen der Formel XVIII.
- Das Medium enthält vorzugsweise mindestens zwei Verbindungen der Formeln 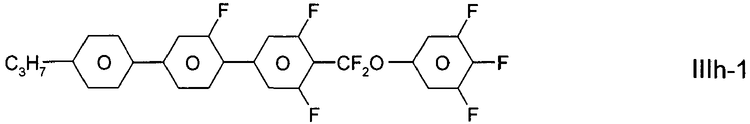 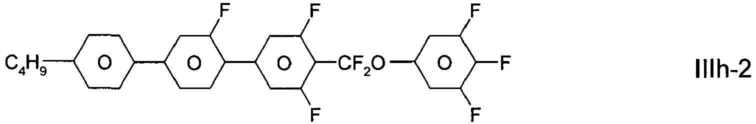 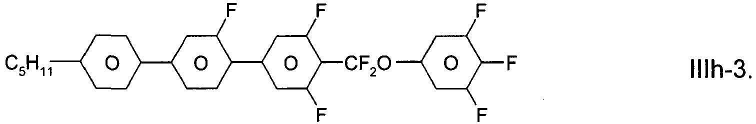
- Das Medium enthält vorzugsweise mindestens zwei Verbindungen der Formeln 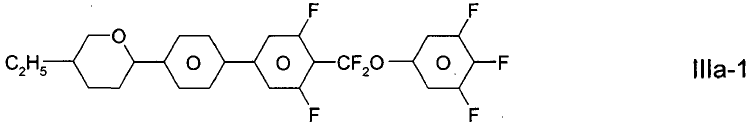 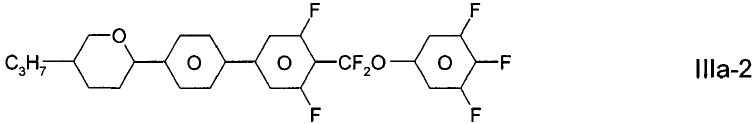 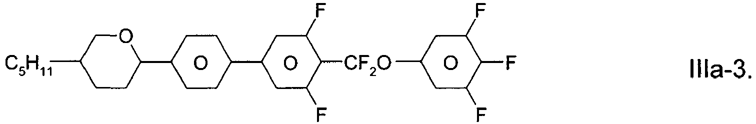
- Das Medium enthält mindestens eine Verbindungen der Formel I und vorzugsweise mindestens eine Verbindung der Formel IIIh-2;
- Das Medium enthält vorzugsweise ≥ 3 Gew.%, besonders bevorzugt ≥ 5 Gew.%, insbesondere 3-60 Gew.%, an Verbindungen der Formel IXb, insbesondere die Verbindung der Formel IXb-1, 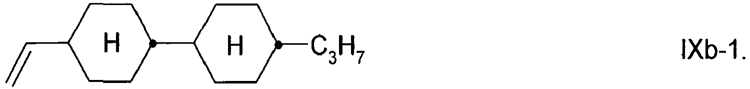
- Das Medium enthält vorzugsweise mindestens eine Verbindung der Formel IXb-1 und vorzugsweise mindestens eine Verbindung der Formel XIII-d.
- Das Medium enthält vorzugsweise mindestens eine Verbindung der Formel PUQU-n-F.
- Das Medium enthält mindestens eine Verbindung der Formel APUQU-n-F.
- Das Medium enthält mindestens eine Verbindung der Formel CPGP-n-m.
- Das Medium enthält mindestens eine Verbindung der Formel PGP-n-m, vorzugsweise zwei oder drei Verbindungen.
- Das Medium enthält mindestens eine Verbindung der Formel CCP-3-OT mit der Struktur 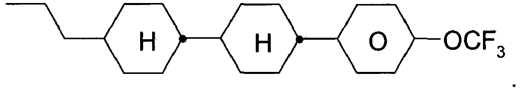
- Das Medium enthält mindestens eine Verbindung der Formel CCP-V-1 mit der Struktur 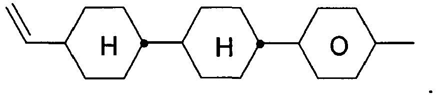
- Das Medium enthält mindestens eine Verbindung der Formel PGP-2-2V mit der Struktur

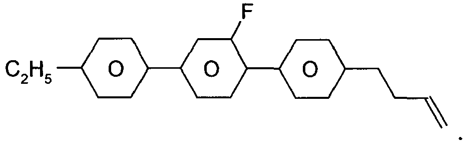

Es wurde gefunden, dass ≥ 2 Gew.% einer oder mehrerer Verbindungen der Formel I im Gemisch mit üblichen Flüssigkristallmaterialien, insbesondere jedoch mit einer oder mehreren Verbindungen der Formeln II bis XXVIII zu einer beträchtlichen Erhöhung der Lichtstabilität und zu hohen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr niedrige Schwellenspannungen und sehr gute Werte für die VHR bei UV-Belastung und sehr hohe Klärpunkte.

Der Ausdruck "Alkyl" bzw. "Alkyl*" oder "alkyl" bzw. "alkyl*" umfasst in dieser Anmeldung geradkettige und verzweigte Alkylgruppen mit 1-6 Kohlenstoffatomen, insbesondere die geradkettigen Gruppen Methyl, Ethyl, Propyl, Butyl, Pentyl und Hexyl. Gruppen mit 2-5 Kohlenstoffatomen sind im allgemeinen bevorzugt.

Der Ausdruck "Alkenyl" bzw. "Alkenyl*" umfasst geradkettige und verzweigte Alkenylgruppen mit 2-6 Kohlenstoffatomen, insbesondere die geradkettigen Gruppen. Bevorzugte Alkenylgruppen sind C₂-C₇-1E-Alkenyl, C₄-C₆-3E-Alkenyl, insbesondere C₂-C₆-1E-Alkenyl. Beispiele besonders bevorzugter Alkenylgruppen sind Vinyl, 1 E-Propenyl, 1 E-Butenyl, 1 E-Pentenyl, 1E-Hexenyl, 3-Butenyl, 3E-Pentenyl, 3E-Hexenyl, 4-Pentenyl, 4Z-Hexenyl, 4E-Hexenyl und 5-Hexenyl. Gruppen mit bis zu 5 Kohlenstoffatomen sind im allgemeinen bevorzugt, insbesondere CH₂=CH, CH₃CH=CH, CH₃CH₂CH₂CH₂=CH oder CH₃ CH₂CH₂=CH.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst vorzugsweise geradkettige Reste der Formel CₙH₂ₙ₊₁-O-(CH₂)m, worin n und m jeweils unabhängig voneinander 1 bis 6 bedeuten. m kann auch 0 bedeuten. Vorzugsweise ist n = 1 und m 1-6 oder m = 0 und n = 1-3.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Mischungen zeichnen sich insbesondere durch hohe Δn-Werte aus und besitzen somit deutlich schnellere Schaltzeilen als die Mischungen aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

Eine günstige synergistische Wirkung von Verbindungen der Formeln II bis XXVIII mit einer oder mehreren Verbindungen der Formel I führt zu besonders vorteilhaften Eigenschaften. Beispielsweise Mischungen enthaltend eine oder mehrere Verbindungen der Formel I, XVa und/oder XVIa zeichnen sich durch ihre niedrige Schwellenspannung aus.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und TFT-Anwendungen, wie z. B. Mobiltelefone und PDAs geeignet. Weiterhin können die erfindungsgemäßen Mischungen in FFS-, VA-IPS, OCB- und IPS-Anzeigen Anwendung finden.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥ 75 °C, gleichzeitig Rotationsviskositäten γ₁ von ≤ 110 mPa·s, besonders bevorzugt ≤ 100 mPa·s zu erreichen, wodurch hervorragende MFK-Anzeigen mit schnellen Schaltzeiten erzielt werden können. Die Rotationsviskositäten sind bei 20 °C bestimmt.

Die erfindungsgemäßen Flüssigkristallmischungen weisen insbesondere hohe Werte für ε_{⊥} bei 20 °C auf, welche vorzugsweise ≥ +2,5, weiter bevorzugt ≥ +3, besonders bevorzugt ≥ +3,5 sind.

Die dielektrische Anisotropie der erfindungsgemäßen Flüssigkristallmischungen Δε ist bei 20 °C vorzugsweise ≥ +1,5, weiter bevorzugt ≥ +3, besonders bevorzugt ≥ +5, insbesondere bevorzugt > +8.

Die Mischungen sind außerdem durch kleine Operationsspannungen gekennzeichnet. Die Schwellenspannung der erfindungsgemäßen Flüssigkristallmischungen ist vorzugsweise ≤ 2.0 V.

Die Doppelbrechung Δn der erfindungsgemäßen Flüssigkristallmischungen ist bei 20 °C vorzugsweise ≥ 0,09, besonders bevorzugt ≥ 0,10.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -25° bis +70°C.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht. Die Herstellung der erfindungsgemäß verwendbaren Flüssigkristallmischungen erfolgt in an sich üblicher Weise, beispielsweise indem man eine oder mehrere Verbindungen der Formeln I mit einer oder mehreren Verbindungen der Formeln II-XXVIII oder mit weiteren flüssigkristallinen Verbindungen und ggf. mit Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin®, z.B. Tinuvin® 770, der Fa. Ciba Chemicals, Antioxidantien, z.B. TEMPOL, Mikropartikel, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Den erfindungsgemäßen Mischungen können weiterhin polymerisierbare Verbindungen, sogenannte reaktive Mesogene (RMs), beispielsweise wie in U.S. 6,861,107 offenbart, in Konzentrationen von bevorzugt 0,12 - 5 Gew.%, besonders bevorzugt 0,2 - 2 % bezogen auf die Mischung, zugesetzt werden. Optional können diese Mischungen auch einen Initiator enthalten, wie beispielsweise in der U.S 6,781,665 beschrieben. Der Initiator, z.B. Irganox-1076 der Fa. Ciba, wird vorzugsweise der Mischung enthaltend polymerisierbare Verbindungen in Mengen von 0-1 % zugesetzt. Derartige Mischungen können für sogenannte Polymer Stabilized VA-Modes (PS-VA) oder PSA (Polymer sustained VA), bei denen eine Polymerisierung der reaktiven Mesogene in der flüssigkristallinen Mischung erfolgen soll, verwendet werden. Voraussetzung hierfür ist, dass die Flüssigkristallmischung selbst keine polymerisierbaren Komponenten enthält.

In einer bevorzugten Ausführungsform der Erfindung sind die polymerisierbaren Verbindungen ausgewählt aus den Verbindungen der Formel M

R^{a}-A¹-(Z¹-A²)ₘ-R^{b} M

worin die einzelnen Reste folgende Bedeutung haben:
- R^{a} und R^{b}: jeweils unabhängig voneinander P, P-Sp-, H, Halogen, SF₅, NO₂, eine Kohlenstoffgruppe oder Kohlenwasserstoffgruppe, wobei mindestens einer der Reste R^{a} und R^{b} eine Gruppe P oder P-Sp- bedeutet oder enthält,
- P: bei jedem Auftreten gleich oder verschieden eine polymerisierbare Gruppe,
- Sp: bei jedem Auftreten gleich oder verschieden eine Abstandsgruppe oder eine Einfachbindung,
- A¹ und A²: jeweils unabhängig voneinander eine aromatische, heteroaromatische, alicyclische oder heterocyclische Gruppe, vorzugsweise mit 4 bis 25 Ringatomen, welche auch anellierte Ringe enthalten kann, und welche auch durch L ein- oder mehrfach substituiert sein kann,
- L: P-Sp-, H, OH, CH₂OH, Halogen, SF₅, NO₂, eine Kohlenstoffgruppe oder Kohlenwasserstoffgruppe,
- Z¹: bei jedem Auftreten gleich oder verschieden -O-, -S-, -CO-, -CO-O-, -OCO-, -O-CO-O-, -OCH₂-, -CH₂O-, -SCH₂-, -CH₂S-, -CF₂O-, -OCF₂-, -CF₂S-, -SCF₂-, -(CH₂)ₙ₁-, -CF₂CH₂-, -CH₂CF₂-, -(CF₂)ₙ₁-, -CH=CH-, -CF=CF-, -C≡C-, -CH=CH- COO-, -OC-O-CH≡CH-, CR⁰R⁰⁰ oder eine Einfachbindung,
- R⁰ und R⁰⁰: jeweils unabhängig voneinander H oder Alkyl mit 1 bis 12 C-Atomen,
- m: 0, 1, 2, 3 oder 4,
- n1: 1, 2, 3 oder 4.

Besonders bevorzugte Verbindungen der Formel M sind solche, worin
- R^{a} und R^{b}: jeweils unabhängig voneinander P, P-Sp-, H, F, Cl, Br, l, -CN, - NO₂, -NCO, -NCS, -OCN, -SCN, SF₅ oder geradkettiges oder verzweigtes Alkyl mit 1 bis 25 C-Atomen, worin auch eine oder mehrere nicht benachbarte CH₂-Gruppen jeweils unabhängig voneinander durch -C(R⁰)=C(R⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, l, CN, P oder P-Sp- ersetzt sein können, wobei mindestens einer der Reste R^{a} und R^{b} eine Gruppe P oder P-Sp- bedeutet oder enthält,
- A¹ und A²: jeweils unabhängig voneinander 1,4-Phenylen, Naphthalin-1,4-diyl, Naphthalin-2,6-diyl, Phenanthren-2,7-diyl, Anthracen-2,7-diyl, Fluoren-2,7-diyl, 2-Oxo-2H-chromen-3,6-diyl, 2-Oxo-2H-Chromen-3,7-diyl, 4-Oxo-4H-chromen-2,6-diyl, 4-Oxo-4H-chromen-3,6-diyl, 4-Oxo-4H-chromen-3,7-diyl (Trivialname Cumarin bzw. Flavon), wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, wobei alle diese Gruppen unsubstituiert oder durch L ein- oder mehrfach substituiert sein können,L P, P-Sp-, OH, CH₂OH, F, Cl, Br, l, -CN, -NO₂, -NCO, -NCS, -OCN, -SCN, -C(=O)N(R^{x})2, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können,
- P: eine polymerisierbare Gruppe,
- Y¹: Halogen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen,
bedeuten.

Weitere bevorzugte Verbindungen der Formel M sind solche ausgewählt aus einer oder mehreren der folgenden Untergruppen:
- m ist 1, 2 oder 3,
- m ist 1 oder 2,
- R^{a} und R^{b} bedeuten gleiche oder verschiedene Gruppen P-Sp-,
- R^{a} und R^{b} bedeuten gleiche oder verschiedene Gruppen P-Sp-, worin ein oder mehrere Gruppen Sp eine Einfachbindung bedeuten,
- m ist 2 oder 3 und R^{a} und R^{b} bedeuten gleiche Gruppen P-Sp-,
- einer der Reste R^{a} und R^{b} bedeutet P-Sp- und der andere bedeutet eine unpolymerisierbare Gruppe, vorzugsweise geradkettiges oder verzweigtes Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO- oder -O-CO-O- so ersetzt sein können, dass O- und/oder S-Atome nicht direkt miteinander verknüpft sind, und worin auch ein oder mehrere H-Atome durch F, Cl, Br, l oder CN ersetzt sein können,
- eine oder mehrere Gruppen Sp bedeuten eine Einfachbindung,
- eine oder mehrere Gruppen Sp bedeuten -(CH₂)ₚ₁-, -(CH₂)ₚ₁-O-, -(CH₂)ₚ₁-OCO- oder -(CH₂)ₚ₁-OCOO-, worin p1 eine ganze Zahl von 1 bis 12 und r1 eine ganze Zahl von 1 bis 8 bedeuten,
- L bedeutet und/oder enthält keine polymerisierbare Gruppe,
- A¹ und A² bedeuten unabhängig voneinander 1,4-Phenylen oder Naphthalin-2,6-diyl, wobei in diesen Gruppen auch eine oder mehrere CH-Gruppen durch N ersetzt sein können, und welche auch ein-oder mehrfach fluoriert sein können,
- Z¹ ist ausgewählt aus der Gruppe bestehend aus -O-, -CO-O-, -OCO-, -OCH₂-, -CH₂O-, -CF₂O-, -OCF₂-, -CH₂CH₂-, -CH=CH-, -CF=CF-, -CH=CF-, -CF=CH-, -C≡C- und einer Einfachbindung,
- L ist eine unpolymerisierbare Gruppe, vorzugsweise ausgewählt aus der Gruppe bestehend aus F, Cl, -CN, geradkettigem und verzweigtem Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO- oder -O-CO-O- so ersetzt sein können, dass O- und/oder S-Atome nicht direkt miteinander verknüpft sind, und worin auch ein oder mehrere H-Atome durch F, Cl, Br, l oder CN ersetzt sein können.

Geeignete und bevorzugte Co-Monomere für die Herstellung von erfindungsgemäßen Mischungen für PS-VA-, PS-IPS und PS-FFS-Anwendungen sind beispielsweise ausgewählt aus den folgenden Formeln: 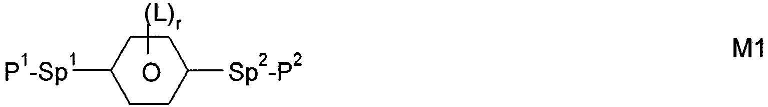 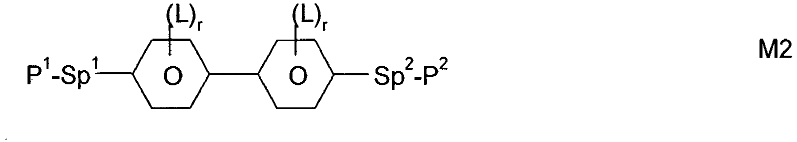 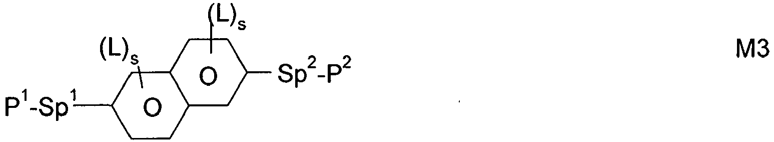 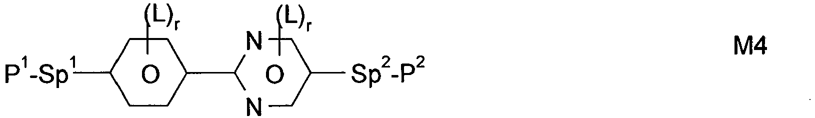 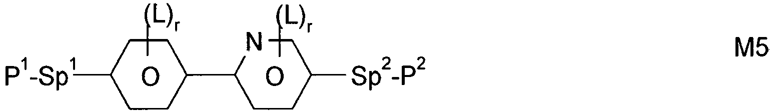 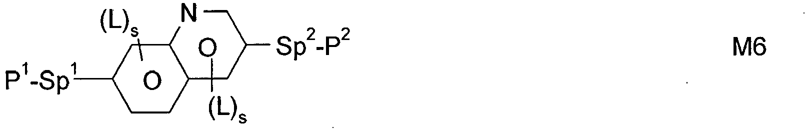 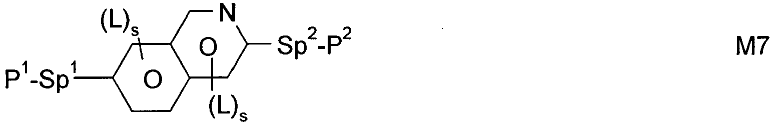 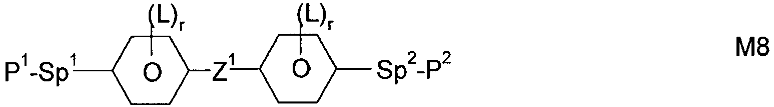 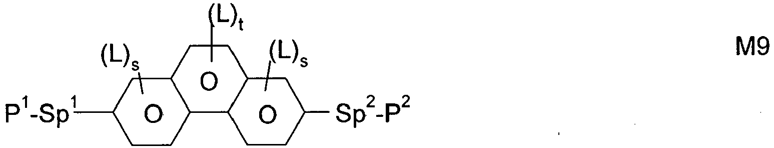 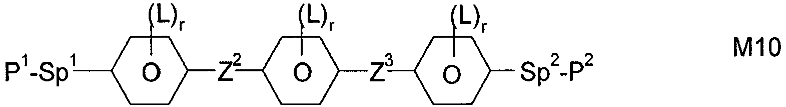 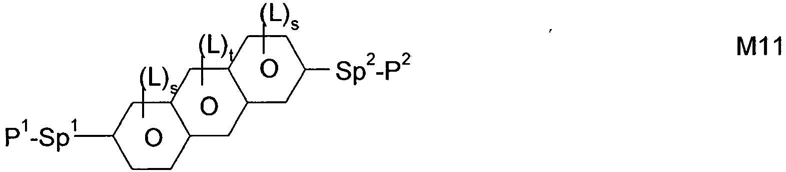 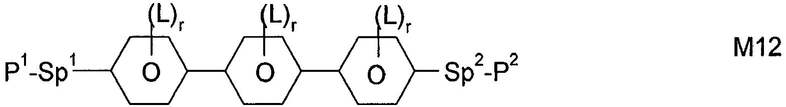 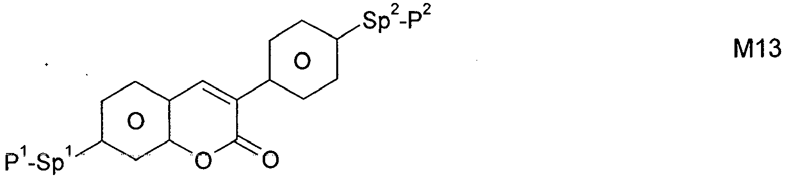 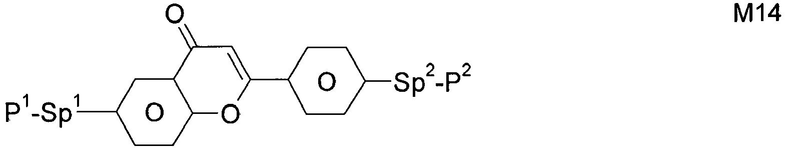 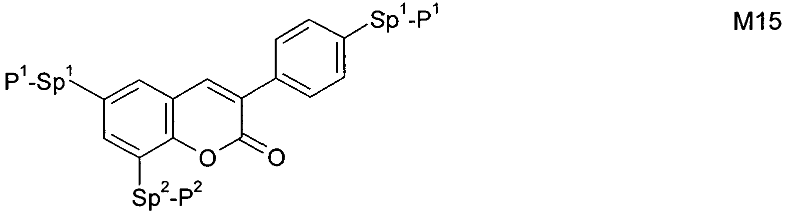 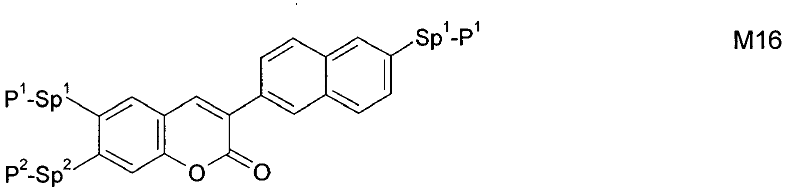 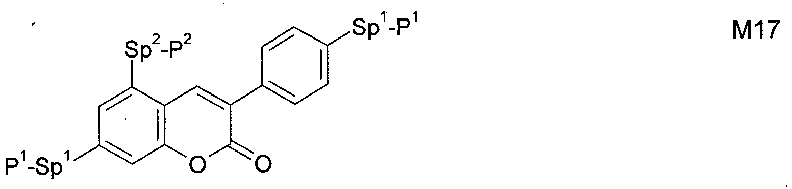 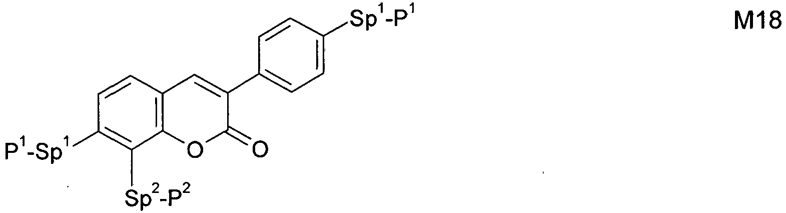 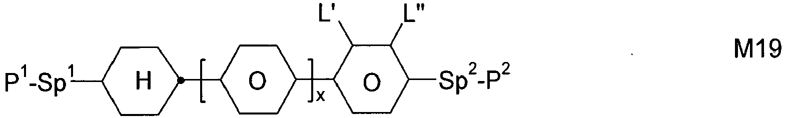 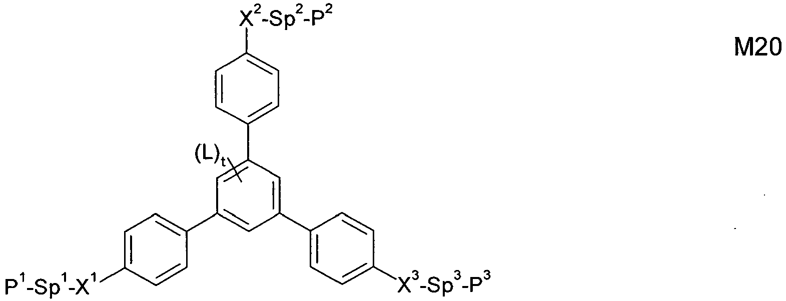 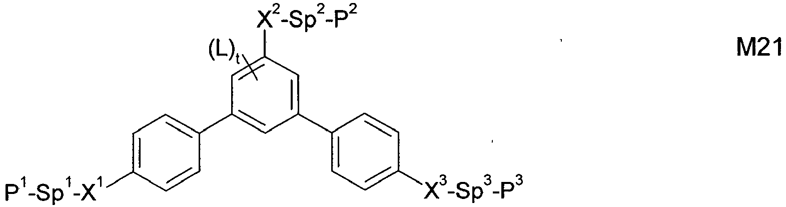 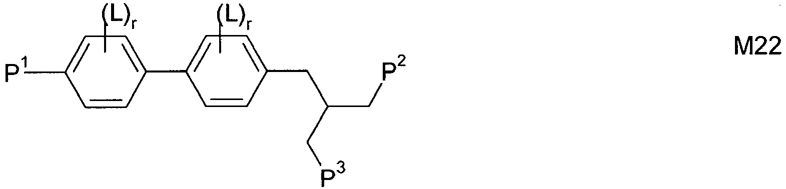 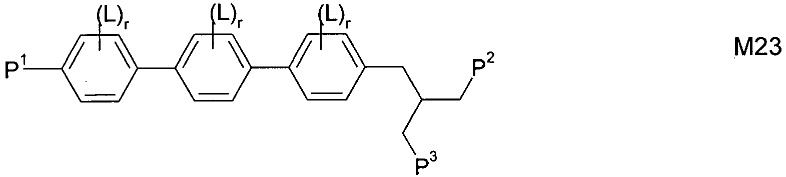 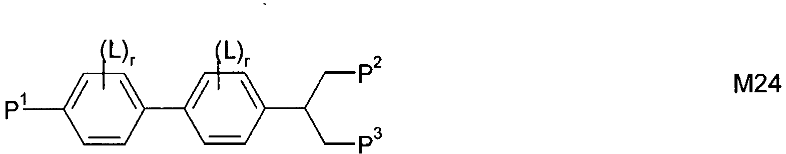 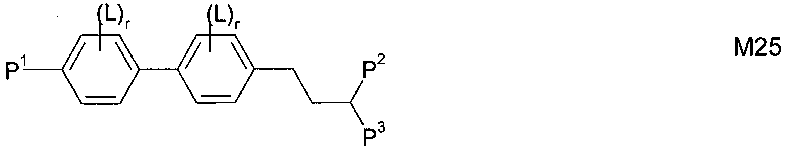 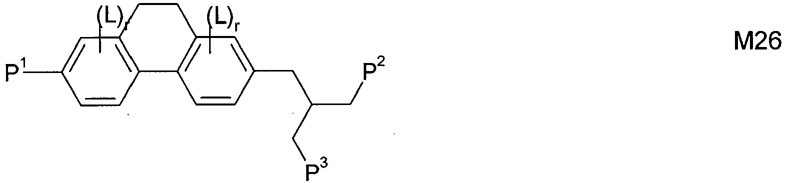 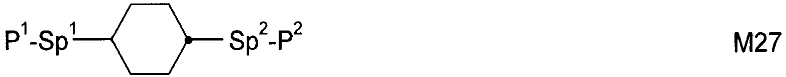 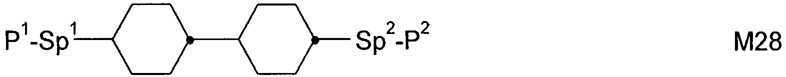 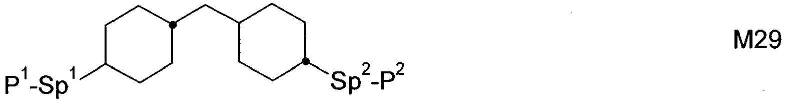 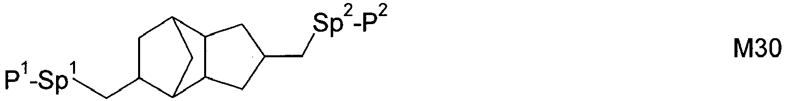 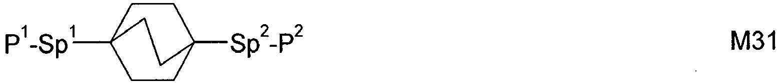 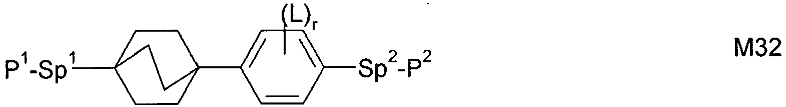 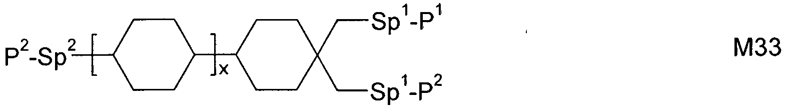 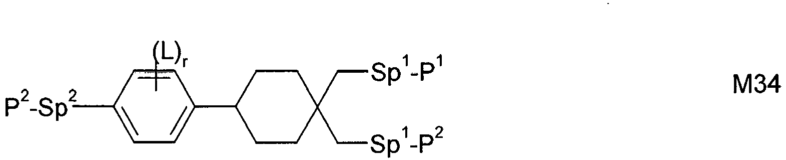 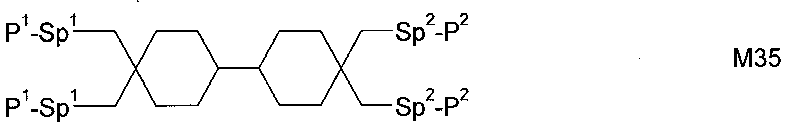 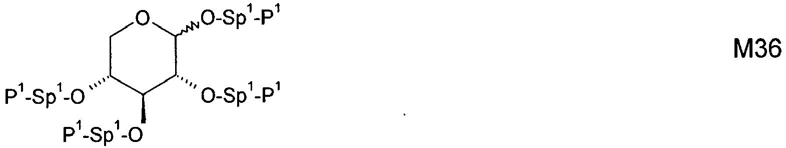 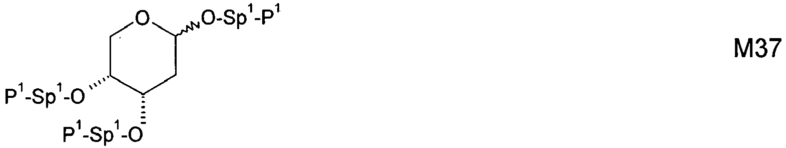 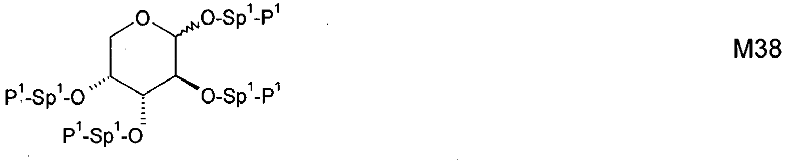 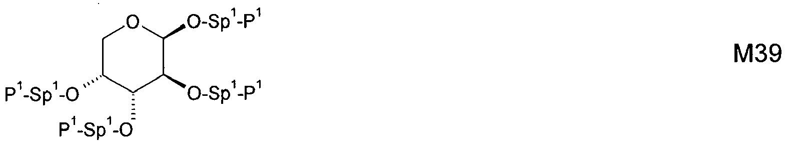 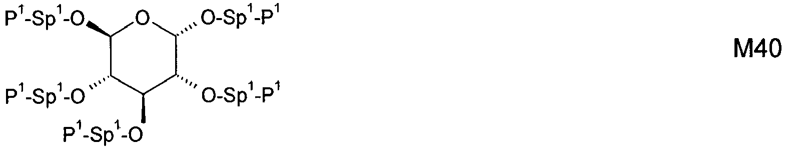 worin die einzelnen Reste folgende Bedeutung besitzen:
- P¹, P² und P³: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹, Sp² und Sp³: jeweils unabhängig voneinander eine Einfachbindung oder eine Abstandsgruppe, vorzugsweise mit einer der vor- und nachstehend für Sp^{a} angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt, wobei auch einer oder mehrere der Reste P¹-Sp¹-, P²-Sp²- und P³-Sp³- einen Rest R^{aa} bedeuten können, mit der Maßgabe dass mindestens einer der vorhandenen Reste P¹-Sp¹-, P²-Sp²- und P³-Sp³- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹⁻Sp¹-ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- X¹, X² und X³: jeweils unabhängig voneinander -CO-O-, -Ö-CO- oder eine Einfachbindung,
- Z¹: -O-, -CO-, -C(R^{y}R^{z})-,oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen, vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

In den Verbindungen der Formeln M1 bis M34 bedeutet 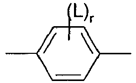 vorzugsweise 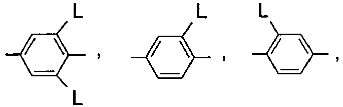 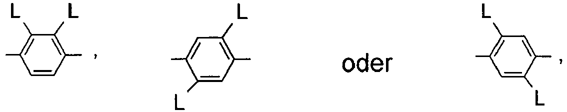 worin L, bei jedem Auftreten gleich oder verschieden, eine der vorstehenden Bedeutungen hat und vorzugsweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃, insbesondere F oder CH₃ bedeutet.

Bevorzugt enthalten die flüssigkristallinen Medien gemäß der vorliegenden Anmeldung insgesamt 0,01 bis 10 %, bevorzugt 0,2 bis 4,0 %, besonders bevorzugt 0,2 bis 2,0 %, an polymerisierbaren Verbindungen.

Insbesondere bevorzugt sind die polymerisierbaren Verbindungen der Formel M, ganz besonders bevorzugt sind die polymerisierbaren Verbindungen ausgewählt aus Tabelle F.

Gegenstand der vorliegenden Erfindung ist auch die Verwendung der erfindungsgemäßen Mischungen in elektrooptischen Anzeigen sowie der Einsatz der erfindungsgemäßen Mischungen in Shutter-Brillen, insbesondere für 3D-Anwendungen, und in TN-, PS-TN-, STN-, TN-TFT-, OCB-, IPS-, PS-IPS-, FFS-, PS-FFS- und PS-VA-IPS-Anzeigen.

Für die vorliegende Erfindung bedeutet "≤" kleiner oder gleich, bevorzugt kleiner und "≥" größer oder gleich, bevorzugt größer.

Für die vorliegende Erfindung bedeuten 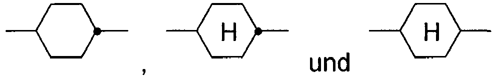 trans-1,4-Cyclohexylen und 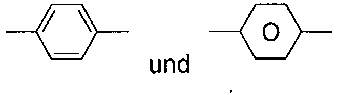 1,4-Phenylen.

Für die vorliegende Erfindung bedeuten die Begriffe "dielektrisch positive Verbindungen" solche Verbindungen mit einem Δε > 1,5, "dielektrisch neutrale Verbindungen" solche mit -1,5 ≤ Δε ≤ 1,5 und "dielektrisch negative" Verbindungen solche mit Δε < -1,5. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 µm Schichtdicke mit homeotroper und mit homogener Oberflächenorientierung bei 1 kHz bestimmt wird. Die Messspannung beträgt typischerweise 0,5 V bis 1,0 V, sie ist jedoch stets niedriger als die kapazitive Schwelle der jeweiligen untersuchten Flüssigkristallmischung.

Als Hostmischung für dielektrisch positive und dielektrisch neutrale Verbindungen wird ZLI-4792 und für dielektrisch negative Verbindungen ZLI-2857, beide von Merck KGaA, Deutschland, verwendet. Aus der Änderung der Dielektrizitätskonstante der Hostmischung nach Zugabe der zu untersuchenden Verbindung und Extrapolation auf 100 % der eingesetzten Verbindung werden die Werte für die jeweiligen zu untersuchenden Verbindungen erhalten. Die zu untersuchende Verbindung wird zu 10 % in der Hostmischung gelöst. Wenn die Löslichkeit der Substanz hierzu zu gering ist, wird die Konzentration schrittweise solange halbiert, bis die Untersuchung bei der gewünschten Temperatur erfolgen kann.
Alle hier beschriebenen Erfindungsvarianten können miteinander kombiniert werden, solange sich die jeweiligen Ausführungsformen nicht gegenseitig ausschließen. Insbesondere ist es ausgehend von der Lehre dieser Schrift im Rahmen des routinemäßigen Optimierens ein naheliegender Vorgang, gerade verschiedene hier beschriebene Varianten zu kombinieren, um zu einer konkreten besonders bevorzugten Ausführungsform zu gelangen.

Die in dieser Anmeldung angegebenen Parameterbereiche, soweit nicht anders angegeben, umfassen alle rationalen und ganzzahligen Zahlenwerte einschließlich der angegebenen Grenzwerte des Parameterbereichs sowie deren Fehlergrenzen. Die für jeweilige Bereiche und Eigenschaften angegebenen oberen und unteren Grenzwerte führen wiederum in Kombination miteinander zu zusätzlichen bevorzugten Bereichen.

In der gesamten Beschreibung und den Ansprüchen dieser Anmeldung sind die Worte "umfassen" und "enthalten" und Variationen dieser Wörter, wie beispielsweise "umfassend" und "umfasst" als "einschließlich, aber nicht beschränkt auf" auszulegen und schließen andere Komponenten nicht aus. Das Wort "umfassen" schließt auch den Begriff "bestehend aus" mit ein, ist aber nicht darauf beschränkt.

Für die vorliegende Erfindung und in den folgenden Beispielen sind die Strukturen der Flüssigkristallverbindungen durch Acronyme angegeben, wobei die Transformation in chemische Formeln gemäß folgender Tabellen A bis C erfolgt. Alle Reste CₙH₂ₙ₊₁, CₘH₂ₘ₊₁ und CₗH₂ₗ₊₁ bzw. CₙH₂ₙ, CₘH₂ₘ und CₗH₂ₗ sind geradkettige Alkylreste bzw. Alkylenreste jeweils mit n, m bzw. I C-Atomen. In Tabelle A sind die Ringelemente der Kerne der Verbindung codiert, in Tabelle B sind die Brückenglieder aufgelistet und in Tabelle C sind die Bedeutungen der Symbole für die linken bzw. rechten Endgruppen der Moleküle aufgelistet. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechts Endgruppe, zusammengesetzt.

**Tabelle A: Ringelemente**

| | | | |
|---|---|---|---|
| **C** | 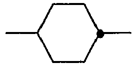 | | |
| **D** | 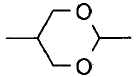 | **DI** | 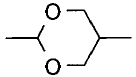 |
| **A** | 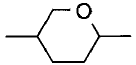 | **AI** | 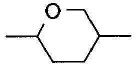 |
| **P** | 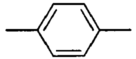 | | |
| **G** | 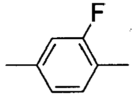 | **GI** | 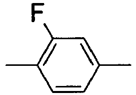 |
| **U** | 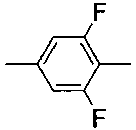 | **UI** | 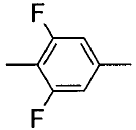 |
| **Y** | 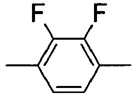 | | |
| **P(F,Cl)** | 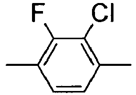 | **P(Cl,F)** | 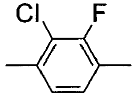 |
| **N** | 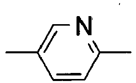 | **NI** | 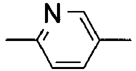 |
| **M** | 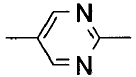 | **MI** | 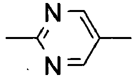 |
| **np** | 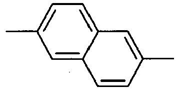 | | |
| **n3f** | 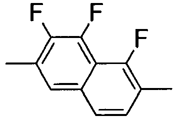 | **n3fl** | 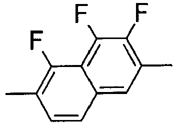 |
| **th** | 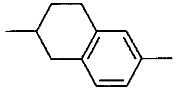 | **thl** | 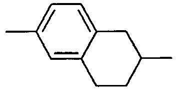 |
| **tH2f** | 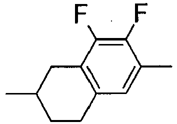 | **tH2fl** | 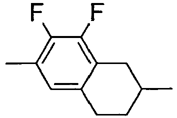 |
| **o2f** | 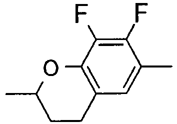 | **o2fl** | 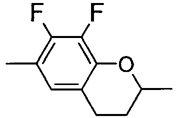 |
| **dh** | 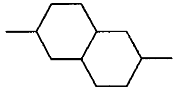 | | |
| K | 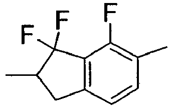 | **KI** | 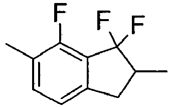 |
| L | 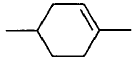 | **LI** | 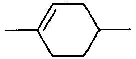 |
| **F** | 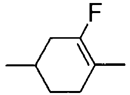 | **FI** | 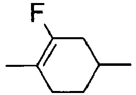 |

**Tabelle B: Brückenglieder**

| | | | |
|---|---|---|---|
| **E** | -CH₂-CH₂- | | |
| **V** | -CH=CH- | | |
| **T** | -C≡C- | | |
| **W** | -CF2-CF2- | | |
| **B** | -CF=CF- | | |
| **Z** | -CO-O- | **ZI** | -O-CO- |
| **X** | -CF=CH- | **XI** | -CH=CF- |
| **O** | -CH₂-O- | **OI** | -O-CH₂- |
| **Q** | -CF₂-O- | **QI** | -O-CF₂- |

**Tabelle C: Endgruppen**

| **Links einzelstehend oder in Kombination** | | **Rechts einzelstehend oder in Kombination** | |
|---|---|---|---|
| **-n-** | CₙH₂ₙ₊₁- | **-n** | -CₙH₂ₙ₊₁ |
| **-nO-** | CₙH₂ₙ₊₁-O- | **-nO** | -O-CₙH₂ₙ₊₁ |
| **-V-** | CH₂=CH- | **-V** | -CH=CH₂ |
| **-nV-** | CₙH₂ₙ₊₁-CH=CH- | **-nV** | -CₙH₂ₙ-CH=CH₂ |
| **-Vn-** | CH₂=CH- CₙH₂ₙ- | **-Vn** | -CH=CH-CₙH₂ₙ₊₁ |
| **-nVm-** | CₙH₂ₙ₊₁-CH=CH-CₘH₂ₘ- | **-nVm** | -CₙH₂ₙ-CH=CH-CₘH₂ₘ₊₁ |
| **-N-** | N≡C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | CFH₂- | **-M** | -CFH₂ |
| **-D-** | CF₂H- | **-D** | -CF₂H |
| **-T-** | CF₃- | **-T** | -CF₃ |
| **-MO-** | CFH₂O- | **-OM** | -OCFH₂ |
| **-DO-** | CF₂HO- | **-OD** | -OCF₂H |
| **-TO-** | CF₂O- | **-OT** | -OCF₃ |
| **-A-** | H-C≡C- | **-A** | -C≡C-H |
| **-nA-** | CₙH₂ₙ₊₁-C≡C- | **-An** | -C≡C-CₙH₂ₙ₊₁ |
| **-NA-** | N≡C-C≡C- | **-AN** | -C≡C-C≡N |
| | | | |

| **Links nur in Kombination** | | **Rechts nur in Kombination** | |
|---|---|---|---|
| **-...n...-** | -CₙH₂ₙ- | **-...n...** | -CₙH₂ₙ- |
| **-...M...-** | -CFH- | **-...M...** | -CFH- |
| **-...D...-** | -CF₂- | **-...D...** | -CF2- |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

| | | | |
|---|---|---|---|
| worin n und m jeweils ganze Zahlen und die drei Punkte "..." Platzhalter für andere Abkürzungen aus dieser Tabelle sind. | | | |

**Tabelle D**

| |
|---|
| In der Tabelle D werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. |
| 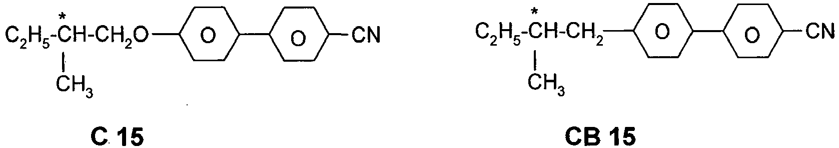 |
| 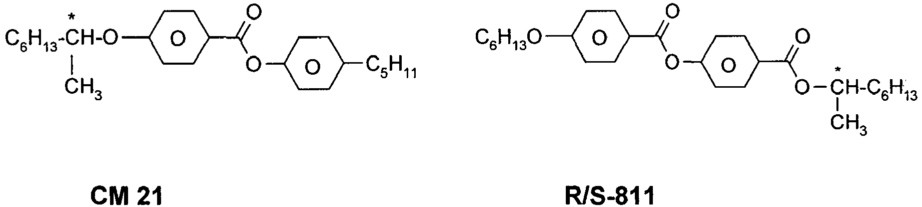 |
| 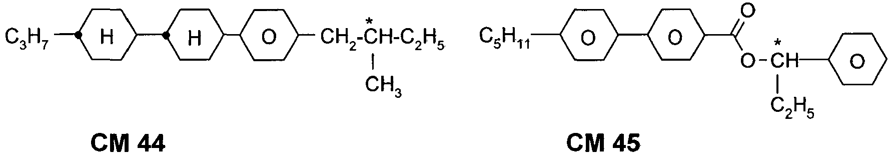 |
| 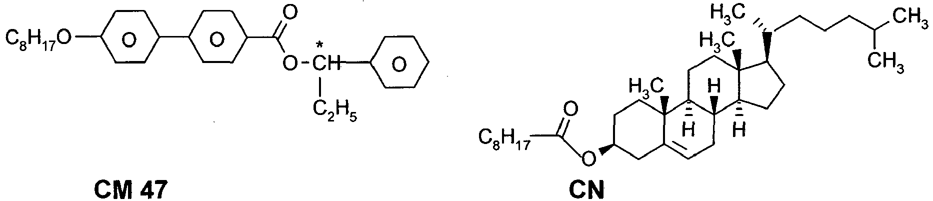 |
| 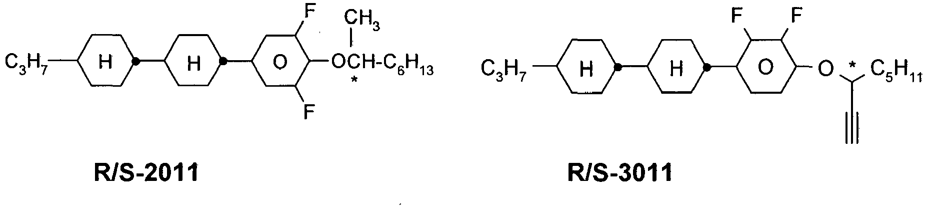 |
| 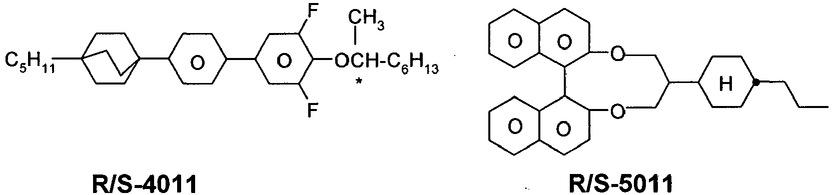 |
| 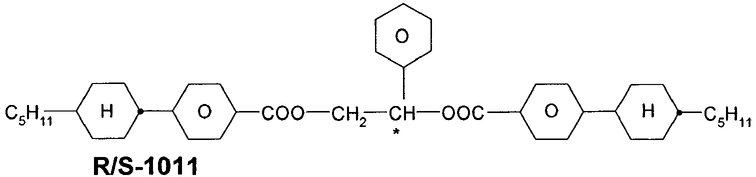 |

**Tabelle E**

| |
|---|
| Stabilisatoren, die beispielsweise den erfindungsgemäßen Mischungen in Mengen von 0-10 Gew.% zugesetzt werden können, werden nachfolgend genannt. (n = 1-12) |
| 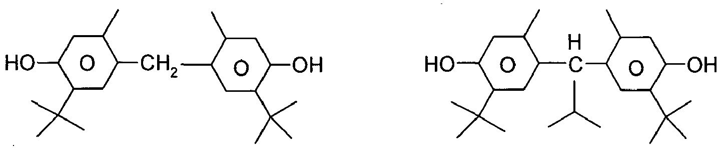 |
| 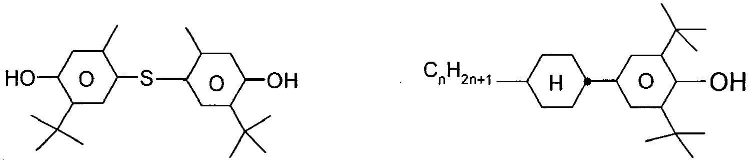 |
| 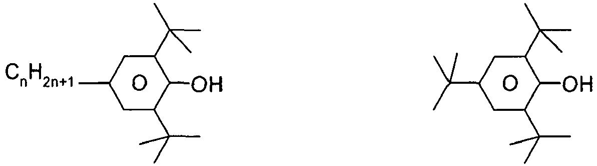 |
| n = 1, 2, 3, 4, 5, 6 oder 7 |
| 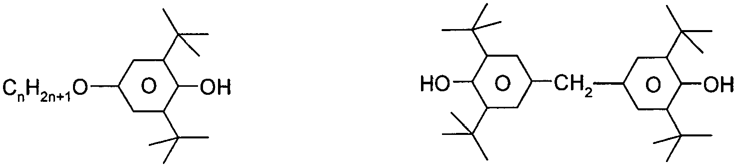 |
| 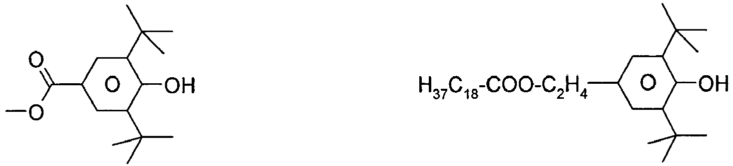 |
| 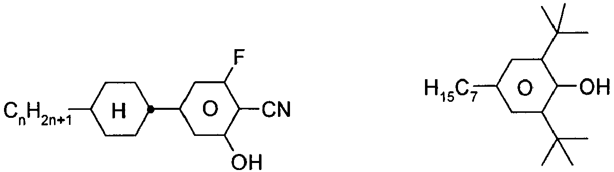 |
| 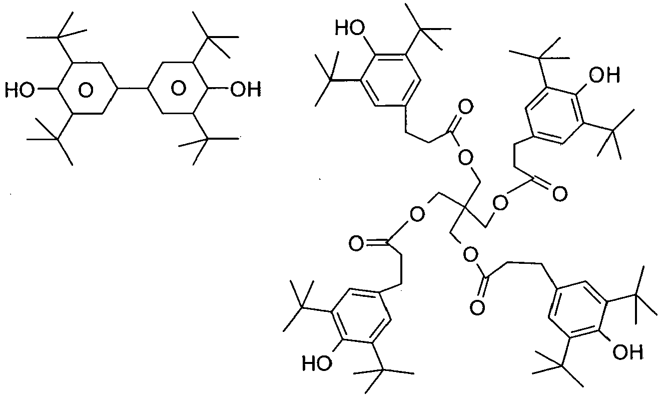 |
| 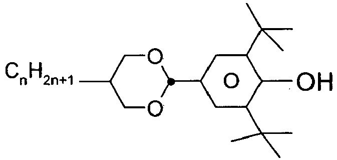 |
| n = 1, 2, 3, 4, 5, 6 oder 7 |
| 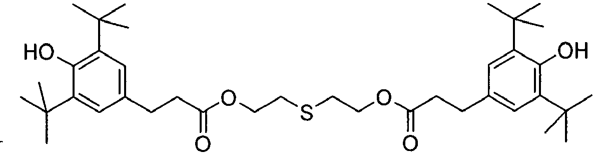 |
| 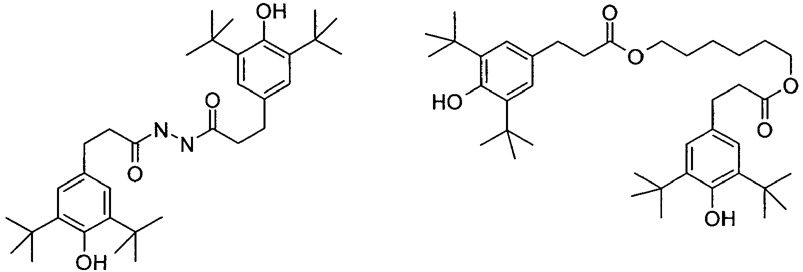 |
| 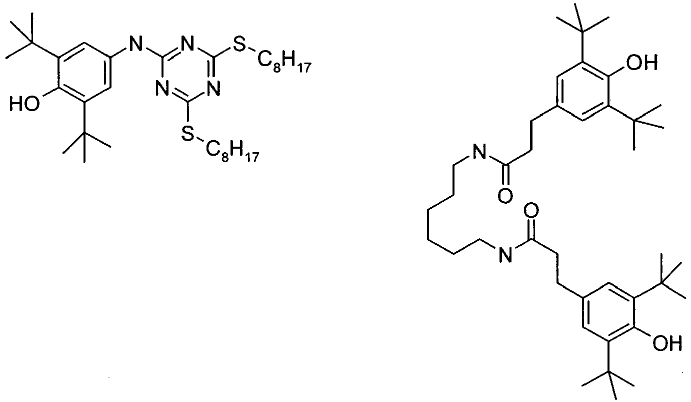 |
| 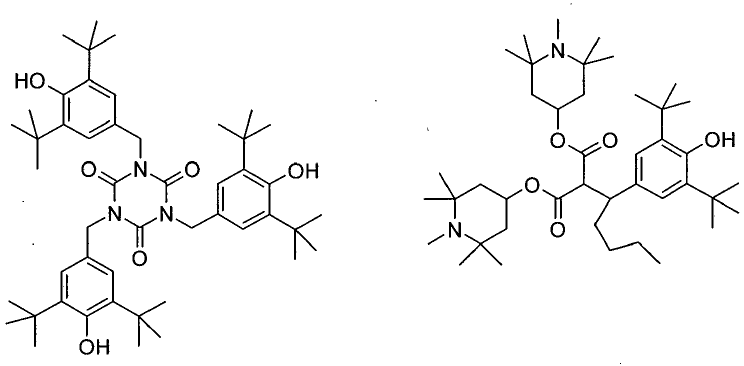 |
| 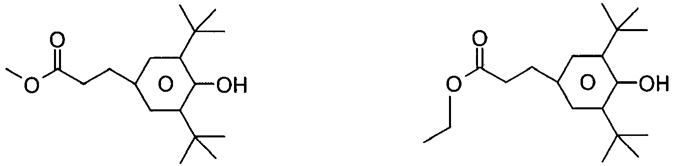 |
| 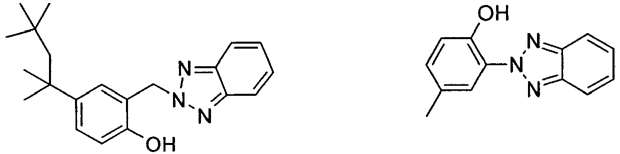 |
| 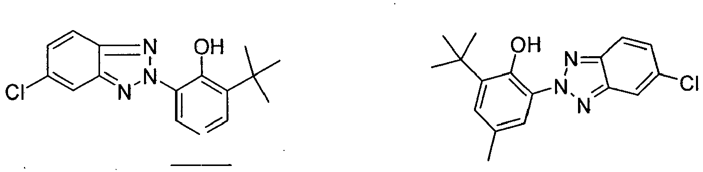 |
| 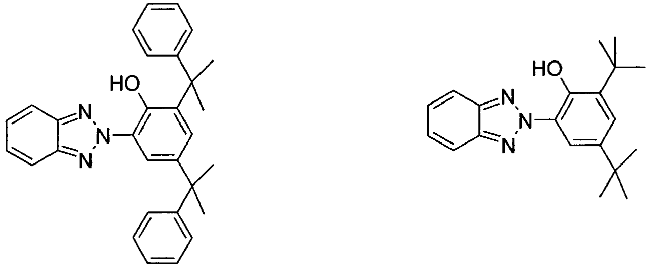 |
| 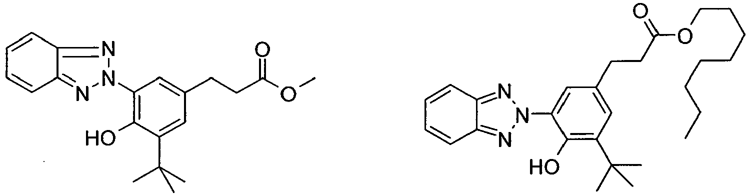 |
| 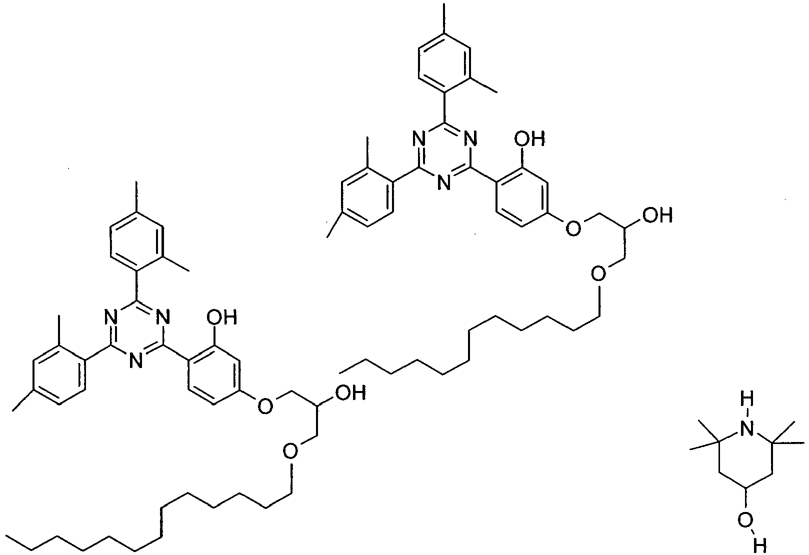 |
| 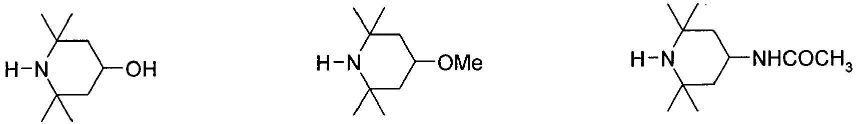 |
| 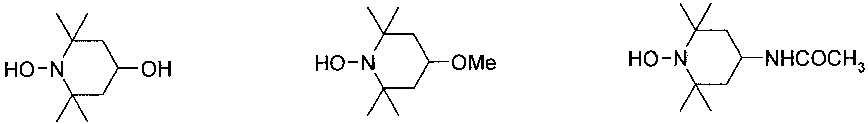 |
| 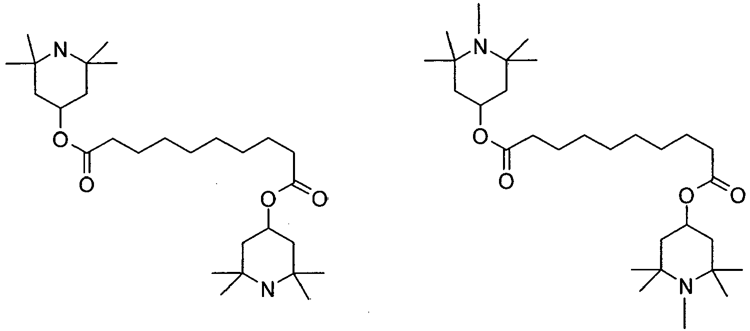 |
| 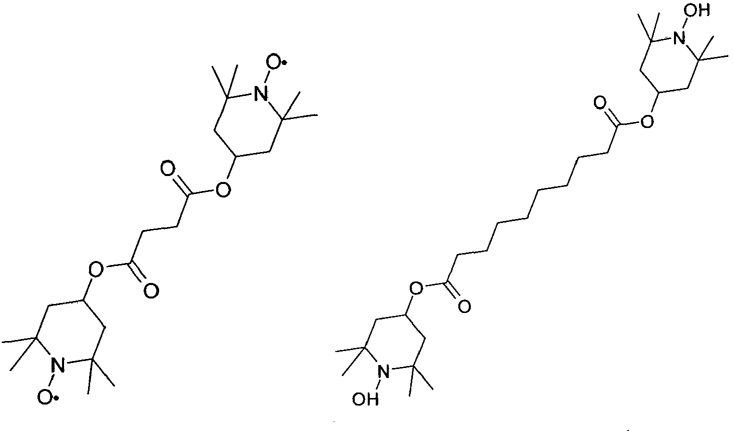 |

Geeignete polymerisierbare Verbindungen (reaktive Mesogene) für den Einsatz in den erfindungsgemäßen Mischungen, vorzugsweise in PSA- und PS-VA-Anwendungen oder PS-IPS/FFS-Anwendungen, werden nachfolgend in Tabelle E genannt:

**Tabelle F**

| |
|---|
| In der Tabelle F sind Beispielverbindungen zusammengestellt, die in den erfindungsgemäßen Mischungen vorzugsweise als polymerisierbare Verbindungen (reaktive mesogene Verbindungen) zur Herstellung beispielsweise von PSV, PS-VA-, PS-IPS-oder PS-FFS-Mischungen verwendet werden können. |
| 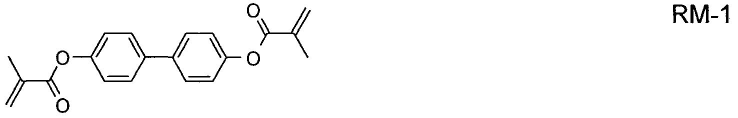 |
| 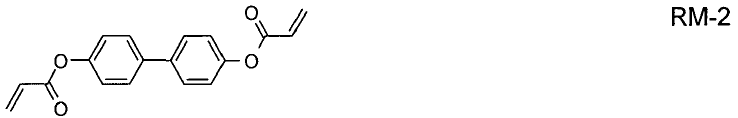 |
| 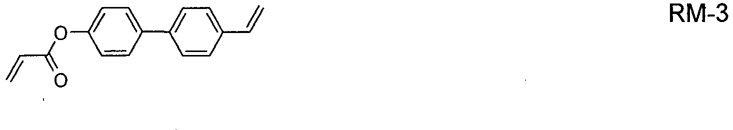 |
| 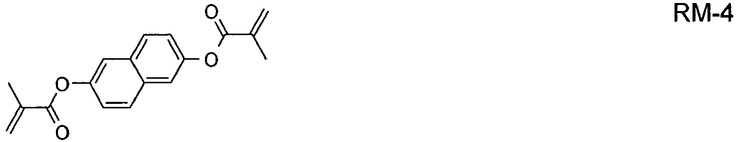 |
| 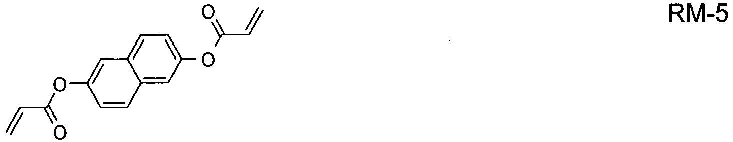 |
| 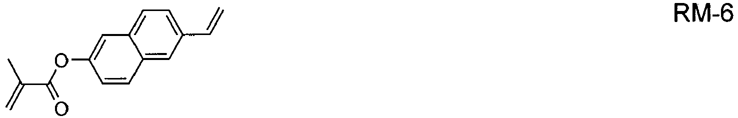 |
| 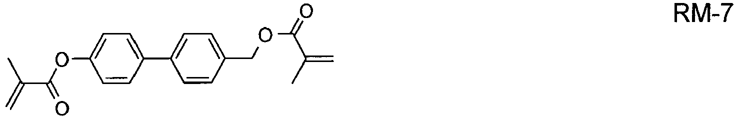 |
| 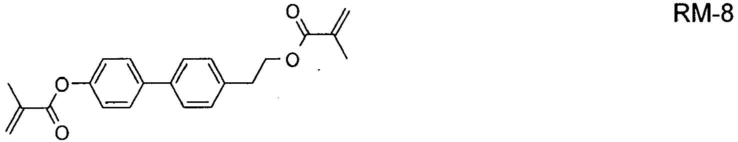 |
| 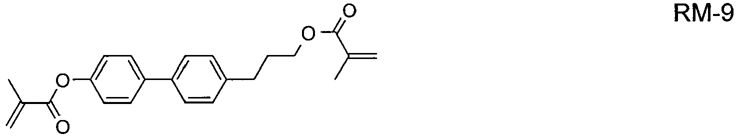 |
| 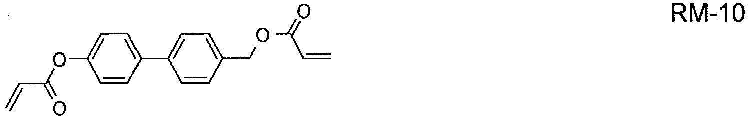 |
| 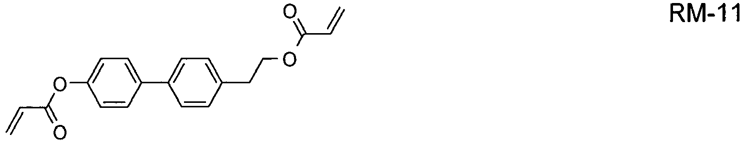 |
| 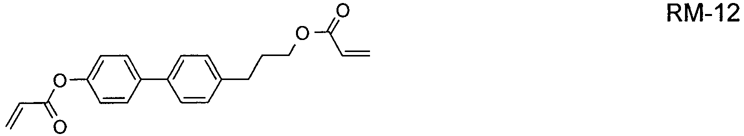 |
| 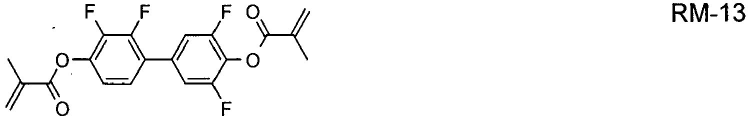 |
| 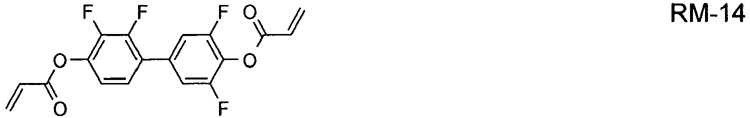 |
| 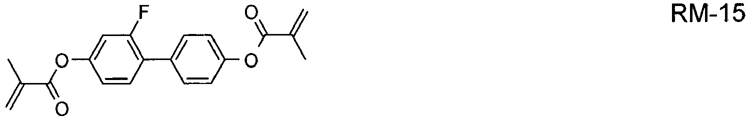 |
| 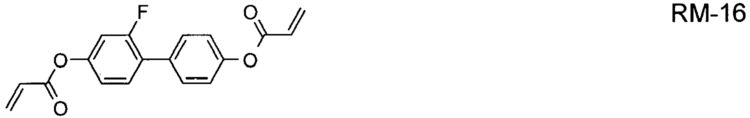 |
| 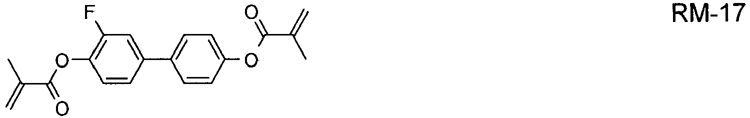 |
| 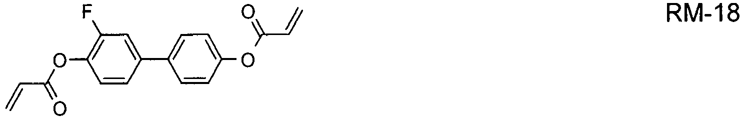 |
| 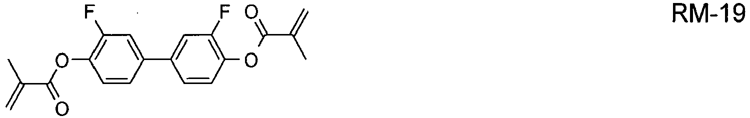 |
| 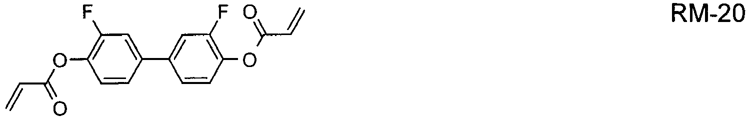 |
| 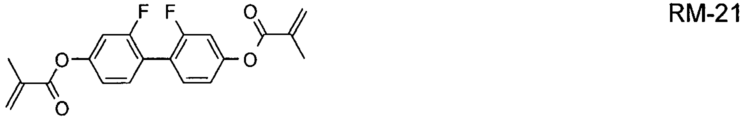 |
| 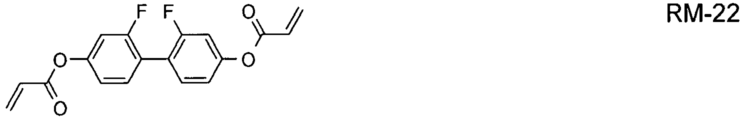 |
| 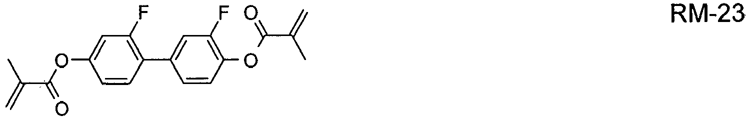 |
| 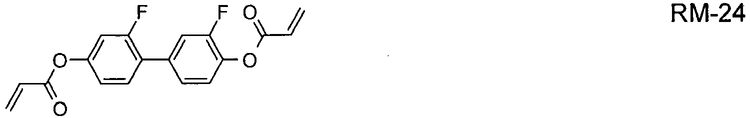 |
| 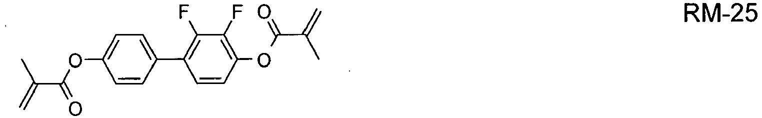 |
| 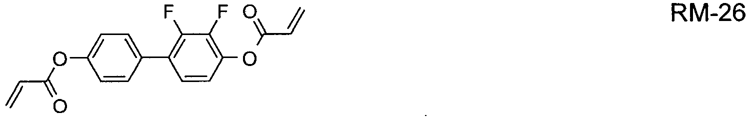 |
| 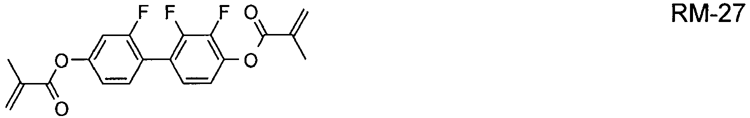 |
| 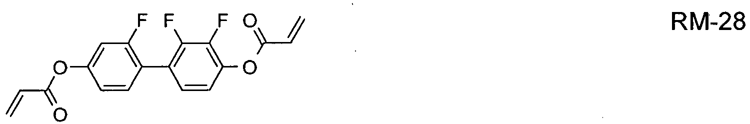 |
| 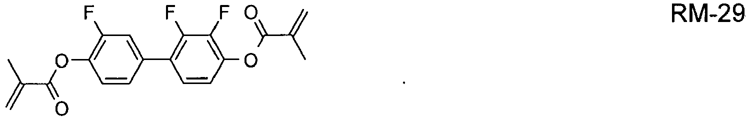 |
| 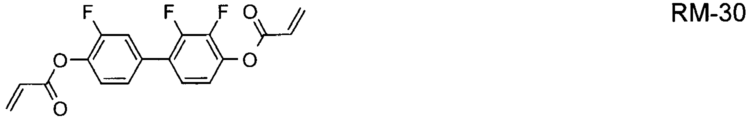 |
| 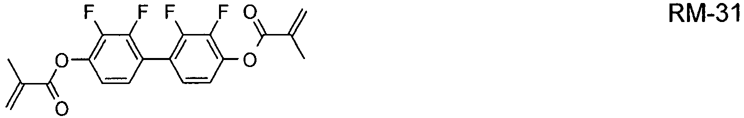 |
| 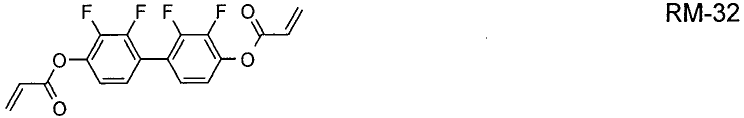 |
| 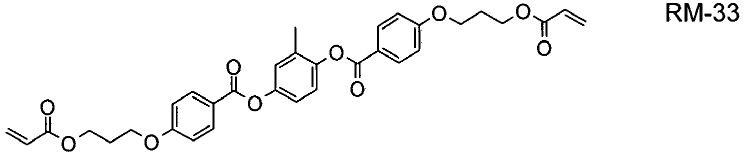 |
| 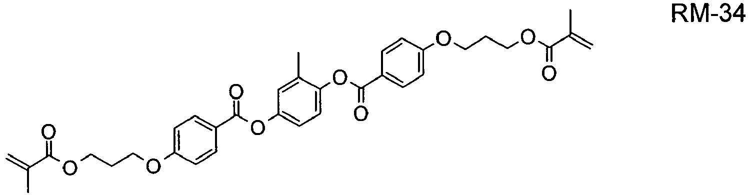 |
| 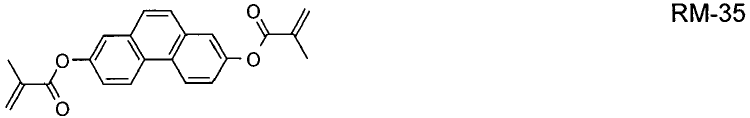 |
| 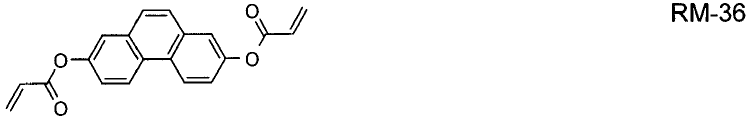 |
| 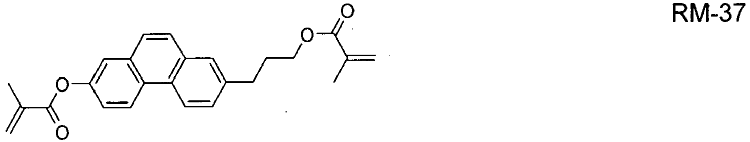 |
| 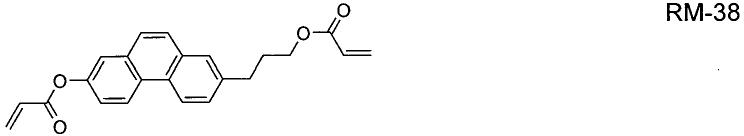 |
| 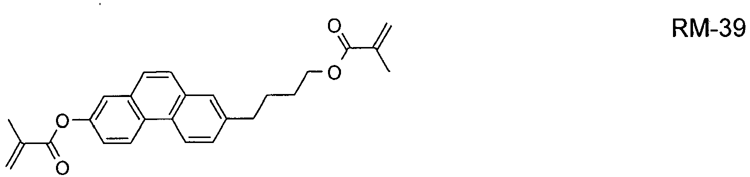 |
| 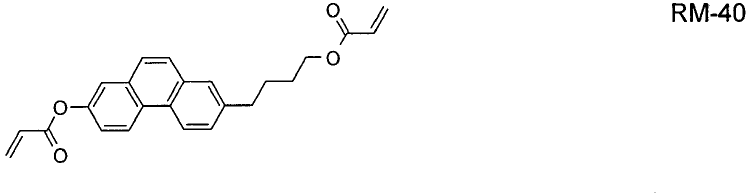 |
| 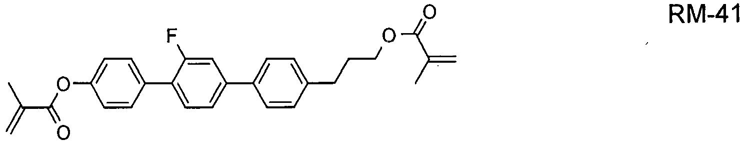 |
| 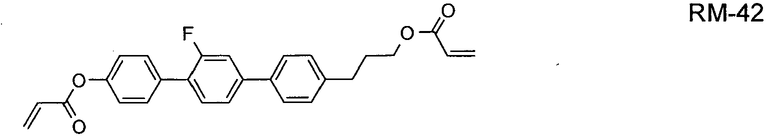 |
| 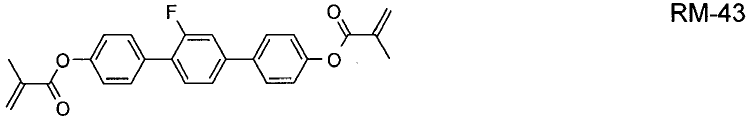 |
| 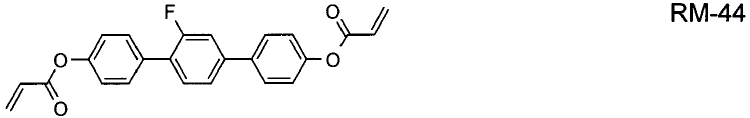 |
| 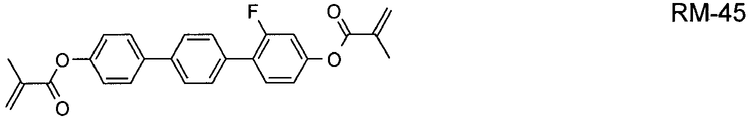 |
| 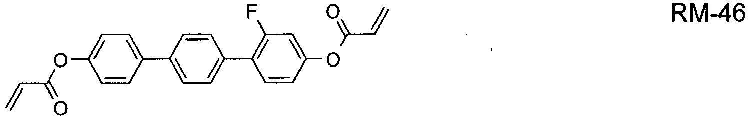 |
| 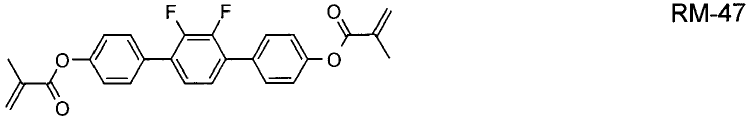 |
| 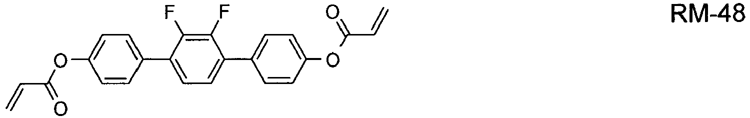 |
| 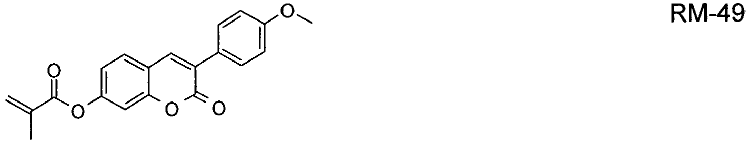 |
| 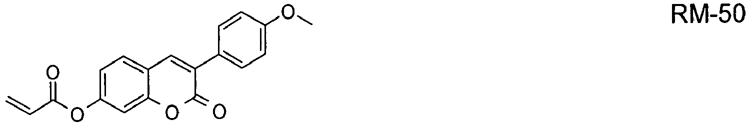 |
| 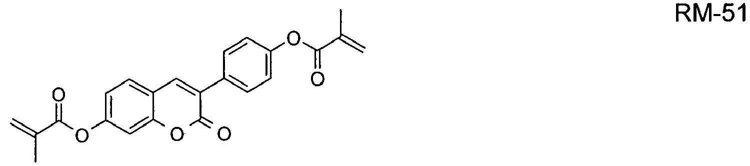 |
| 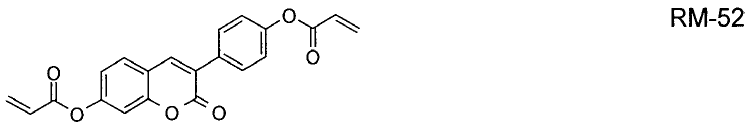 |
| 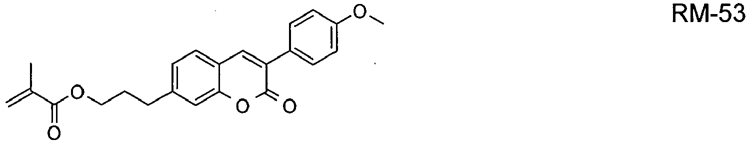 |
| 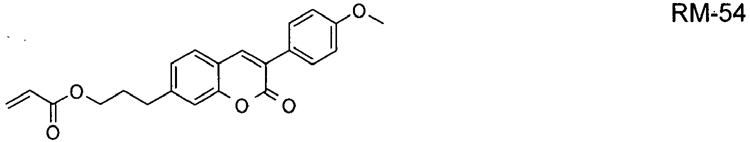 |
| 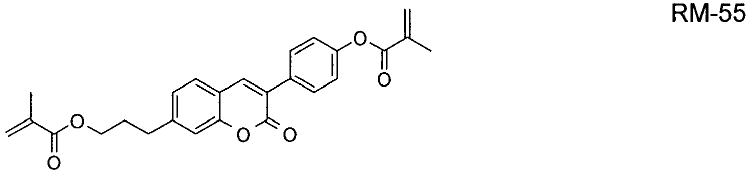 |
| 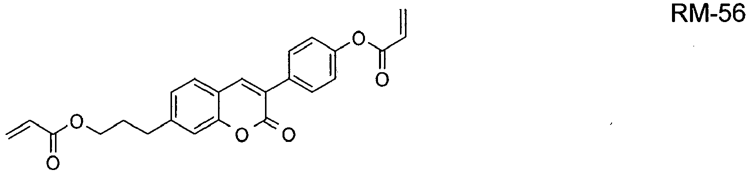 |
| 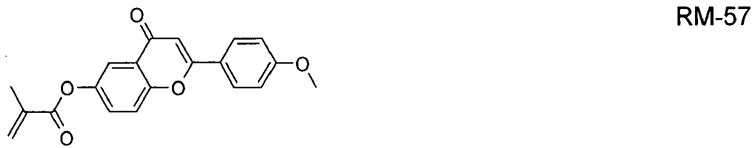 |
| 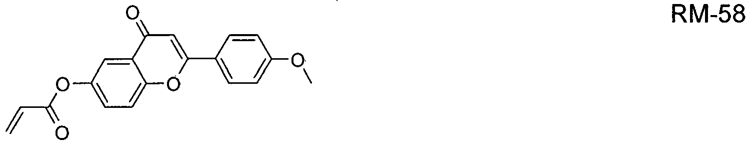 |
| 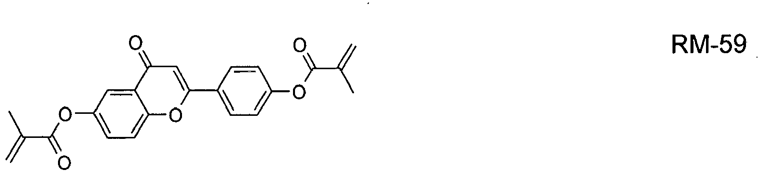 |
| 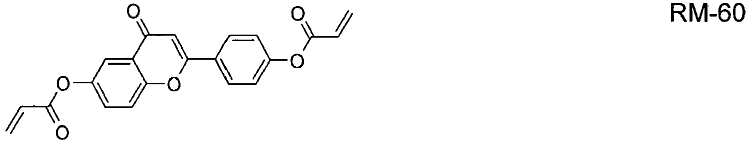 |
| 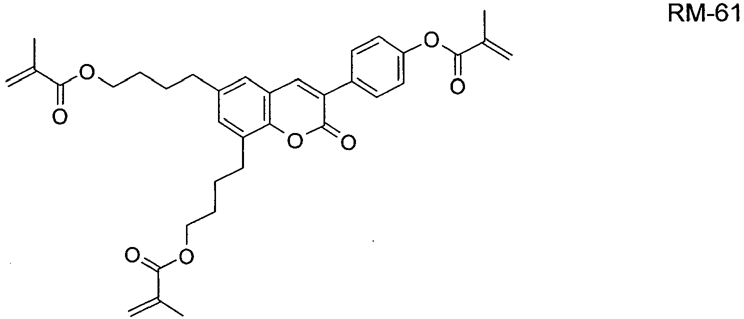 |
| 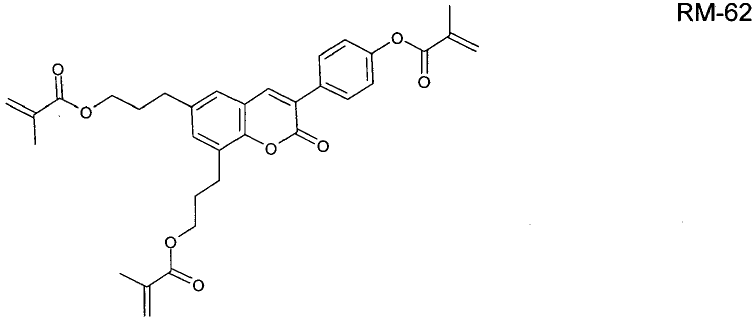 |
| 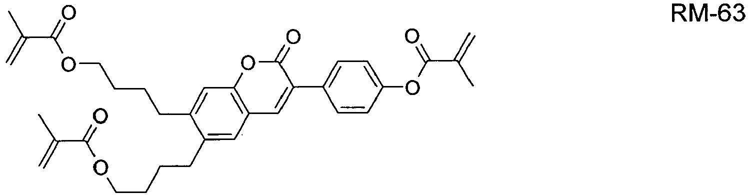 |
| 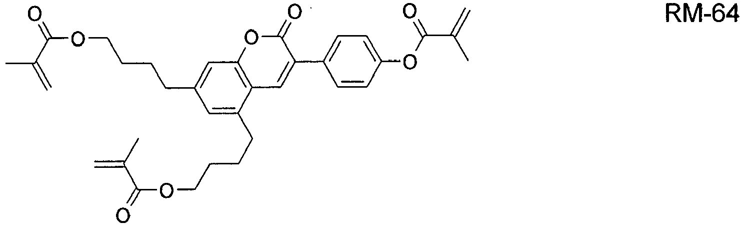 |
| 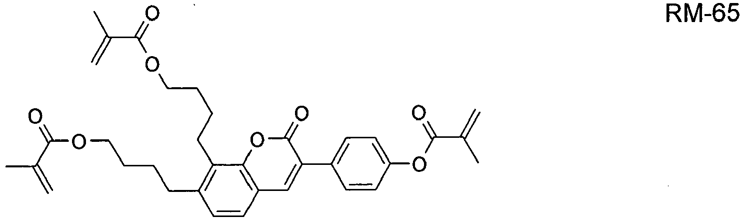 |
| 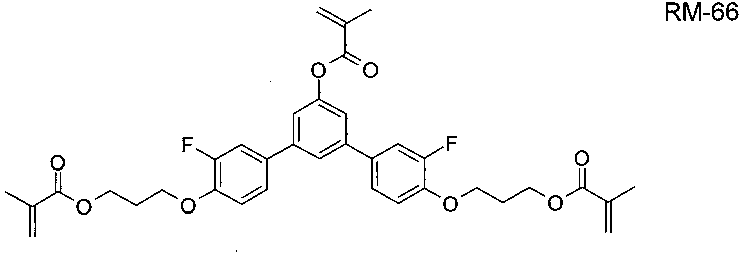 |
| 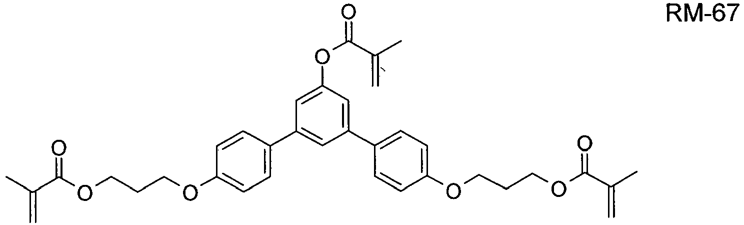 |
| 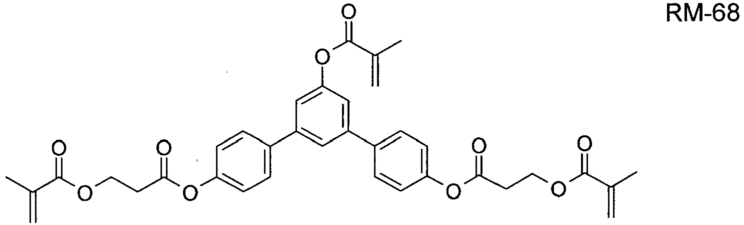 |
| 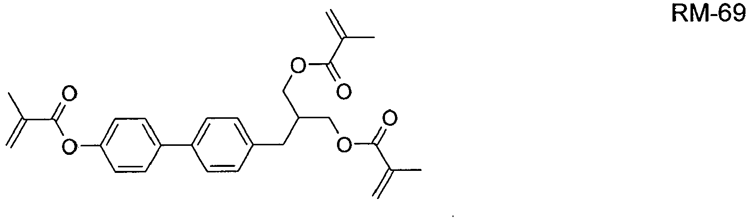 |
| 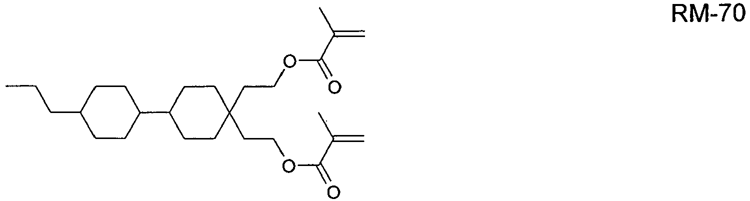 |
| 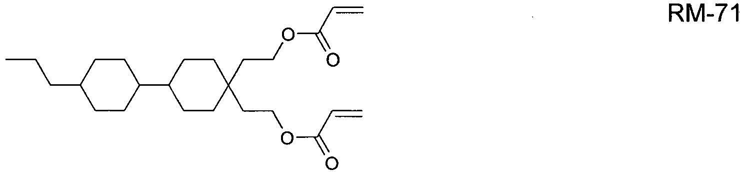 |
| 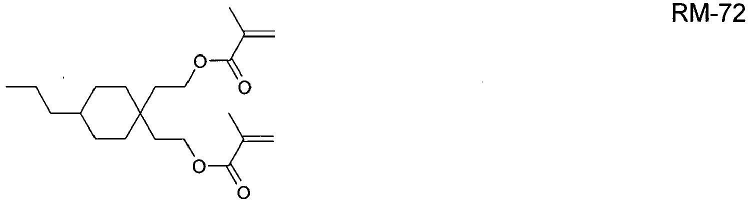 |
| 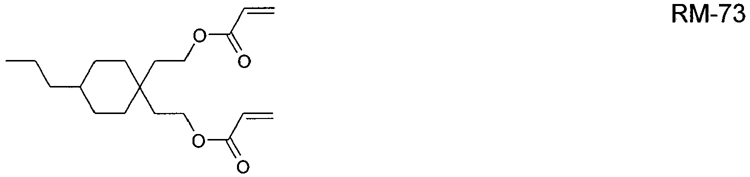 |
| 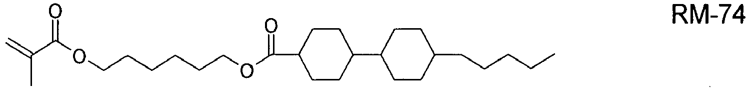 |
| 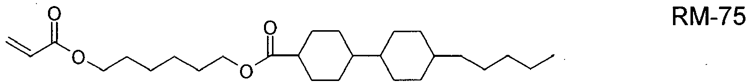 |
| 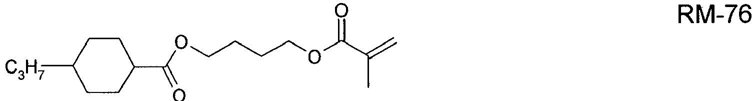 |
| 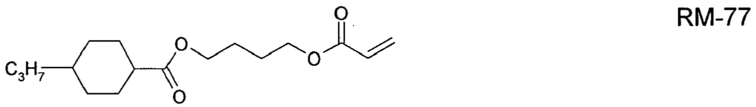 |
| 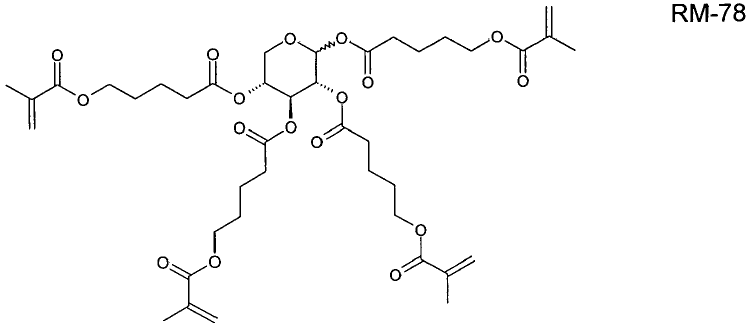 |
| 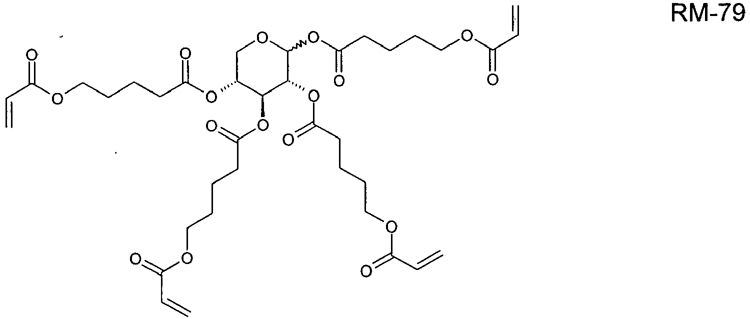 |
| 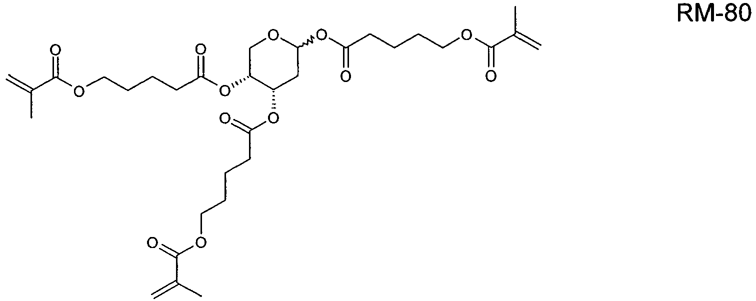 |
| 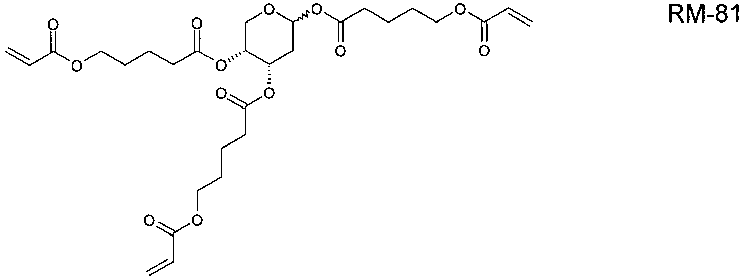 |
| 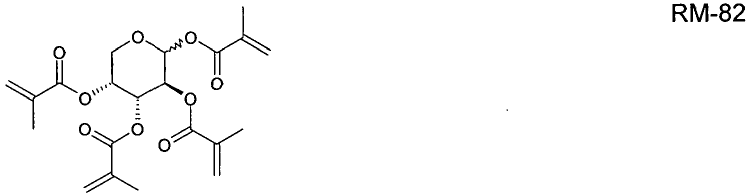 |
| 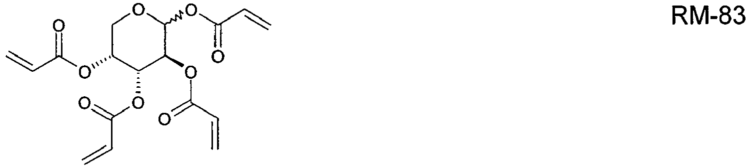 |

Sofern die erfindungsgemäßen Mischungen eine oder mehrere mesogene Verbindungen enthalten, ist die mesogene Verbindung in einer bevorzugten Ausführungsform eine Verbindung ausgewählt aus der Tabelle F.

Die folgenden Beispiele sollen die Erfindung erläutern, ohne sie zu begrenzen.

### Beispiele

Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Weiterhin bedeuten

Alle physikalischen Eigenschaften werden nach "Merck Liquid Crystals, Physical Properties of Liquid Crystals" Status Nov. 1997, Merck KGaA, Deutschland bestimmt und gelten für eine Temperatur von 20 °C, sofern nicht explizit anders angegeben.

**Vergleichsbeispiel V1**

| | | | |
|---|---|---|---|
| CC-3-V | 8,0 % | Klärpunkt [°C]: | 102,0 |
| CPGP-5-2 | 5,0 % | S->N Übergang [°C]: | -14,0 |
| CPGP-4-3 | 5,0 % | Δn [589 nm, 20 °C]: | 0,1959 |
| CPGP-5-3 | 5,0 % | Δε [1 kHz, 20 °C]: | 4,8 |
| CP-3-O1 | 15,0 % | ε_{∥} [1 kHz, 20°C]: | 8,3 |
| PGIGI-3-F | 4,0 % | ε_{⊥} [1 kHz, 20°C]: | 3,5 |
| PGP-2-2V | 15,0 % | | |
| PGP-2-3 | 4,0 % | LTS-Bulk [-10°C]: | >1000 h |
| PGP-2-4 | 5,0 % | LTS-Bulk [-25°C]: | - |
| PGP-2-5 | 10,0 % | LTS-Bulk [-30°C]: | - |
| PGUQU-4-F | 4,0 % | | |
| PGUQU-5-F | 3,0 % | | |
| PP-1-2V1 | 7,0 % | | |
| PUQU-3-F | 10,0 % | | |
| G-4O-O3 | - | | |

**Beispiel M1**

| | | | |
|---|---|---|---|
| CC-3-V | 8,0 % | Klärpunkt [°C]: | 105,5 |
| CPGP-5-2 | 5,0 % | S->N Übergang [°C]: | -30,0 |
| CPGP-4-3 | 6,0 % | Δn [589 nm, 20 °C]: | 0,2006 |
| CPGP-5-3 | 5,0 % | Δε [1 kHz, 20 °C]: | 5,3 |
| CP-3-O1 | 10,0 % | ε_{∥} [1 kHz, 20°C]: | 9,0 |
| PGIGI-3-F | 6,0 % | ε_{⊥} [1 kHz, 20°C]: | 3,7 |
| PGP-2-2V | 16,0 % | | |
| PGP-2-3 | 5,0 % | LTS-Bulk [-10°C]: | - |
| PGP-2-4 | 5,0 % | LTS-Bulk [-25°C]: | >1000 h |
| PGP-2-5 | 10,0 % | LTS-Bulk [-30°C]: | 888 h |
| PGUQU-4-F | 6,0 % | | |
| PGUQU-5-F | 3,0 % | | |
| PP-1-2V1 | 3,0 % | | |
| PUQU-3-F | 9,0 % | | |
| G-4O-O3 | 3,0 % | | |

Aus dem Vergleich der Mischungen V1 und M1 (erfindungsgemäß) wird sofort ersichtlich, dass durch die Hinzufügung geringer Mengen der Verbindung G-4O-O3 der nematische Phasenbereich zu deutlich tieferen Temperaturen verschoben werden kann. Gleichzeitig wird sowohl das Δn von 0,1959 auf 0,2006 erhöht und als auch die Tieftemperaturstabilität (LTS-Bulk) von -10°C auf -30°C signifikant verbessert.

**Beispiel M2**

| | | | |
|---|---|---|---|
| CC-3-V | 38,0 % | Klärpunkt [°C]: | 78,0 |
| CCP-V-1 | 6,0 % | | |
| CCP-3-OT | 9,0 % | Δn [589 nm, 20 °C]: | 0,1066 |
| CPGP-4-3 | 6 , 0 % | Δε [1 kHz, 20 °C]: | 9,4 |
| APUQU-2-F | 8,0 % | ε_{∥} [1 kHz, 20°C]: | 13,4 |
| APUQU-3-F | 8,5 % | ε_{⊥} [1 kHz, 20°C]: | 4,0 |
| PGUQU-3-F | 3,0 % | | |
| PGUQU-4-F | 7,0 % | LTS-Bulk [-20°C]: | >1000 h |
| DPGU-4-F | 5,0 % | LTS-Bulk [-25°C]: | >1000 h |
| CPY-2-O2 | 1,5 % | LTS-Bulk [-30°C]: | >1000 h |
| G-4O-O3 | 8,0 % | | |

**Beispiel M3**

| | | | |
|---|---|---|---|
| CC-3-V | 44,0 % | Klärpunkt [°C]: | 79,0 |
| APUQU-2-F | 8,5 % | | |
| APUQU-3-F | 8,0 % | Δn [589 nm, 20 °C]: | 0,1084 |
| CCP-3-OT | 6,0 % | Δε [1 kHz, 20 °C]: | 9,6 |
| CCP-V-1 | 8,0 % | ε_{∥} [1 kHz, 20°C]: | 13,2 |
| PGUQU-4-F | 4,5 % | ε_{⊥} [1 kHz, 20°C]: | 3,5 |
| PGP-2-2V | 7,5 % | | |
| DPGU-4-F | 5,5 % | LTS-Bulk [-20°C]: | >1000 h |
| PUQU-3-F | 6,5 % | LTS-Bulk [-25°C]: | >1000 h |
| G-4O-O3 | 1,5 % | LTS-Bulk [-30°C]: | 24 h |

**Beispiel M4**

| | | | |
|---|---|---|---|
| CC-3-V | 25,0 % | Klärpunkt [°C]: | 80,0 |
| CC-3-V1 | 6,0 % | | |
| CC-3-2V1 | 4,0 % | Δn [589 nm, 20 °C]: | 0,1108 |
| CCP-V-1 | 15,0 % | Δε [1 kHz, 20 °C]: | 10,9 |
| CCP-30CF3 | 3,0 % | ε_{∥} [1 kHz, 20°C]: | 14,9 |
| APUQU-3-F | 4,0 % | ε_{⊥} [1 kHz, 20°C]: | 4,0 |
| CDUQU-3-F | 7,0 % | | |
| DGUQU-4-F | 5,0 % | LTS-Bulk [-30°C]: | >744 h |
| DPGU-4-F | 5,5 % | | |
| PGP-2-2V | 5,0 % | | |
| PGUQU-3-F | 6,0 % | | |
| PGUQU-4-F | 6,0 % | | |
| PPGU-3-F | 0,5 % | | |
| G-4O-O3 | 8,0 % | | |

## Patentansprüche

1. Flüssigkristallines Medium, **dadurch gekennzeichnet, dass** das Medium mindestens eine Verbindung der Formel I, 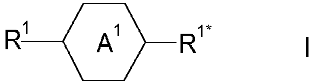 worin
R¹ und R^{1*} jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 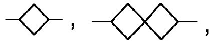 -O- so ersetzt sein können, dass O-Atome nicht direkt miteinander verknüpft sind, und worin auch ein oder mehrere H-Atome durch Halogen ersetzt sein können,
A¹ 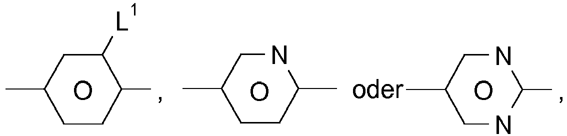
L¹ F, Cl, CF₃, OCF₃ oder CHF₂,
bedeuten,
enthält.

2. Flüssigkristallines Medium nach Anspruch 1, **dadurch gekennzeichnet, dass** das Medium mindestens eine Verbindung der Formeln I-1 bis I-10, 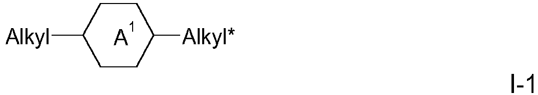 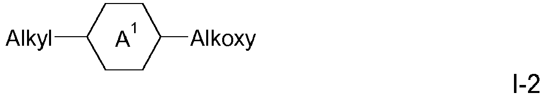 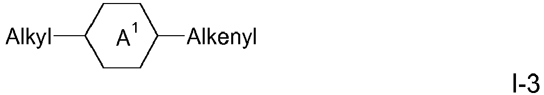 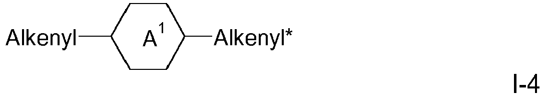 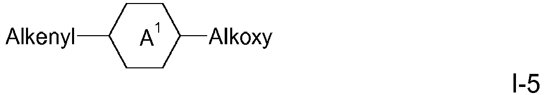 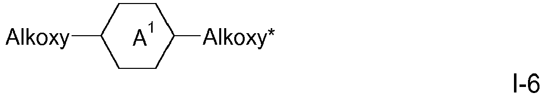 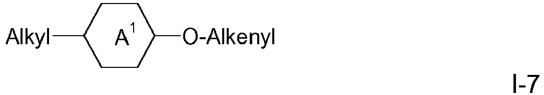 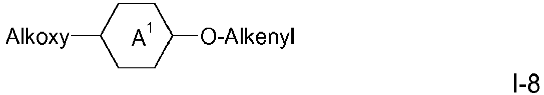 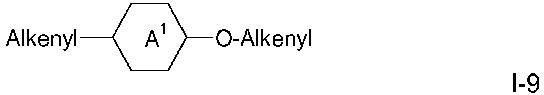 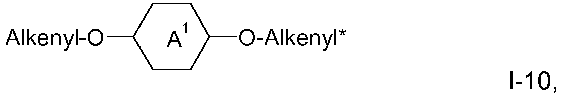 worin
Alkyl und Alkyl* jeweils unabhängig voneinander einen geradkettigen Alkylrest mit 1-6 C-Atomen,
Alkenyl und Alkenyl* jeweils unabhängig voneinander einen geradkettigen Alkenylrest mit 2-6 C-Atomen,
Alkoxy und Alkoxy* jeweils unabhängig voneinander einen geradkettigen Alkoxyrest mit 1-6 C-Atomen, bedeuten und
A¹ eine der unter Anspruch 1 angegebenen Bedeutung hat, enthält.

3. Flüssigkristallines Medium nach Anspruch 1 oder 2, **dadurch gekennzeichnet, dass** es zusätzlich eine oder mehrere Verbindungen ausgewählt aus den Formeln II und/oder III, 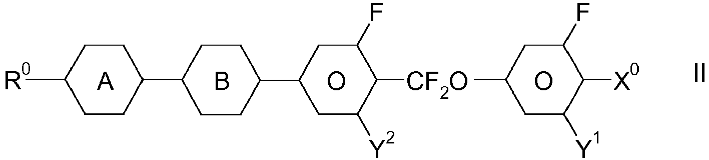 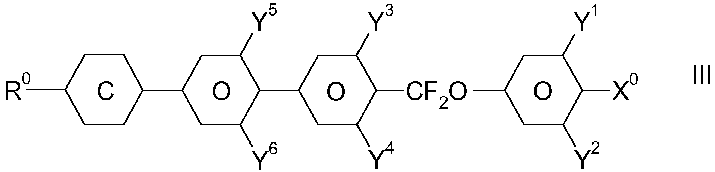 worin
R⁰ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH, 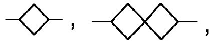 -O-, -CO-O- oder -O-CO- so ersetzt sein können, dass O-Atome nicht direkt miteinander verknüpft sind,
X⁰ F, Cl, CN, SF₅, SCN, NCS, halogenierter Alkylrest, halogenierter Alkenylrest, halogenierter Alkoxyrest oder halogenierter Alkenyloxyrest mit bis zu 6 C-Atomen, und
Y¹⁻⁶ jeweils unabhängig voneinander H, F oder Cl, 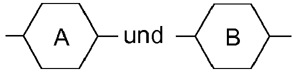 jeweils unabhängig voneinander 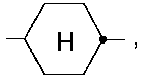 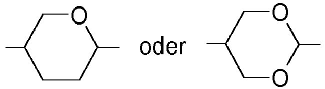 bedeuten, und 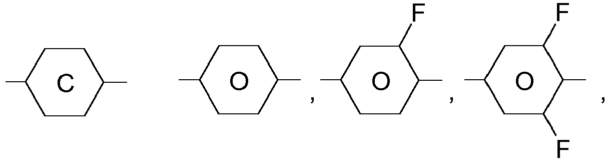 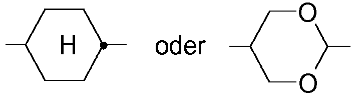 bedeutet; enthält.

4. Flüssigkristallines Medium nach einem oder mehreren der Ansprüche 1 bis 3, **dadurch gekennzeichnet, dass** es zusätzlich eine oder mehrere Verbindungen ausgewählt aus den Formeln IX bis XII, 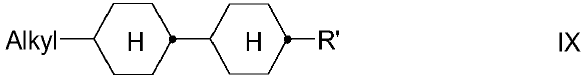 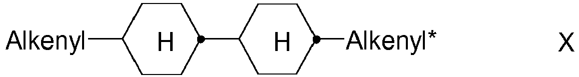 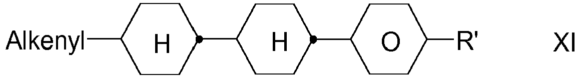 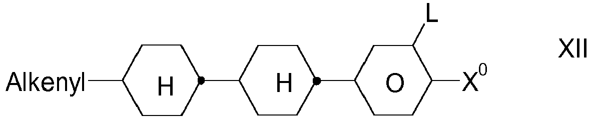 worin X⁰ die in Anspruch 3 angegebenen Bedeutungen besitzt,
L H oder F,
"Alkyl" C₁₋₆-Alkyl,
R' C₁₋₆-Alkyl, C₁₋₆-Alkoxy oder C₂₋₆-Alkenyl, und
"Alkenyl" und "Alkenyl*"jeweils unabhängig voneinander C₂₋₆-Alkenyl bedeuten,
enthält.

5. Flüssigkristallines Medium nach einem oder mehreren der Ansprüche 1 bis 4, **dadurch gekennzeichnet, dass** es zusätzlich eine oder mehrere Verbindungen der Formel XIII, 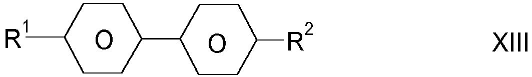 worin R¹ und R² jeweils unabhängig voneinander n-Alkyl, Alkoxy, Oxaalkyl, Fluoralkyl oder Alkenyl mit jeweils bis zu 6 C-Atomen bedeuten,
enthält.

6. Flüssigkristallines Medium nach einem oder mehreren der Ansprüche 1 bis 5, **dadurch gekennzeichnet, dass** es zusätzlich eine oder mehrere Verbindungen der Formel XVII, 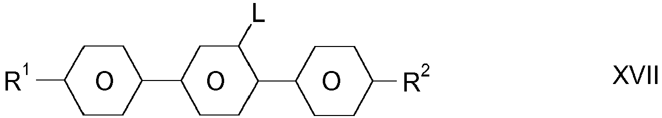 worin R¹ und R² jeweils unabhängig voneinander n-Alkyl, Alkoxy, Oxaalkyl, Fluoralkyl oder Alkenyl mit jeweils bis zu 8 C-Atomen, und L H oder F bedeuten,
enthält.

7. Flüssigkristallines Medium nach einem oder mehreren der Ansprüche 1 bis 6, **dadurch gekennzeichnet, dass** es zusätzlich eine oder mehrere Verbindungen ausgewählt aus der Gruppe der Verbindungen der Formeln XXVII, XXVIII und XXIX, 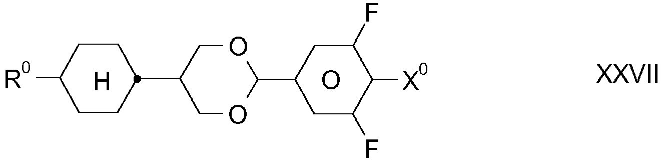 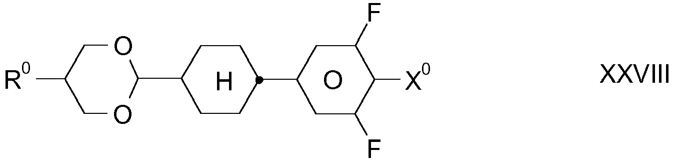 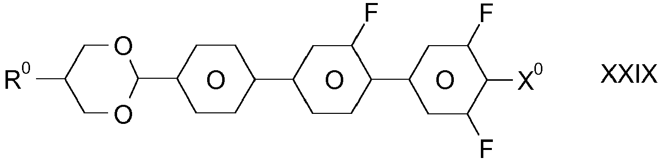 worin R⁰ und X⁰ die in Anspruch 3 angegebenen Bedeutungen haben, enthält.

8. Flüssigkristallines Medium nach einem oder mehreren der Ansprüche 1 bis 7, **dadurch gekennzeichnet, dass** es zusätzlich eine oder mehrere Verbindungen ausgewählt aus der Gruppe der Verbindungen der Formeln XIX, XX, XXI, XXII, XXIII und XXIV, 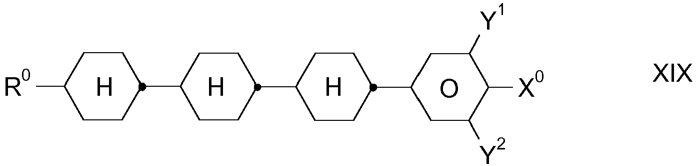 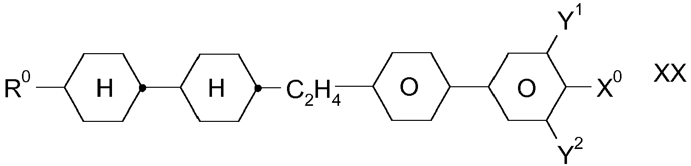 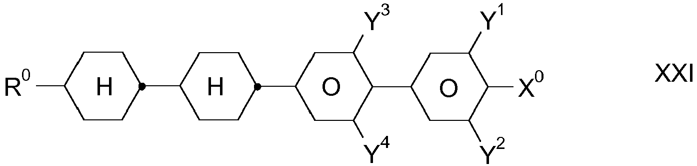 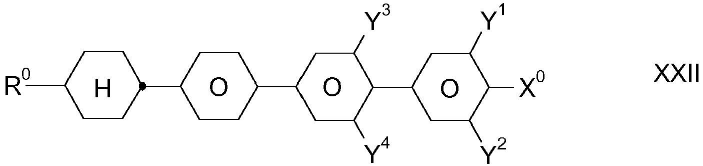 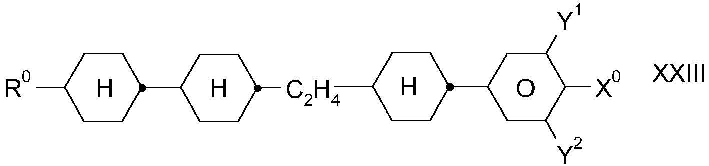 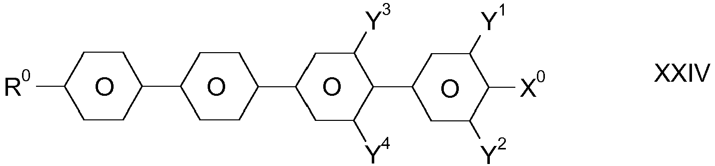 worin R⁰ und X⁰ die in Anspruch 3 angegebenen Bedeutungen haben und Y¹⁻⁴ jeweils unabhängig voneinander H oder F bedeuten,
enthält.

9. Flüssigkristallines Medium nach einem oder mehreren der Ansprüche 1 bis 8, **dadurch gekennzeichnet, dass** es zusätzlich ein oder mehrere Additiv(e) ausgewählt aus der Gruppe der UV-Stabilisatoren, Dotierstoffe und Antioxidantien enthält.

10. Flüssigkristallines Medium nach einem oder mehreren der Ansprüche 1 bis 9, **dadurch gekennzeichnet, dass** es zusätzlich ein oder mehrere polymerisierbare Verbindungen enthält.

11. Verfahren zur Herstellung eines flüssigkristallinen Mediums nach einem oder mehreren der Ansprüche 1 bis 10, **dadurch gekennzeichnet, dass** man eine oder mehrere Verbindungen der Formel I, wie in Anspruch 1 definiert, mit einer oder mehreren Verbindungen gemäß einem oder mehreren der Ansprüche 3 bis 8 oder mit weiteren flüssigkristallinen Verbindungen und gegebenenfalls noch mit ein oder mehreren Additiven und/oder mindestens einer polymerisierbaren Verbindung mischt.

12. Verwendung eines flüssigkristallinen Mediums nach einem oder mehreren der Ansprüche 1 bis 10 für elektrooptische Zwecke.

13. Verwendung des flüssigkristallinen Mediums nach Anspruch 12 in Shutter-Brillen, für 3D-Anwendungen, in TN-, PS-TN-, STN-, TN-TFT-, OCB-, IPS-, PS-IPS-, FFS-, PS-FFS- und PS-VA-IPS-Anzeigen.

14. Elektrooptische Flüssigkristallanzeige enthaltend ein flüssigkristallines Medium nach einem oder mehreren der Ansprüche 1 bis 10.

## Claims

1. Liquid-crystalline medium, **characterised in that** the medium comprises at least one compound of the formula I, 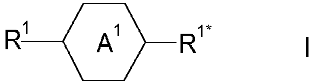 in which
R¹ and R^{1*} in each case, independently of one another, denote an alkyl or alkoxy radical having 1 to 15 C atoms, where, in addition, one or more CH₂ groups in these radicals may in each case be replaced, independently of one another, by -C≡C-, -CF₂O-, -CH=CH-, 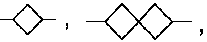 -O- in such a way that O atoms are not linked directly to one another, and in which, in addition, one or more H atoms may be replaced by halogen,
A¹ denotes 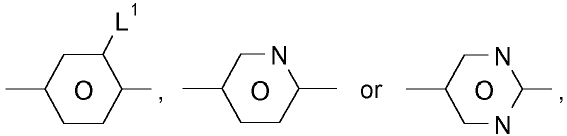
L¹ denotes F, Cl, CF₃, OCF₃ or CHF₂.

2. Liquid-crystalline medium according to Claim 1, **characterised in that** the medium comprises at least one compound of the formulae I-1 to I-10, 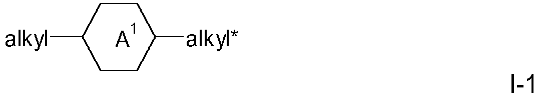 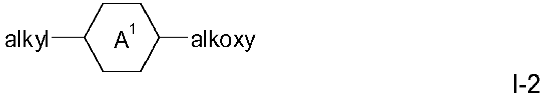 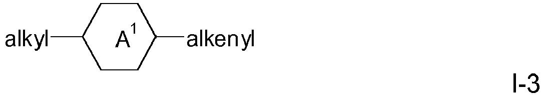 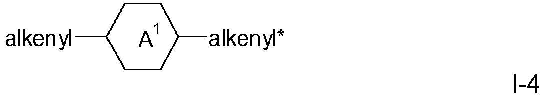 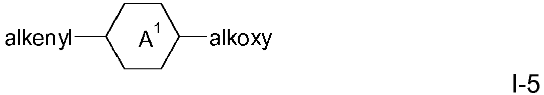 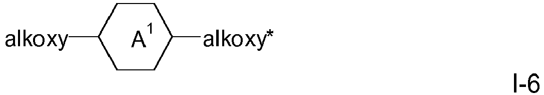 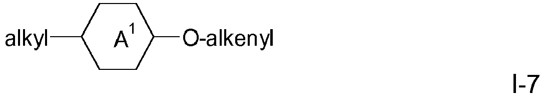 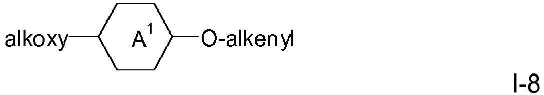 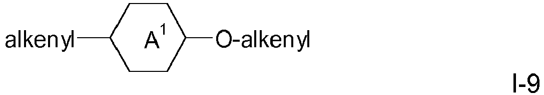 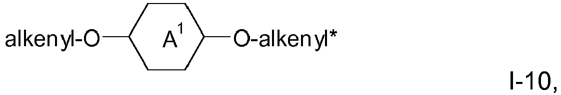 in which
alkyl and alkyl* in each case, independently of one another, denote a straight-chain alkyl radical having 1-6 C atoms,
alkenyl and alkenyl* in each case, independently of one another, denote a straight-chain alkenyl radical having 2-6 C atoms,
alkoxy and alkoxy* in each case, independently of one another, denote a straight-chain alkoxy radical having 1-6 C atoms, and
A¹ has one of the meanings indicated under Claim 1.

3. Liquid-crystalline medium according to Claim 1 or 2, **characterised in that** it additionally comprises one or more compounds selected from the formulae II and/or III, 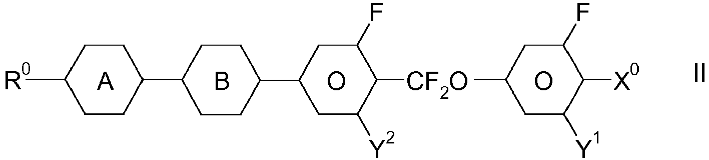 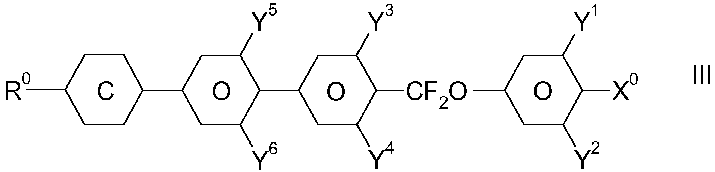 in which
R⁰ denotes a halogenated or unsubstituted alkyl or alkoxy radical having 1 to 15 C atoms, where, in addition, one or more CH₂ groups in these radicals may in each case be replaced, independently of one another, by -C≡C-, -CF₂O-, -CH=CH-, 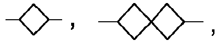 -O-, -CO-O- or -O-CO- in such a way that O atoms are not linked directly to one another,
X⁰ denotes F, Cl, CN, SF5, SCN, NCS, a halogenated alkyl radical, a halogenated alkenyl radical, a halogenated alkoxy radical or a halogenated alkenyloxy radical having up to 6 C atoms, and
Y¹⁻⁶ in each case, independently of one another, denote H, F or Cl, 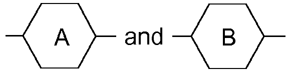 in each case, independently of one another, denote 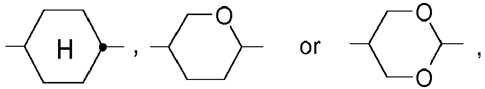 and

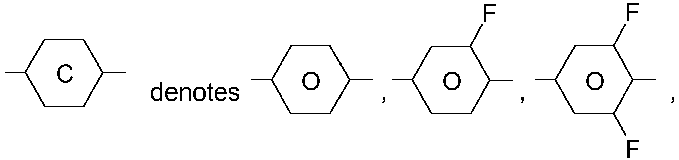

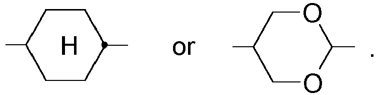

4. Liquid-crystalline medium according to one or more of Claims 1 to 3, **characterised in that** it additionally comprises one or more compounds selected from the formulae IX to XII, 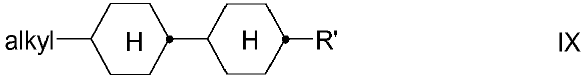 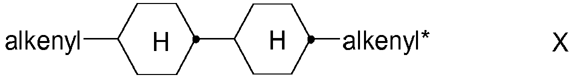 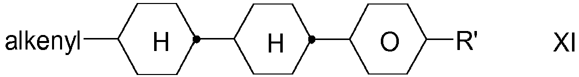 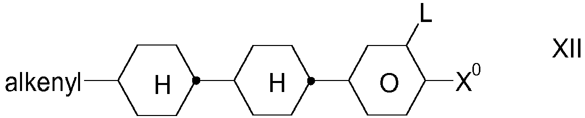 in which X⁰ has the meanings indicated in Claim 3,
L denotes H or F,
"alkyl" denotes C₁₋₆-alkyl,
R' denotes C₁₋₆-alkyl, C₁₋₆-alkoxy or C₂₋₆-alkenyl, and
"alkenyl" and "alkenyl*" in each case, independently of one another, denote C₂₋₆-alkenyl.

5. Liquid-crystalline medium according to one or more of Claims 1 to 4, **characterised in that** it additionally comprises one or more compounds of the formula XIII, 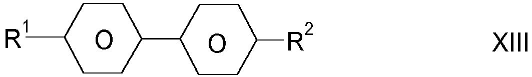 in which R¹ and R² in each case, independently of one another, denote n-alkyl, alkoxy, oxaalkyl, fluoroalkyl or alkenyl, each having up to 6 C atoms.

6. Liquid-crystalline medium according to one or more of Claims 1 to 5, **characterised in that** it additionally comprises one or more compounds of the formula XVII, 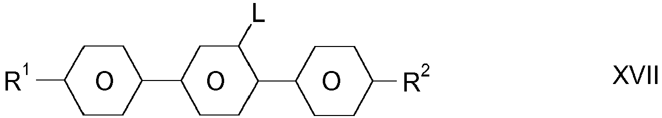 in which R¹ and R² in each case, independently of one another, denote n-alkyl, alkoxy, oxaalkyl, fluoroalkyl or alkenyl, each having up to 8 C atoms, and L denotes H or F.

7. Liquid-crystalline medium according to one or more of Claims 1 to 6, **characterised in that** it additionally comprises one or more compounds selected from the group of the compounds of the formulae XXVII, XXVIII and XXIX, 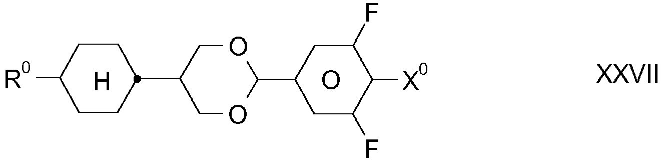 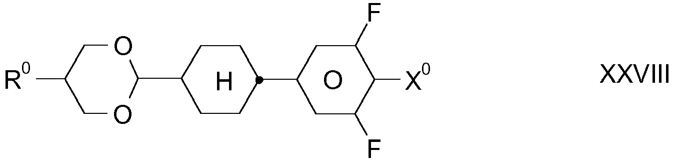 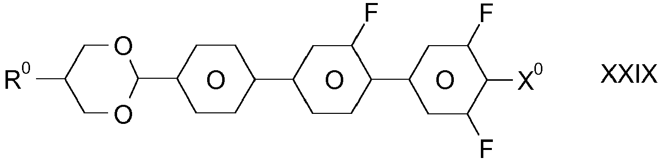 in which R⁰ and X⁰ have the meanings indicated in Claim 3.

8. Liquid-crystalline medium according to one or more of Claims 1 to 7, **characterised in that** it additionally comprises one or more compounds selected from the group of the compounds of the formulae XIX, XX, XXI, XXII, XXIII and XXIV, 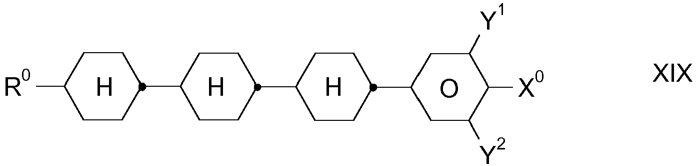 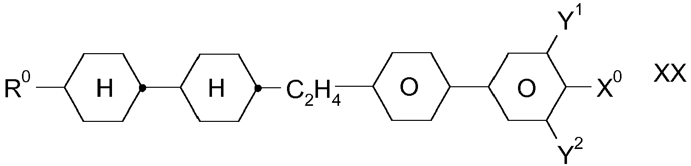 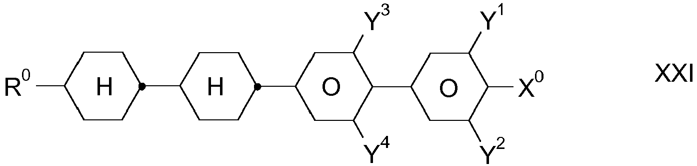 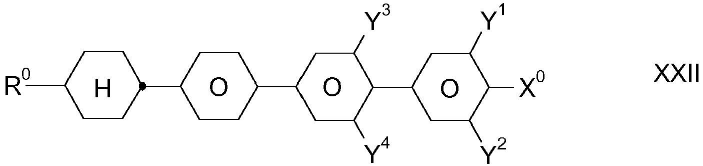 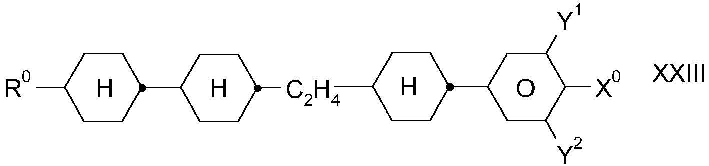 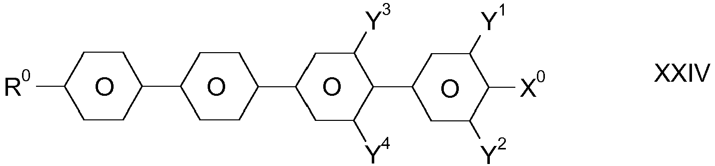 in which R⁰ and X⁰ have the meanings indicated in Claim 3, and Y¹⁻⁴ in each case, independently of one another, denote H or F.

9. Liquid-crystalline medium according to one or more of Claims 1 to 8, **characterised in that** it additionally comprises one or more additive(s) selected from the group of the UV stabilisers, dopants and antioxidants.

10. Liquid-crystalline medium according to one or more of Claims 1 to 9, **characterised in that** it additionally comprises one or more polymerisable compounds.

11. Process for the preparation of a liquid-crystalline medium according to one or more of Claims 1 to 10, **characterised in that** one or more compounds of the formula I, as defined in Claim 1, are mixed with one or more compounds according to one or more of Claims 3 to 8 or with further liquid-crystalline compounds and optionally also with one or more additives and/or at least one polymerisable compound.

12. Use of a liquid-crystalline medium according to one or more of Claims 1 to 10 for electro-optical purposes.

13. Use of the liquid-crystalline medium according to Claim 12 in shutter spectacles, for 3D applications, in TN, PS-TN, STN, TN-TFT, OCB, IPS, PS-IPS, FFS, PS-FFS and PS-VA-IPS displays.

14. Electro-optical liquid-crystal display containing a liquid-crystalline medium according to one or more of Claims 1 to 10.

## Revendications

1. Milieu cristallin liquide, **caractérisé en ce que** le milieu comprend au moins un composé de la formule I, 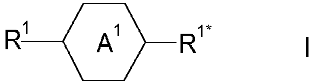 dans laquelle
R¹ et R^{1*} représentent, dans chaque cas de manière indépendante l'un de l'autre, un radical alkyle ou alcoxy qui comporte de 1 à 15 atome(s) de C, où, en outre, un ou plusieurs groupe(s) CH2 dans ces radicaux peut/peuvent, dans chaque cas de manière indépendante les uns des autres, être remplacé(s) par -C≡C-, -CF₂O-, -CH=CH-, 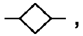 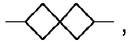 -O- de telle sorte que des atomes de O ne soient pas liés directement les uns aux autres, et où, en outre, un ou plusieurs atome(s) de H peut/peuvent être remplacé(s) par halogène,
A¹ représente, 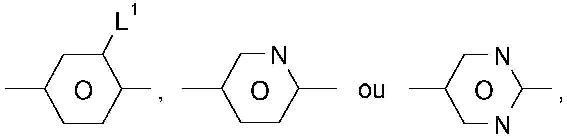
L¹ représente F, Cl, CF₃, OCF₃ ou CHF₂.

2. Milieu cristallin liquide selon la revendication 1, **caractérisé en ce que** le milieu comprend au moins un composé des formules I-1 à I-10, 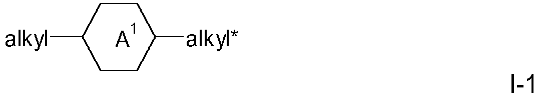 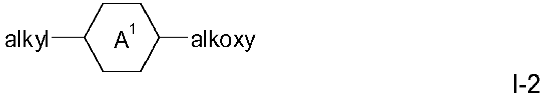 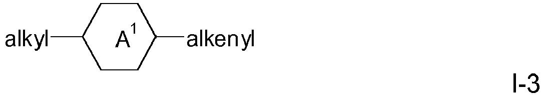 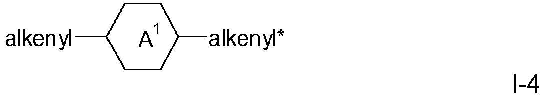 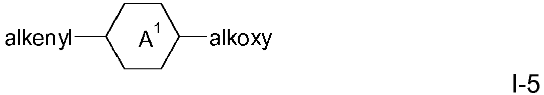 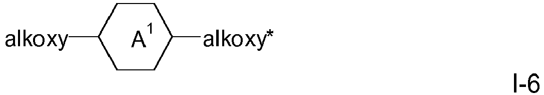 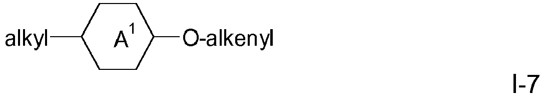 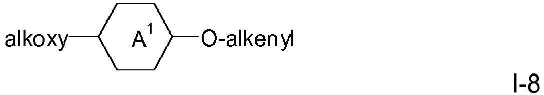 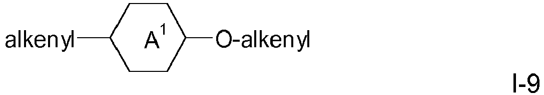 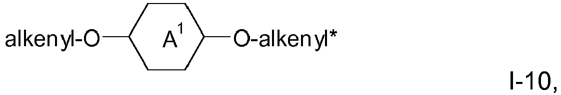 dans lesquelles
alkyl et alkyl* représentent, dans chaque cas de manière indépendante l'un de l'autre, un radical alkyle en chaîne droite qui comporte de 1 à 6 atome(s) de C,
alkenyl et alkenyl* représentent, dans chaque cas de manière indépendante l'un de l'autre, un radical alkényle en chaîne droite qui comporte de 2 à 6 atomes de C,
alkoxy et alkoxy* représentent, dans chaque cas de manière indépendante l'un de l'autre, un radical alcoxy en chaîne droite qui comporte de 1 à 6 atome(s) de C, et
A¹ présente l'une des significations qui ont été indiquées selon la revendication 1.

3. Milieu cristallin liquide selon la revendication 1 ou 2, **caractérisé en ce qu'**il comprend de façon additionnelle un ou plusieurs composé(s) qui est/sont sélectionné(s) parmi les formules II et/ou III, 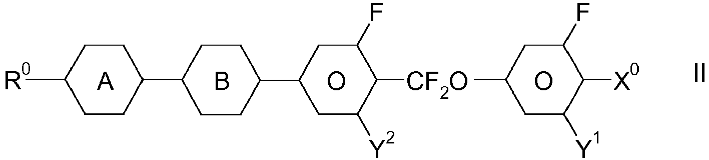 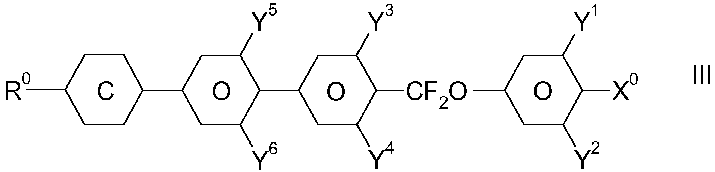 dans lesquelles
R⁰ représente un radical alkyle ou alcoxy halogéné ou non substitué qui comporte de 1 à 15 atome(s) de C, où, en outre, un ou plusieurs groupe(s) CH2 dans ces radicaux peut/peuvent dans chaque cas être remplacé(s), de manière indépendante les uns des autres, par -C≡C-, -CF₂O-, -CH=CH-, 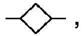 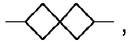 -O-, -CO-O- ou -O-CO- de telle sorte que des atomes de O ne soient pas liés directement les uns aux autres,
X⁰ représente F, Cl, CN, SF5, SCN, NCS, un radical alkyle halogéné, un radical alkényle halogéné, un radical alcoxy halogéné ou un radical alkényloxy halogéné qui comporte jusqu'à 6 atomes de C, et
Y¹⁻⁶ représentent, dans chaque cas de manière indépendante les uns des autres, H, F ou Cl, 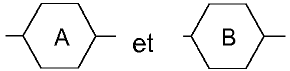 représentent, dans chaque cas de manière indépendante l'un de l'autre, 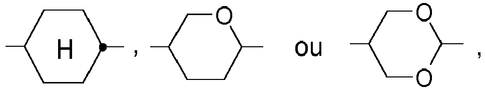 et 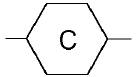 représente

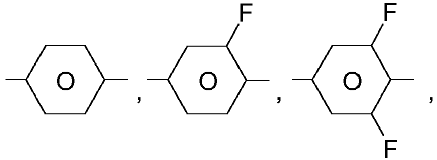

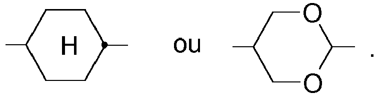

4. Milieu cristallin liquide selon une ou plusieurs des revendications 1 à 3, **caractérisé en ce qu'**il comprend de façon additionnelle un ou plusieurs composé(s) qui est/sont sélectionné(s) parmi les formules IX à XII, 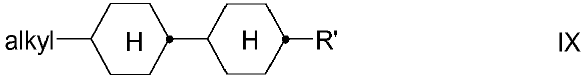 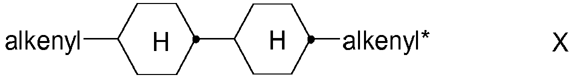 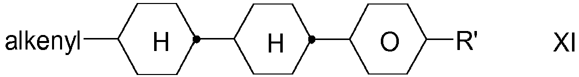 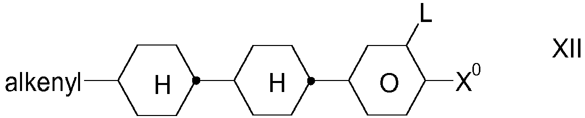 dans lesquelles X⁰ présente les significations qui ont été indiquées selon la revendication 3,
L représente H ou F,
"alkyl" représente C₁₋₆-alkyle,
R' représente C₁₋₆-alkyle, C₁₋₆-alcoxy ou C₂₋₆-alkényle, et
"alkenyl" et "alkenyl*" représentent, dans chaque cas de manière indépendante l'un de l'autre, C₂₋₆-alkényle.

5. Milieu cristallin liquide selon une ou plusieurs des revendications 1 à 4, **caractérisé en ce qu'**il comprend de façon additionnelle un ou plusieurs composé(s) de la formule XIII, 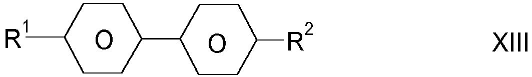 dans laquelle R¹ et R² représentent, dans chaque cas de manière indépendante l'un de l'autre, n-alkyle, alcoxy, oxaalkyle, fluoroalkyle ou alkényle, dont chacun comporte jusqu'à 6 atomes de C.

6. Milieu cristallin liquide selon une ou plusieurs des revendications 1 à 5, **caractérisé en ce qu'**il comprend de façon additionnelle un ou plusieurs composé(s) de la formule XVII, 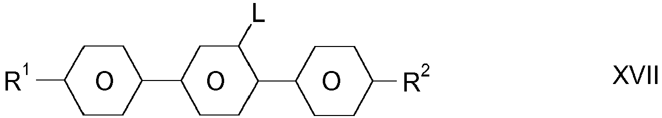 dans laquelle R¹ et R² représentent, dans chaque cas de manière indépendante l'un de l'autre, n-alkyle, alcoxy, oxaalkyle, fluoroalkyle ou alkényle, dont chacun comporte jusqu'à 8 atomes de C, et L représente H ou F.

7. Milieu cristallin liquide selon une ou plusieurs des revendications 1 à 6, **caractérisé en ce qu'**il comprend de façon additionnelle un ou plusieurs composé(s) qui est/sont sélectionné(s) parmi le groupe des composés des formules XXVII, XXVIII et XXIX, 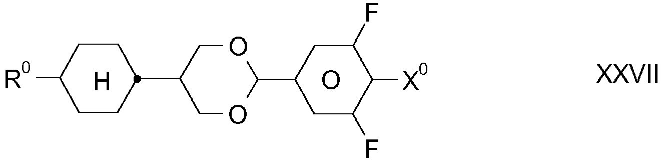 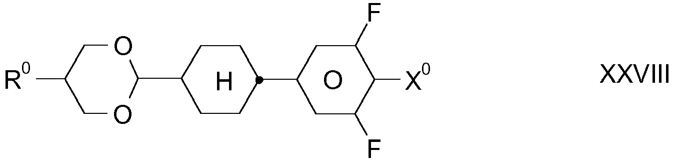 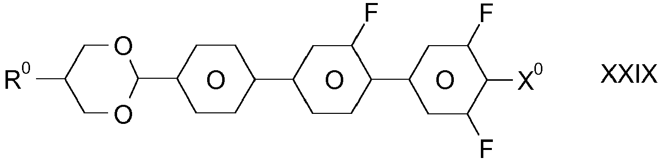 dans lesquelles R⁰ et X⁰ présentent les significations qui ont été indiquées selon la revendication 3.

8. Milieu cristallin liquide selon une ou plusieurs des revendications 1 à 7, **caractérisé en ce qu'**il comprend de façon additionnelle un ou plusieurs composé(s) qui est/sont sélectionné(s) parmi le groupe des composés des formules XIX, XX, XXI, XXII, XXIII et XXIV, 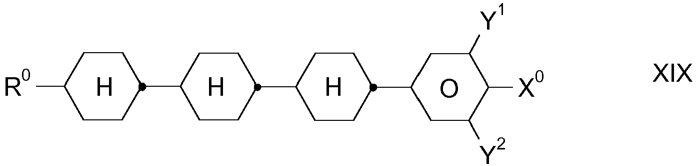 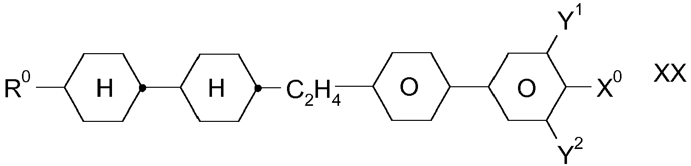 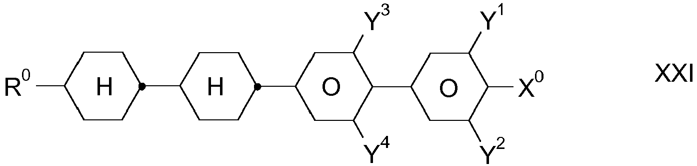 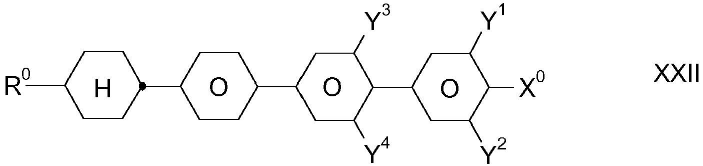 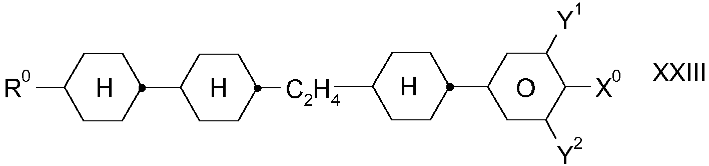 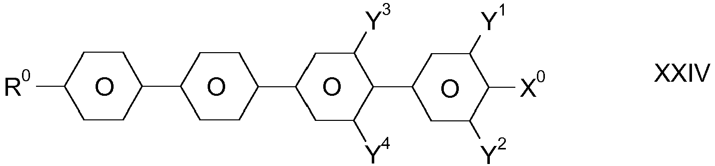 dans lesquelles R⁰ et X⁰ présentent les significations qui ont été indiquées selon la revendication 3, et Y¹⁻⁴ représentent, dans chaque cas de manière indépendante les uns des autres, H ou F.

9. Milieu cristallin liquide selon une ou plusieurs des revendications 1 à 8, **caractérisé en ce qu'**il comprend de façon additionnelle un ou plusieurs additif(s) qui est/sont sélectionné(s) parmi le groupe constitué par les stabiliseurs UV, les dopants et les antioxydants.

10. Milieu cristallin liquide selon une ou plusieurs des revendications 1 à 9, **caractérisé en ce qu'**il comprend de façon additionnelle un ou plusieurs composé(s) polymérisable(s).

11. Procédé pour la préparation d'un milieu cristallin liquide selon une ou plusieurs des revendications 1 à 10, **caractérisé en ce qu'**un ou plusieurs composé(s) de la formule I, comme il a été défini selon la revendication 1, est/sont mélangé(s) avec un ou plusieurs composé(s) selon une ou plusieurs des revendications 3 à 8 ou avec d'autres composés cristallins liquides et en option, également avec un ou plusieurs additif(s) et/ou au moins un composé polymérisable.

12. Utilisation d'un milieu cristallin liquide selon une ou plusieurs des revendications 1 à 10 à des fins électro-optiques.

13. Utilisation du milieu cristallin liquide selon la revendication 12 dans des lunettes à obturateurs, pour des applications 3D, dans des affichages TN, PS-TN, STN, TN-TFT, OCB, IPS, PS-IPS, FFS, PS-FFS et PS-VA-IPS.

14. Affichage à cristaux liquides électro-optique contenant un milieu cristallin liquide selon une ou plusieurs des revendications 1 à 10.
